# EUROPEAN PATENT APPLICATION

(11) **EP 1 997 856 A1**
(43) Date of publication of application: **03.12.2008**
(21) Application number: 07707056.3
(22) Date of filing: 19.01.2007
(51) Int. Cl.: C09B 45/34, B41M 5/26, C07D 209/48, C07D 401/12, C07D 403/12, C07D 403/14, C07D 405/12, C07D 405/14, C07D 409/12, C07D 413/12, C07D 417/12, G11B 7/244

(54) **OPTICAL RECORDING MEDIUM AND AZO METAL CHELATE COMPOUND**

(30) Priority: 20.01.2006 JP 2006013069
(71) Applicant: Mitsui Chemicals, Inc., Tokyo 105-7117 (JP)
(72) Inventor: NISHIMOTO, Taizo, Chiba 299-0265 (JP); ISHIDA, Tsutomu, Chiba 297-0017 (JP); SEINO, Kazuhiro, Osaka 581-0034 (JP); MIYASATO, Masataka, Chiba 299-0265 (JP); FUJII, Kenichi, Chiba 299-0265 (JP); IMAI, Masao, Ciba 299-0265 (JP); TAKAHASHI, Eiichi, Chiba 299-0265 (JP); UENO, Keiji, Chiba 299-0265 (JP); USUI, Hideo, Chiba 299-0265 (JP); ASO, Yoshiaki, Chiba 299-0265 (JP); OGISO, Akira, Chiba 299-0265 (JP); SUZUKI, Rihoko, Chiba 299-0265 (JP); KINOSHITA, Satoshi, Osaka 581-0034 (JP); KOHSAKA, Akihiro, Osaka 581-0034 (JP); KATO, Kenichi, Osaka 581-0034 (JP); YOSHIDA, Takafumi, Osaka 581-0034 (JP); SASAKI, Hiroyuki, Osaka 581-0034 (JP); KUMAGAE, Yojiro, Osaka 581-0034 (JP)
(74) Representative: Stuart, Ian Alexander
(86) International application number: PCT/JP2007/050768
(87) International publication number: WO 2007/083732

(57) **Abstract**

The present invention is to provide an optical recording medium which is capable of performing good recording and reproducing by using a laser having a wavelength of 300 to 900 nm, a novel azo metal chelate compound, and a novel azo compound. Furthermore, the present invention is to provide an optical recording medium having an azo metal chelate compound in a recording layer.

## Description

### TECHNICAL FIELD

The present invention relates to an optical recording medium. Particularly, the invention relates to an optical recording medium which is capable of performing recording and reproducing by a blue laser light as one of visible lasers. In addition, the invention relates to a novel azo metal chelate compound.

### BACKGROUND ART

At present, as an external information storage unit capable of storing large-capacity information and allowing easy random access, various optical recording media such as CD-R/RW, DVD-R/RW, MO and the like have been widely recognized and have come into wide use. Of these media, an organic dye-based optical recording medium including typical examples of CD-R and DVD-R is considered to have superiority because it is cheap and easily processed.

On the other hand, due to the increase in the amount of information to be handled, higher recording density in media has been desired. As a means for achieving such high density recording media, shortening of wavelength of laser wavelengths used for recording and reproducing can be cited. However, an optical recording medium capable of performing high density recording and reproducing using a blue laser having a wavelength of 400 nm to 500 nm has been proposed and its practical use has been speeded.

At the present as an organic dye compound capable of performing recording with a blue laser of 400 nm to 500 nm, there have been proposed that in addition to a cyanine-based dye compound and a porphyrin-based dye compound, a polyene-based dye compound, an azo-based dye compound, a dicyanovinylphenyl compound, a coumarin compound, a pyrimidine compound, a naphthalocyanine compound, a 5-membered heterocyclic ring compound, a bisazole compound, an aminopyridine compound, a bispyridinium compound, an oxonol compound, a styryl compound, an aminobutadiene compound, a metal chelate compound, a quinone compound, a quinodimethane compound, a hydrazone compound, a triazine compound, a carbostyryl compound or a naphthyridine compound, a condensed heterocyclic compound, a stilbene compound and the like.

Of such compounds, since the azo-based dye compound has a high extinction coefficient, easily selects an absorption wavelength by substituents, and is excellent in the solubility to a solvent and coating stability, many proposals have been made (Patent Documents 1 to 12).
Patent Document 1: Japanese Patent Laid-open No. 1999-42856
Patent Document 2: Japanese Patent Laid-open No. 1999-334204
Patent Document 3: Japanese Patent Laid-open No. 1999-334205
Patent Document 4: Japanese Patent Laid-open No. 2001-158862
Patent Document 5: Japanese Patent Laid-open No. 2001-214084
Patent Document 6: Japanese Patent Laid-open No. 2001-271001
Patent Document 7: Japanese Patent Laid-open No. 2002-74740
Patent Document 8: Japanese Patent Laid-open No. 2002-200848
Patent Document 9: Japanese Patent Laid-open No. 2002-293031
Patent Document 10: Japanese Patent Laid-open No. 2003-170662
Patent Document 11: Japanese Patent Laid-open No. 2004-291244
Patent Document 12: Japanese PCT International Patent Laid-open No. 2004-523396

### DISCLOSURE OF THE INVENTION

Examples of the performance required for an organic dye compound capable of performing recording with a blue laser include the solubility to a solvent when it is applied on a substrate, coating property, storage stability, light resistance, stability against reproduction light and the like as well as recording characteristics when it is used for an optical recording medium. However, organic dye compounds for a blue laser satisfying these characteristics at the same time have not yet been realized.

If reproduction is carried out at the same blue-violet laser wavelength as that used for recording, irradiation with even feeble light such as reproduction light promotes photochemical reactions, causing a problem that a dye is deteriorating on every reproduction. Of the above required characteristics, the stability against reproduction light is a required property in order to avoid such a problem. However, in present, the reproduction light needs to have a wavelength longer than that of recording light as a countermeasure to the above problem, and consequently demand for high density is not fully satisfied.

An object of the present invention is to provide an optical recording medium containing a recording layer suitable to ultra-high density recording which is capable of performing good recording and reproducing by using a laser light having a wavelength of 300 nm to 900 nm, particularly a blue-violet laser light selected from a wavelength range of 400 nm to 410 nm, and particularly an optical recording medium which is excellent in the recording sensitivity and the stability against reproduction light.
Furthermore, the invention is to provide a novel compound which can be suitably used for the optical recording medium.

In order to solve the above objects, the present inventors have repeatedly conducted an extensive study and as a result, the present invention has been completed. That is, the present invention relates to:
(1) an optical recording medium having at least a recording layer containing an organic dye and a reflective layer on a substrate, wherein the recording layer contains a metal chelate compound of azo compounds represented by the general formula (1) below and a metal, 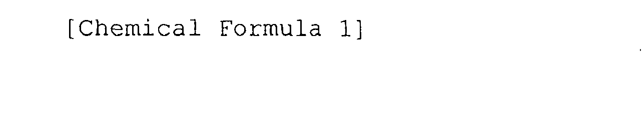 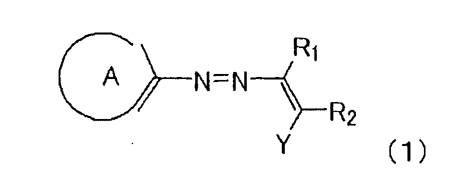 wherein, in the formula, ring A represents a carbocyclic aromatic ring which may have a substituent or a heterocyclic aromatic ring which may have a substituent, and two adjacent atoms in the ring are bonded with each other via a linking group represented by the general formula (a) below and form at least one imide ring;
   R₁ and R₂ each independently represent a hydrogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, a substituted or unsubstituted hetero arylcarbonyl group, a substituted or unsubstituted alkylaminothiocarbonyl group, a substituted or unsubstituted arylaminothiocarbonyl group, a substituted or unsubstituted alkylthiocarbonyl group, a substituted or unsubstituted arylthiocarbonyl group, a substituted or unsubstituted hetero arylthiocarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted arylcarbonyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted acylthio group, a substituted or unsubstituted arylcarbonylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group, and may be bonded with each other to form a hydrocarbon ring which may have a substituent or a heterocyclic ring which may have a substituent;
   when ring A has substituents, and the hydrocarbon ring or the heterocyclic ring formed by bonding R₁ and R₂ has substituents, substituents may be bonded with each other to form a ring; and Y represents a group having an active hydrogen, 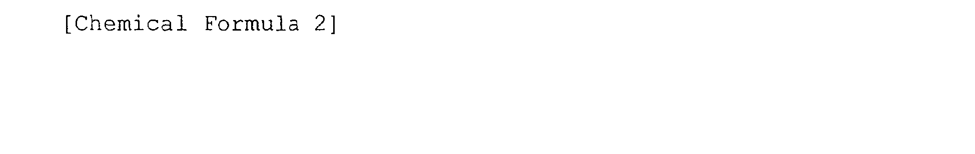 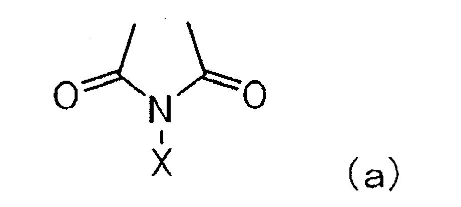 wherein, in the formula, X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
(2) the optical recording medium as set forth in (1) having at least a recording layer containing an organic dye and a reflective layer on a substrate, wherein the recording layer contains a metal chelate compound of azo compounds represented by the general formula (2) below and a metal, 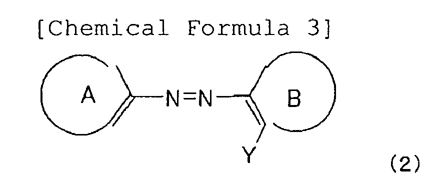 wherein, in the formula, ring A represents a carbocyclic aromatic ring which may have a substituent or a heterocyclic aromatic ring which may have a substituent, and two adjacent atoms in the ring are bonded with each other via a linking group represented by the above general formula (a) and form at least one imide ring;
   ring B represents a hydrocarbon ring which may have a substituent or a heterocyclic ring which may have a substituent; when rings A and B have substituents, substituents may be bonded with each other to form a ring; and
   Y represents a group having an active hydrogen;
(3) the optical recording medium as set forth in (2), wherein the recording layer contains a metal chelate compound of azo compounds represented by the general formula (3) below and a metal, 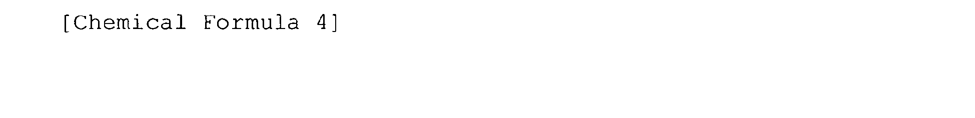 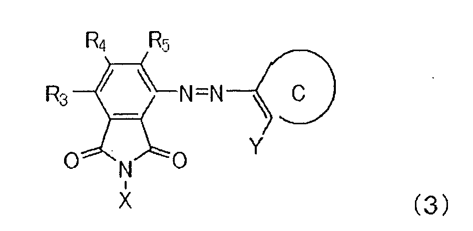 wherein, in the formula, X represents the same as those described above;
   R₃ to R₅ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
   Y represents a group having an active hydrogen; and ring C represents the general formulae (C-1) to (C-7) below, 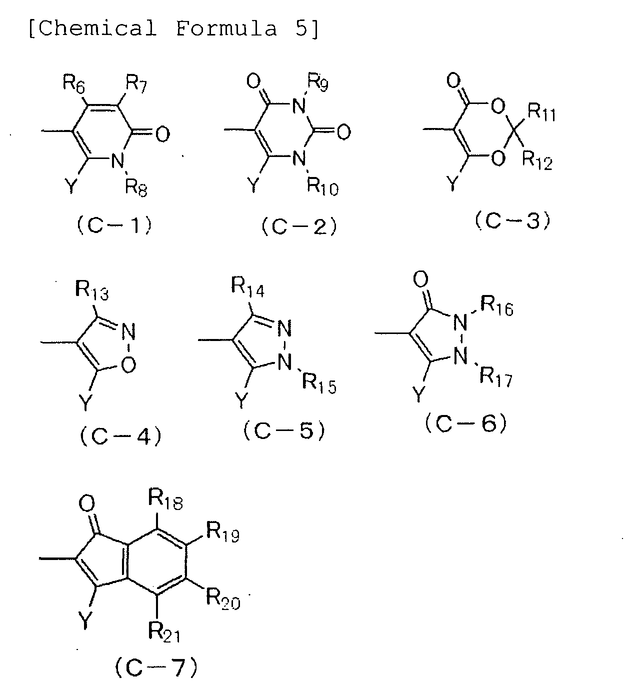 wherein, in the formula, R₆ to R₂₁ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylsulfonyl group, a substituted or unsubstituted arylsulfonyl group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
   R₁₁ and R₁₂ may be bonded with each other to form a 5 to 6-membered ring; and Y represents the same as those described above;
(4) the optical recording medium as set forth in (2), wherein the recording layer contains a metal chelate compound of azo compounds represented by the general formula (4) below and a metal, 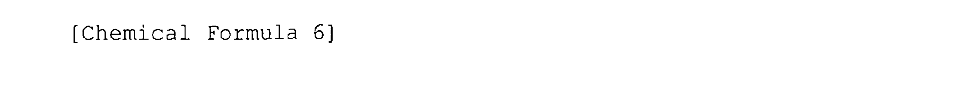 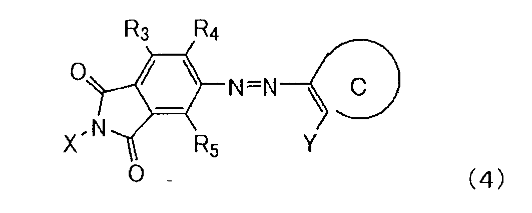 wherein, in the formula, X, R₃ to R₅, Y and ring C represent the same as those described above;
(5) the optical recording medium as set forth in (1), wherein the recording layer contains a metal chelate compound of azo compounds represented by the general formula (5) below and a metal, 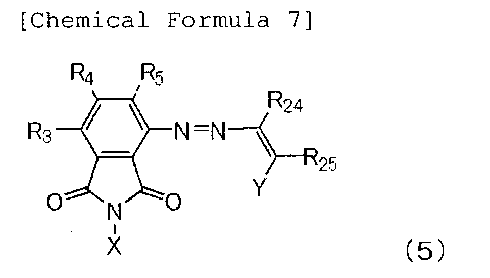 wherein, in the formula, X, R₃ to R₅ and Y represent the same as those described above;
   R₂₄ represents a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, or a substituted or unsubstituted hetero arylcarbonyl group; and
   R₂₅ represents a hydroxyl group, an amino group, a substituted or unsubstituted alkyl group, a substituted or unsubstituted aralkyl group, a substituted or unsubstituted aryl group, a substituted or unsubstituted hetero aryl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted alkylamino group, a substituted or unsubstituted arylamino group, or a substituted or unsubstituted hetero arylamino group;
(6) the optical recording medium as set forth in (1), wherein the recording layer contains a metal chelate compound of azo compounds represented by the general formula (6) below and a metal, 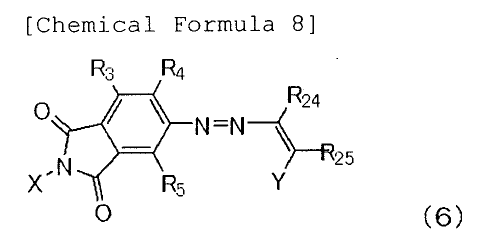 wherein, in the formula, X, R₃ to R₅, Y, R₂₄ and R₂₅ represent the same as those described above;
(7) a metal chelate compound of azo compounds represented by the general formula (1) below and a metal, 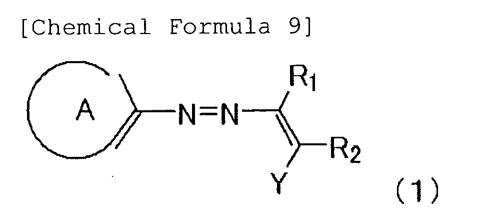 wherein, in the formula, ring A represents a carbocyclic aromatic ring which may have a substituent or a heterocyclic aromatic ring which may have a substituent, and two adjacent atoms in the ring are bonded with each other via a linking group represented by the general formula (a) below and form at least one imide ring;
   R₁ and R₂ each independently represent a hydrogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, a substituted or unsubstituted hetero arylcarbonyl group, a substituted or unsubstituted alkylaminothiocarbonyl group, a substituted or unsubstituted arylaminothiocarbonyl group, a substituted or unsubstituted alkylthiocarbonyl group, a substituted or unsubstituted arylthiocarbonyl group, a substituted or unsubstituted hetero arylthiocarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted arylcarbonyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted acylthio group, a substituted or unsubstituted arylcarbonylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group, and may be bonded with each other to form a hydrocarbon ring which may have a substituent or a heterocyclic ring which may have a substituent;
   when ring A has substituents, and the hydrocarbon ring or the heterocyclic ring formed by bonding R₁ and R₂ has substituents, substituents may be bonded with each other to form a ring; and Y represents a group having an active hydrogen, 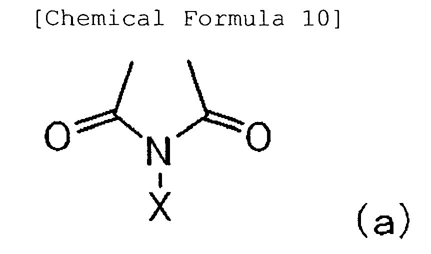 wherein, in the formula, X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
(8) a metal chelate compound of azo compounds represented by the general formula (2) below and a metal, 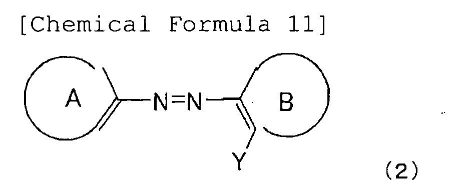 wherein, in the formula, ring A represents a carbocyclic aromatic ring which may have a substituent or a heterocyclic aromatic ring which may have a substituent, and two adjacent atoms in the ring are bonded with each other via a linking group represented by the above general formula (a) and form at least one imide ring;
   ring B represents a hydrocarbon ring which may have a substituent or a heterocyclic ring which may have a substituent;
   when rings A and B have substituents, substituents-may be bonded with each other to form a ring; and
   Y represents a group having an active hydrogen;
(9) a metal chelate compound of azo compounds represented by the general formula (3) below and a metal, 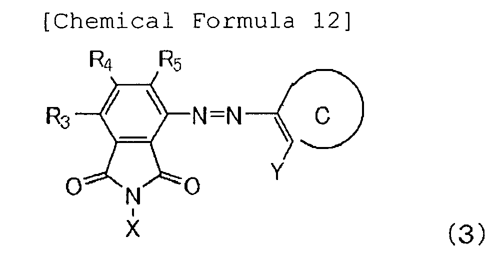 wherein, in the formula, X represents the same as those described above;
   R₃ to R₅ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
   Y represents a group having an active hydrogen; and ring C represents the general formulae (C-1) to (C-7) below, 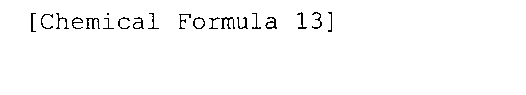 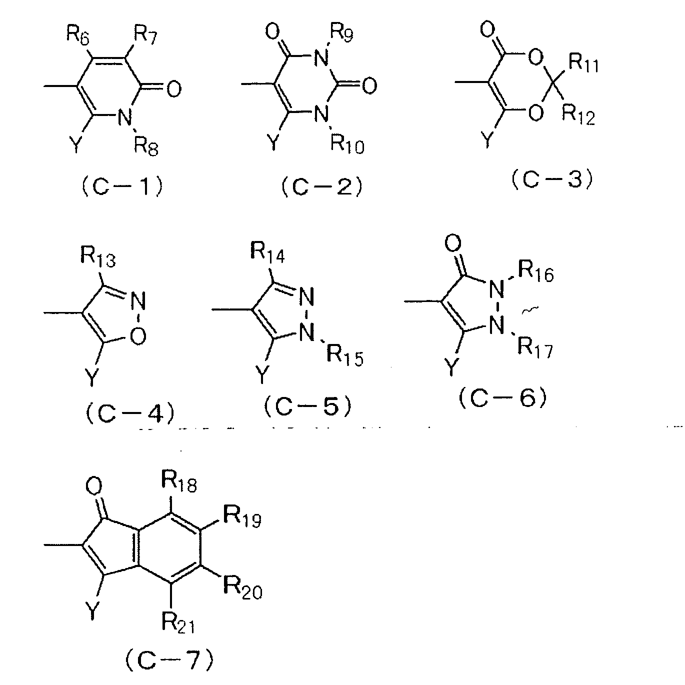 wherein, in the formula, R₆ to R₂₁ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylsulfonyl group, a substituted or unsubstituted arylsulfonyl group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
   R₁₁ and R₁₂ may be bonded with each other to form a 5 to 6-membered ring; and Y represents the same as those described above;
(10) a metal chelate compound of azo compounds represented by the general formula (4) below and a metal, 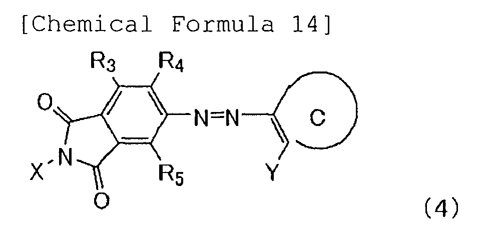 wherein, in the formula, X, R₃ to R₅, Y and ring C represent the same as those described above;
(11) a metal chelate compound of azo compounds represented by the general formula (5) below and a metal, 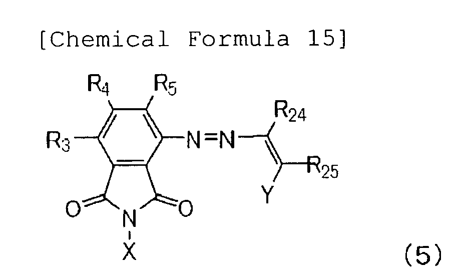 wherein, in the formula, X, R₃ to R₅ and Y represent the same as those described above;
   R₂₄ represents a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, or a substituted or unsubstituted hetero arylcarbonyl group; and
   R₂₅ represents a hydroxyl group, an amino group, a substituted or unsubstituted alkyl group, a substituted or unsubstituted aralkyl group, a substituted or unsubstituted aryl group, a substituted or unsubstituted hetero aryl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted alkylamino group, a substituted or unsubstituted arylamino group, or a substituted or unsubstituted hetero arylamino group;
(12) a metal chelate compound of azo compounds represented by the general formula (6) below and a metal, 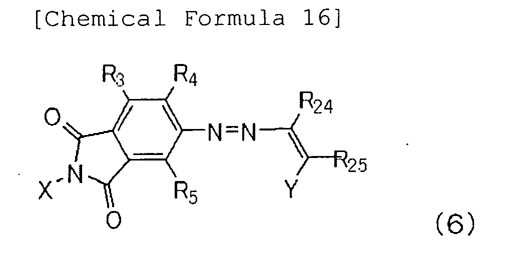 wherein, in the formula, X, R₃ to R₅, Y, R₂₄ and R₂₅ represent the same as those described above;
(13) an azo compound represented by the general formula (2) below, 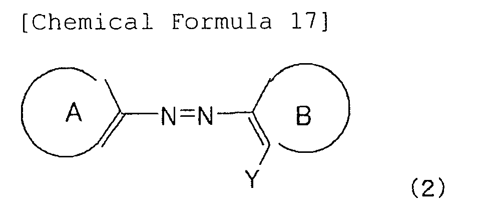 wherein, in the formula, ring A represents a carbocyclic aromatic ring which may have a substituent or a heterocyclic aromatic ring which may have a substituent, and two adjacent atoms in the ring are bonded with each other via a linking group represented by the general formula (a) below and form at least one imide ring;
   ring B represents a hydrocarbon ring which may have a substituent or a heterocyclic ring which may have a substituent;
   when rings A and B have substituents, substituents may be bonded with each other to form a ring; and
   Y represents a group having an active hydrogen, 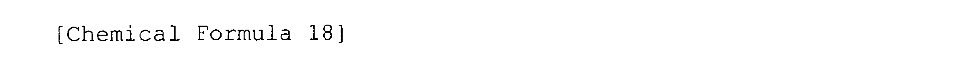 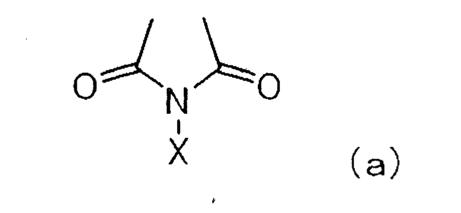 wherein, in the formula, X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted heteroc arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
(14) the azo compound as set forth in (13), represented by the general formula (10) below, 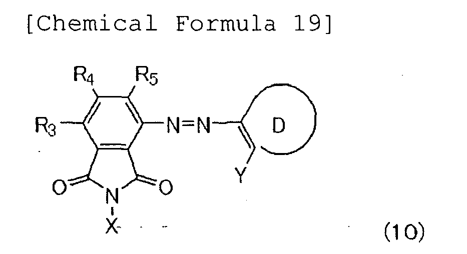 wherein, in the formula, X, R₃ to R₅ and Y represent the same as those described above; and ring D represents the general formulae (D-1) to (D-5) below, 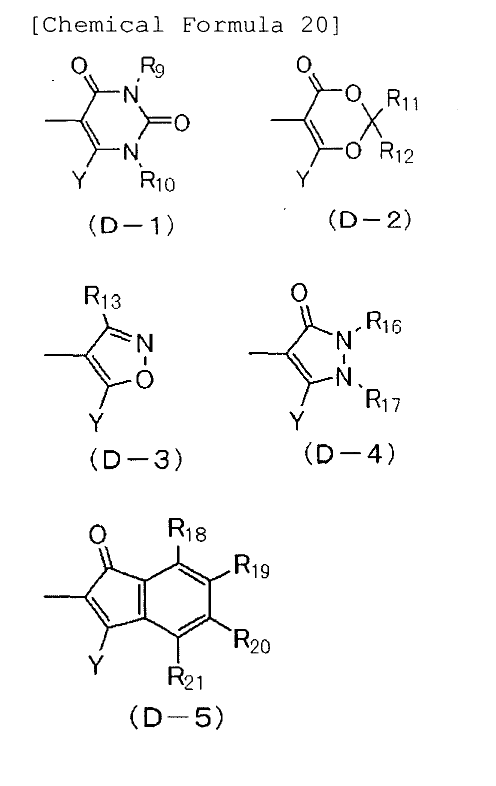 wherein, in the formula, R₉ to R₁₃ and R₁₆ to R₂₁ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylsulfonyl group, a substituted or unsubstituted arylsulfonyl group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group; and
   R₁₁ and R₁₂ may be bonded with each other to form a 5 to 6-membered ring; and
(15) the azo compound as set forth in (13), represented by the general formula (11) below, 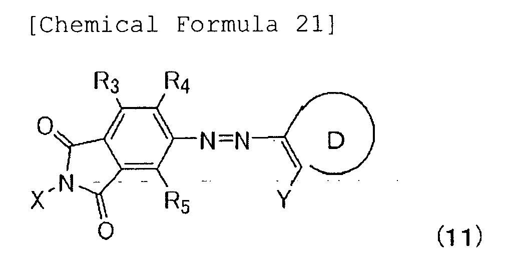
wherein, in the formula, X, R₃ to R₅, Y and ring D represent the same as those described above.

By using the azo metal chelate compound according to the present invention for the recording layer, it is possible to provide an optical recording medium which has been paid attention to as a high-density optical recording medium and is capable of performing recording and reproducing by using a laser light having a wavelength of 300 to 900 nm, particularly a blue-violet laser having a wavelength of 400 to 410 nm.

### BRIEF DESCRIPTION OF THE DRAWINGS

Fig. 1 is a schematic diagram illustrating an example of a configuration of the optical recording medium according to the present invention.
Fig. 2 is a schematic diagram illustrating another example of a configuration of the optical recording medium according to the present invention.
Fig. 3 is a schematic diagram illustrating still another example of a configuration of the optical recording medium according to the present invention.
Fig. 4 is a schematic diagram illustrating another example of a configuration of the optical recording medium according to the present invention.
Fig. 5 i.s a schematic diagram illustrating still another example of a configuration of the optical recording medium according to the present invention.
Fig. 6 is a schematic diagram illustrating another example of a configuration of the optical recording medium according to the present invention.

### BEST MODE FOR CARRYING OUT THE INVENTION

The present invention relates to an optical recording medium containing an azo metal chelate compound in a recording layer of the optical recording medium. The invention relates to a novel optical recording medium which is capable of performing recording and reproducing by using a laser light selected from a wavelength range of 300 nm to 900 nm, particularly a wavelength range of 390 nm to 430 nm and further a wavelength range of 400 nm to 410nm, and a novel azo metal chelate compound.

The optical recording medium according to the present invention refers to an optical recording medium on which information can be recorded and reproduced back. Here, as an appropriate example, the optical recording medium of the present invention having a recording layer and a reflective layer on a substrate will be explained. Incidentally, in the following, there will be described an optical recording medium in the form of an optical disk, for example, having a guide groove on a supporting substrate, a reflective film and a recording layer containing an organic dye as a main component on the guide groove, in which recording and reproducing of signals are carried out by irradiation with a laser light having a wavelength of 300 to 500 nm. However, the optical recording medium of the present invention is not limited to such shape or configuration, and includes those with various shapes such as card-type, sheet-type or the like, those without having a reflective layer, and furthermore those which can also be applied to recording and reproducing with a shorter wavelength laser.

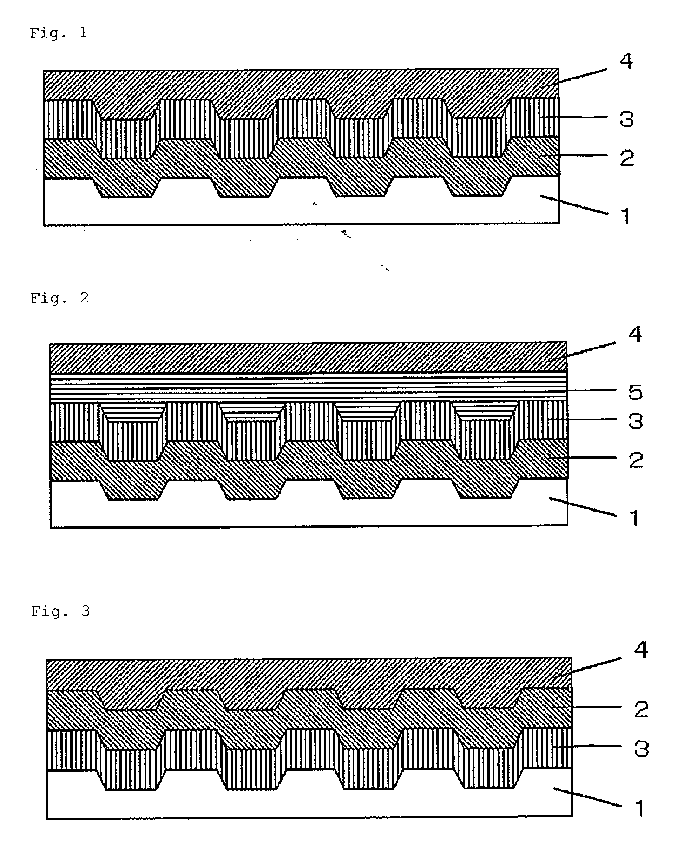

The optical recording medium of the present invention has, for example, a four-layer structure as shown in Fig. 1 in which a substrate 1, a recording layer 2, a reflective layer 3 and a protective layer 4 are stacked in the order presented. Alternatively, the optical recording medium has an adhered structure as shown in Fig. 2. That is, a recording layer 2 is formed on a substrate 1, a reflective layer 3 is formed in close contact with the recording layer 2, and a protective layer 4 is adhered on the reflective layer 3 via an adhesive layer 5. However, another layer may exist under or on the recording layer 2 and another layer may exist on the reflective layer 3. Furthermore, as shown in Fig. 3, the optical recording medium may have a structure that a substrate 1, a reflective layer 3, a recording layer 2 and a protective layer 4 are stacked in the order presented, and recording and reproduction are carried out from the side of the protective layer. Further, it may have a medium structure as disclosed in Japanese Patent Laid-open No. 1998-326435 in which a thickness of a light transmitting layer is defined by a numerical aperture N.A. of an optical system and a laser wavelength λ. Furthermore, the optical recording medium of the present invention may have a structure as disclosed in Japanese Patent Laid-open No. 1999-203729 which has two or more recording layers as required.

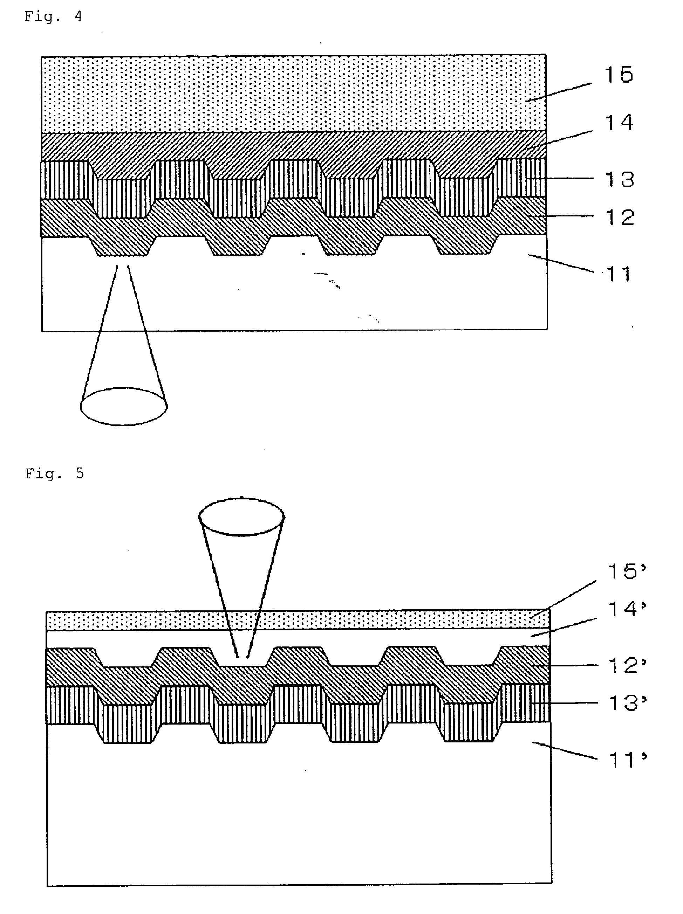

Further, the examples where the present invention is applied to optical disk include embodiments in which a substrate 11, a recording layer 12, a reflective layer 13 and a protective layer 14 are stacked in the order presented, and furthermore a dummy substrate 15 is adhered onto the protective layer 14 which also serves as an adhesive layer, as shown in Fig. 4. Of course, there may be a configuration without having the substrate 15. There may be another layer between the substrate 11 and the recording layer 12, between the recording layer 12 and the reflective layer 13, between the reflective layer 13 and the protective layer 14 or between the protective layer 14 and the dummy substrate 15. In the optical disk shown in Fig. 4, recording and reproduction are carried out from the side of the substrate 11.

Also, as another embodiment, there may be a configuration disclosed in Japanese Patent Laid-open No. 1998-302310, for example, as shown in Fig. 5, in which, on a supporting substrate 11' having a guide groove, a reflective layer 13' and a recording layer 12' having an organic dye as a main component are deposited on in the order presented, a light transmitting layer 15' is formed on the recording layer 12' through an optionally formed transparent protective layer 14', and recording and reproducing of information are carried out from the side of the light transmitting layer 15' . In addition, conversely, a guide groove may be formed on the side of the light transmitting layer 15' on which are stacked the transparent protective layer 14', the recording layer 12' and the reflective layer 13', and the supporting substrate 11' may be adhered thereto.

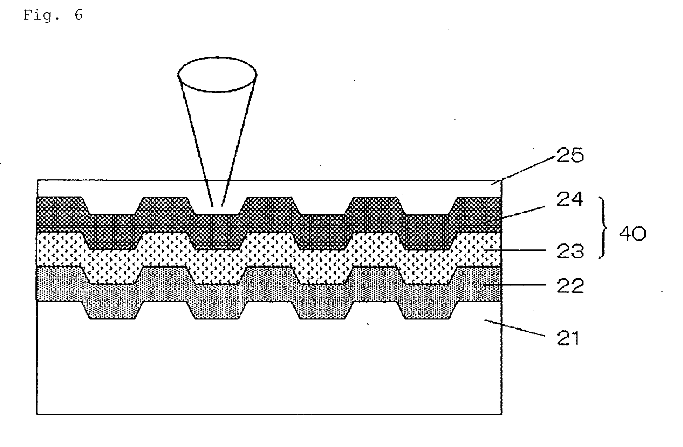

Or, as still another embodiment, there may be a configuration disclosed in Japanese Patent Laid-open No. 2002-175645, for example, as shown in Fig. 6, in which on a supporting substrate 21 having a guide groove ,a recording layer 22 having an organic dye as a main component is deposited, on the recording layer 22 a dielectric layer 40 having a nitride layer 23 and an oxide layer 24 stacked is formed in the order, and on the dielectric layer 40 a light transmitting layer 25 is formed, if necessary through an adhesive agent. Here information is recorded and reproduced from the side of the light transmitting layer 25. In addition, conversely, the guide groove may be formed on the side of the light transmitting layer 25, on which the dielectric layer 40 having the oxide layer 24 and the nitride layer 23 is stacked in order, and the recording layer 22 are stacked, and the supporting substrate 21 is adhered thereto. In this way, the compound of the present invention can be applied to such an optical recording medium in which a dielectric layer may be formed on an information recording layer without using a reflective layer for achieving an optical enhancement effect by virtue of multiple interference so that suitable initial reflectance is attained.

In the present invention, a recording layer is formed on a substrate, but the recording layer of the present invention contains at least one kind of azo metal chelate compounds as a recording dye formed from azo compounds represented by the general formula (1) and a divalent or higher valent metal salt. Herein, the recording dye refers to a dye which has sufficient absorption at the laser wavelength range concerned and, with accompanying photo-thermal conversion upon irradiation with a laser light having a prescribed energy, changes the refractive index and/or shape caused by physical and chemical change, deterioration and decomposition to form a site (mark) with altered reflectance. Furthermore, by using the compound according to the present invention, it is possible to obtain an optical recording medium capable of having a sufficient degree of modulation. The optical recording medium of the present invention is an optical recording medium which is capable of performing recording and reproducing at the recording laser wavelength selected from a wavelength range of 300 nm to 900 nm, and in which good signal characteristics are attained at the recording laser wavelength and the reproducing laser wavelength selected from a wavelength range of 390 nm to 430 nm, and furthermore a wavelength range of 400 nm to 410 nm of the above-mentioned range.

The metal chelate compound of azo compounds represented by the general formula (1) and a metal according to the present invention can satisfy optical constants required for the recording layer at the above-mentioned wavelength of a laser light since it is possible to optionally select an absorption wavelength with absorption coefficient maintained through selection of substituents. Further, the metal chelate compound is a very useful organic dye which is capaplbe of obtaining good mark shapes with low laser energy.

The present invention will be described in more detail below.
In the optical recording medium of the present invention, a metal chelate compound of azo compounds represented by the general formula (1) and a metal is contained in one or more recording layers,

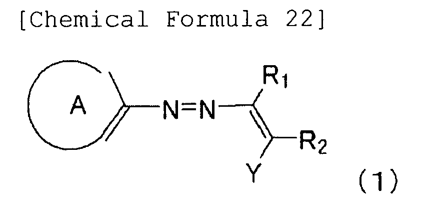

wherein, in the formula, ring A represents a carbocyclic aromatic ring which may have a substituent or a heterocyclic aromatic ring which may have a substituent, and two adjacent atoms in the ring are bonded with each other via a linking group represented by the general formula (a) below and form at least one imide ring;
R₁ and R₂ each independently represent a hydrogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, a substituted or unsubstituted hetero arylcarbonyl group, a substituted or unsubstituted alkylaminothiocarbonyl group, a substituted or unsubstituted arylaminothiocarbonyl group, a substituted or unsubstituted alkylthiocarbonyl group, a substituted or unsubstituted arylthiocarbonyl group, a substituted or unsubstituted hetero arylthiocarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted arylcarbonyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted acylthio group, a substituted or unsubstituted arylcarbonylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group, and may be bonded with each other to form a hydrocarbon ring which may have a substituent or a heterocyclic ring which may have a substituent;
when ring A has substituents, and the hydrocarbon ring or the heterocyclic ring formed by bonding R₁ and R₂ has substituents, substituents may be bonded with each other to form a ring; and
Y represents a group having an active hydrogen,

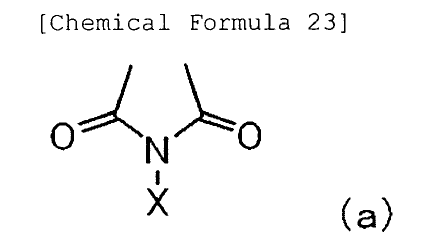

wherein, in the formula, X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkynyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group.

In the compound represented by the general formula (1), ring A represents a carbocyclic aromatic ring which may have a substituent or a heterocyclic aromatic ring which may have a substituent, and two adjacent atoms in the ring are bonded with each other via a linking group represented by the general formula (a) and form at least one imide ring,

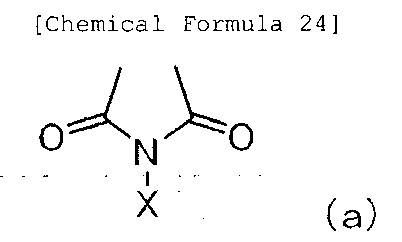

wherein, in the formula, X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group.

Herein, examples of ring A include a substituted or unsubstituted carbocyclic aromatic ring or a substituted or unsubstituted heterocyclic aromatic ring.
Preferable examples thereof include a substituted or unsubstituted carbocyclic aromatic ring having 6 to 26 carbon atoms and a substituted or unsubstituted heterocyclic aromatic ring having 3 to 26 carbon atoms, more preferable examples include a substituted or unsubstituted carbocyclic aromatic ring having 6 to 20 carbon atoms and a substituted or unsubstituted heterocyclic aromatic ring having 3 to 20 carbon atoms, further preferable examples include substituted or unsubstituted carbocyclic aromatic rings having 6 to 20 carbon atoms, and the most preferable examples include substituted or unsubstituted benzene rings.

In the above, examples of the carbocyclic aromatic ring include a substituted or unsubstituted benzene ring, a substituted or unsubstituted naphthalene ring, a substituted or unsubstituted pentalene ring, a substituted or unsubstituted indene ring, a substituted or unsubstituted indacene ring, a substituted or unsubstituted azulene ring, a substituted or unsubstituted heptalene ring, a substituted or unsubstituted biphenylene ring, a substituted or unsubstituted phenanthrene ring, a substituted or unsubstituted anthracene ring, a substituted or unsubstituted fluoranthene ring, a substituted or unsubstituted acenaphthylene ring, a substituted or unsubstituted triphenylene ring, a substituted or unsubstituted pyrene ring, a substituted or unsubstituted chrysene ring, a substituted or unsubstituted naphthacene ring, a substituted or unsubstituted pleiadene ring, a substituted or unsubstituted picene ring, a substituted or unsubstituted perylene ring, a substituted or unsubstituted pentaphene ring, a substituted or unsubstituted pentacene ring, a substituted or unsubstituted tetraphenylene ring, a substituted or unsubstituted hexaphene ring, a substituted or unsubstituted hexacene ring, a substituted or unsubstituted rubicene ring, a substituted or unsubstituted coronene ring, a substituted or unsubstituted trinaphthylene ring, a substituted or unsubstituted heptaphene ring, a substituted or unsubstituted heptacene ring, a substituted or unsubstituted pyranthrene ring, a substituted or unsubstituted ovalene ring and the like.

Examples of the heterocyclic aromatic ring include a substituted or unsubstituted furan ring, a substituted or unsubstituted thiophene ring, a substituted or unsubstituted pyrrole ring, a substituted or unsubstituted pyrazole ring, a substituted or unsubstituted imidazole ring, a substituted or unsubstituted oxazole ring, a substituted or unsubstituted thiazole ring, a substituted or unsubstituted pyridine ring, a substituted or unsubstituted pyridazine ring, a substituted or unsubstituted pyrimidine ring, a substituted or unsubstituted pyrazine ring, a substituted or unsubstituted quinoline ring, a substituted or unsubstituted isoquinoline ring, a substituted or unsubstituted quinoxaline ring, a substituted or unsubstituted indolizine ring, a substituted or unsubstituted indole ring, a substituted or unsubstituted indazole ring, a substituted or unsubstituted purine ring, a substituted or unsubstituted phthalazine ring, a substituted or unsubstituted naphthyridine ring, a substituted or unsubstituted quinazoline ring, a substituted or unsubstituted cinnoline ring, a substituted or unsubstituted pteridine ring, a substituted or unsubstituted carboline ring, a substituted or unsubstituted phenanthridine ring, a substituted or unsubstituted acridine ring, a substituted or unsubstituted perimidine ring, a substituted or unsubstituted phenanthroline ring, a substituted or unsubstituted phenazine ring, a substituted or unsubstituted furazan ring and the like.

Herein, when ring A may have substituents, examples of the substituent include a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, a substituted or unsubstituted hetero arylcarbonyl group, a substituted or unsubstituted alkylaminothiocarbonyl group, a substituted or unsubstituted arylaminothiocarbonyl group, a substituted or unsubstituted alkylthiocarbonyl group, a substituted or unsubstituted arylthiocarbonyl group, a substituted or unsubstituted hetero arylthiocarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted arylcarbonyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted acylthio group, a substituted or unsubstituted arylcarbonylthio group, a substituted or unsubstituted alkylsulfonyl group, a substituted or unsubstituted arylsulfonyl group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, and a group having a substituted or unsubstituted metalocenyl group, and substituents may be bonded with each other to form a ring.

Preferable examples thereof include halogen atoms such as a fluorine atom, a chlorine atom, a bromine atom and the like, a hydroxyl group, an amino group, a nitro group, a cyano group, a carboxyl group, a substituted or unsubstituted alkoxycarbonyl group having 2 to 7 carbon atoms, a substituted or unsubstituted aralkyloxycarbonyl group having 8 to 16 carbon atoms, a substituted or unsubstituted aryloxycarbonyl group having 7 to 11 carbon atoms, a substituted or unsubstituted alkylaminocarbonyl group having 2 to 9 carbon atoms, a substituted or unsubstituted arylaminocarbonyl group having 7 to 15 carbon atoms, a substituted or unsubstituted acyl group having 1 to 7 carbon atoms, a substituted or unsubstituted arylcarbonyl group having 7 to 11 carbon atoms, a substituted or unsubstituted hetero arylcarbonyl group having 6 to 11 carbon atoms, a substituted or unsubstituted alkylaminothiocarbonyl group having 2 to 9 carbon atoms, a substituted or unsubstituted arylaminothiocarbonyl group having 7 to 15 carbon atoms, a substituted or unsubstituted alkylthiocarbonyl group having 2 to 7 carbon atoms, a substituted or unsubstituted arylthiocarbonyl group having 7 to 11 carbon atoms, a substituted or unsubstituted hetero arylthiocarbonyl group having 6 to 11 carbon atoms, a substituted or unsubstituted alkyl group having 1 to 10 carbon atoms, a substituted or unsubstituted aralkyl group having 7 to 16 carbon atoms, a substituted or unsubstituted aryl group having 6 to 20 carbon atoms, a substituted or unsubstituted hetero aryl group having 5 to 10 carbon atoms, a substituted or unsubstituted alkenyl group having 2 to 6 carbon atoms, a substituted or unsubstituted alkynyl group having 2 to 9 carbon atoms, a substituted or unsubstituted metalocenyl group, a substituted or unsubstituted alkoxy group having 1 to 8 carbon atoms, a substituted or unsubstituted aralkyloxy group having 7 to 16 carbon atoms, a substituted or unsubstituted aryloxy group having 6 to 10 carbon atoms, a substituted or unsubstituted hetero aryloxy group having 5 to 10 carbon atoms, a substituted or unsubstituted acyloxy group having 1 to 8 carbon atoms, a substituted or unsubstituted arylcarbonyloxy group having 7 to 11 carbon atoms, a substituted or unsubstituted alkylthio group having 1 to 6 carbon atoms, a substituted or unsubstituted aralkylthio group having 7 to 16 carbon atoms, a substituted or unsubstituted arylthio group having 6 to 10 carbon atoms, a substituted or unsubstituted hetero arylthio group having 5 to 10 carbon atoms, a substituted or unsubstituted acylthio group having 2 to 8 carbon atoms, a substituted or unsubstituted arylcarbonylthio group having 7 to 11 carbon atoms, a substituted or unsubstituted alkylsulfonyl group having 2 to 7 carbon atoms, a substituted or unsubstituted arylsulfonyl group having 6 to 10 carbon atoms, a substituted or unsubstituted alkylamino group having 1 to 8 carbon atoms, a substituted or unsubstituted arylamino group having 6 to 14 carbon atoms, a substituted or unsubstituted hetero arylamino group having 5 to 10 carbon atoms, a substituted or unsubstituted alkylcarbonylamino group having 2 to 7 carbon atoms, a substituted or unsubstituted arylcarbonylamino group having 7 to 11 carbon atoms, and a group having a substituted or unsubstituted metalocenyl group.

When ring A may have substituents, concrete examples of the substituent include halogen atoms such as a fluorine atom, a chlorine atom, a bromine atom and the like;
a hydroxyl group;
an amino group;
a nitro group; a cyano group;
a carboxyl group;
substituted or unsubstituted alkoxycarbonyl groups having 2 to 7 carbon atoms such as a methoxycarbonyl group, an ethoxycarbonyl group, an n-propoxycarbonyl group, an isopropoxycarbonyl group, an n-butoxycarbonyl group, a tert-butoxycarbonyl group, a sec-butoxycarbonyl group, an n-pentyloxycarbonyl group, an n-hexyloxycarbonyl group and the like;
substituted or unsubstituted aralkyloxycarbonyl groups having 8 to 16 carbon atoms such as a benzyloxycarbonyl group, a 4-nitrobenzyloxycarbonyl group, a 4-cyanobenzyloxycarbonyl group, a 4-hydroxybenzyloxycarbonyl group, a 2-methylbenzyloxycarbonyl group, a 3-methylbenzyloxycarbonyl group, a 4-methylbenzyloxycarbonyl group, a 4-trifluoromethylbenzyloxycarbonyl group, a 1-naphthylmethoxycarbonyl group, a 2-naphthylmethoxycarbonyl group, a 4-cyano-1-naphthylmethoxycarbonyl group, a 4-hydroxy-1-naphthylmethoxycarbonyl group, a 6-hydroxy-2-naphthylmethoxycarbonyl group, a 4-methyl-1-naphthylmethoxycarbonyl group, a 6-methyl-2-naphthylmethoxycarbonyl group, a 4-trifluoromethyl-1-naphthylmethoxycarbonyl group, a fluorene-9-ylethoxycarbonyl group and the like;
substituted or unsubstituted aryloxycarbonyl groups having 7 to 11 carbon atoms such as a phenyloxycarbonyl group, a 2-methylphenyloxycarbonyl group, a 4-methylphenyloxycarbonyl group, a 4-tert-butylphenyloxycarbonyl group, a 2-methoxyphenyloxycarbonyl group, a 4-isopropylphenyloxycarbonyl group, a naphthyloxycarbonyl group and the like;
substituted or unsubstituted alkylaminocarbonyl groups having 2 to 9 carbon atoms such as a methylaminocarbonyl group, an ethylaminocarbonyl group, an n-propylaminocarbonyl group, an n-butylaminocarbonyl group, a dimethylaminocarbonyl group, a diethylaminocarbonyl group, a di-n-propylaminocarbonyl group, a di-n-butylaminocarbonyl group and the like;
substituted or unsubstituted arylaminocarbonyl groups having 7 to 15 carbon atoms such as a phenylaminocarbonyl group, a 4-methylphenylaminocarbonyl group, a 4-chlorophenylaminocarbonyl group, a 4-bromophenylaminocarbonyl group, a diphenylaminocarbonyl group, a di(4-methylphenyl)aminocarbonyl group, a di(4-chlorophenyl)aminocarbonyl group, a di(4-bromophenyl)aminocarbonyl group and the like;
substituted or unsubstituted acyl groups having 1 to 7 carbon atoms such as a formyl group, an acetyl group, a propionyl group, a butyryl group, an isobutyryl group, a valeryl group, an isovaleryl group, a pivaloyl group, a hexanoyl group, a heptanoyl group and the like;
substituted or unsubstituted arylcarbonyl groups having 7 to 11 carbon atoms such as a phenylcarbonyl group, a 4-methylphenylcarbonyl group, a 4-chlorophenylcarbonyl group, a 4-bromophenylcarbonyl group, a naphthylcarbonyl group and the like;
substituted or unsubstituted hetero arylcarbonyl groups having 6 to 11 carbon atoms such as a 4-pyridylcarbonyl group, a 3-pyridylcarbonyl group, a 2-pyridylcarbonyl group, a 4-methyl-2-pyridylcarbonyl group, a 5-methyl-2-pyridylcarbonyl group, a 6-methyl-2-pyridylcarbonyl group, a 4,6-dimethyl-2-pyridylcarbonyl group, a 4-methyl-5-nitro-2-pyridylcarbonyl group, a 3-hydroxy-2-pyridylcarbonyl group, a 6-fluoro-3-pyridylcarbonyl group, a 6-methoxy-3-pyridylcarbonyl group, a 6-methoxy-2-pyridylcarbonyl group, a 2-pyrimidylcarbonyl group, a 4-pyrimidylcarbonyl group, a 5-pyrimidylcarbonyl group, a 2,6-dimethyl-4-pyrimidylcarbonyl group, a 4-quinolylcarbonyl group, a 3-quinolylcarbonyl group, a 4-methyl-2-quinolylcarbonyl group, a 3-pyrrolylcarbonyl group, a 2-pyrrolylcarbonyl group, a 1-methyl-3-pyrrolylcarbonyl group, a 1-methyl-2-pyrrolylcarbonyl group, a 3-furylcarbonyl group, a 2-furylcarbonyl group, a 3-thienylcarbonyl group, a 2-thienylcarbonyl group, a 4-methyl-3-thienylcarbonyl group, a 5-methyl-2-thienylcarbonyl group, a 3-methyl-2-thienylcarbonyl group, a 2-oxazolylcarbonyl group, a 3-isoxazolylcarbonyl group, a 2-thiazolylcarbonyl group, a 2-thiadiazolylcarbonyl group, a 2-benzoxazolylcarbonyl group, a 2-benzothiazolylcarbonyl group, a 2-benzimidazolylcarbonyl group and the like;
substituted or unsubstituted alkylaminothiocarbonyl groups having 2 to 9 carbon atoms such as a methylaminothiocarbonyl group, an ethylaminothiocarbonyl group, an n-propylaminothiocarbonyl group, an n-butylaminothiocarbonyl group, a dimethylaminothiocarbonyl group, a diethylaminothiocarbonyl group, a di-n-propylaminothiocarbonyl group, a di-n-butylaminothiocarbonyl group and the like;
substituted or unsubstituted arylaminothiocarbonyl groups having 7 to 15 carbon atoms such as a phenylaminothiocarbonyl group, a 4-methylphenylaminothiocarbonyl group, a 4-chlorophenylaminothiocarbonyl group, a 4-bromophenylaminothiocarbonyl group, a diphenylaminothiocarbonyl group, a di(4-methylphenyl)aminothiocarbonyl group, a di(4-chlorophenyl)aminothiocarbonyl group, a di(4-bromophenyl)aminothiocarbonyl group and the like;
substituted or unsubstituted alkylthiocarbonyl groups having 2 to 7 carbon atoms such as a thioacetyl group, a thiopropionyl group, a thiobutyryl group, a thioisobutyryl group, a thiovaleryl group, a thioisovaleryl group, a thiopivaloyl group, a thiohexanoyl group, a thioheptanoyl group and the like;
substituted or unsubstituted arylthiocarbonyl groups having 7 to 11 carbon atoms such as a phenylthiocarbonyl group, a 4-methylphenylthiocarbonyl group, a 4-chlorophenylthiocarbonyl group, a 4-bromophenylthiocarbonyl group, a naphthylthiocarbonyl group and the like;
substituted or unsubstituted hetero arylthiocarbonyl groups having 6 to 11 carbon atoms such as a 4-pyridylthiocarbonyl group, a 3-pyridylthiocarbonyl group, a 2-pyridylthiocarbonyl group, a 4-methyl-2-pyridylthiocarbonyl group, a 5-methyl-2-pyridylthiocarbonyl group, a 6-methyl-2-pyridylthiocarbonyl group, a 4,6-dimethyl-2-pyridylthiocarbonyl group, a 4-methyl-5-nitro-2-pyridylthiocarbonyl group, a 3-hydroxy-2-pyridylthiocarbonyl group, a 6-fluoro-3-pyridylthiocarbonyl group, a 6-methoxy-3-pyridylthiocarbonyl group, a 6-methoxy-2-pyridylthiocarbonyl group, a 2-pyrimidylthiocarbonyl group, a 4-pyrimidylthiocarbonyl group, a 5-pyrimidylthiocarbonyl group, a 2,6-dimethyl-4-pyrimidylthiocarbonyl group, a 4-quinolylthiocarbonyl group, a 3-quinolylthiocarbonyl group, a 4-methyl-2-quinolylthiocarbonyl group, a 3-pyrrolylthiocarbonyl group, a 2-pyrrolylthiocarbonyl group, a 1-methyl-3-pyrrolylthiocarbonyl group, a 1-methyl-2-pyrrolylthiocarbonyl group, a 3-furylthiocarbonyl group, a 2-furylthiocarbonyl group, a 3-thienylthiocarbonyl group, a 2-thienylthiocarbonyl group, a 4-methyl-3-thienylthiocarbonyl group, a 5-methyl-2-thienylthiocarbonyl group, a 3-methyl-2-thienylthiocarbonyl group, a 2-oxazolylthiocarbonyl group, a 3-isoxazolylthiocarbonyl group, a 2-thiazolylthiocarbonyl group, a 2-thiadiazolylthiocarbonyl group, a 2-benzoxazolylthiocarbonyl group, a 2-benzothiazolylthiocarbonyl group, a 2-benzimidazolylthiocarbonyl group and the like;
substituted or unsubstituted alkyl groups having 1 to 10 carbon atoms such as a methyl group, an ethyl group, an n-propyl group, an isopropyl group, an n-butyl group, a sec-butyl group, a tert-butyl group, an n-pentyl group, an n-hexyl group, a cyclopropyl group, a cyclobutyl group, a cyclopentyl group, a cyclohexyl group, a 1,2,2-trimethylbutyl group, a 1,1,2-trimethylbutyl group, a 1-ethyl-2-methylpropyl group, a cyclo-hexyl group, an n-heptyl group, a 2-methylhexyl group, a 3-methylhexyl group, a 4-methylhexyl group, a 5-methylhexyl group, a 2, 4 -dimethylpentyl group, an n-octyl group, a 2-ethylhexyl group, a 2,5-dimethylhexyl group, a 2,5,5-triethylpentyl group, a 2,4-dimethylhexyl group, a 2,2,4-trimethylpentyl group, a 2-chloroethyl group, a 2-bromoethyl group, a 2-iodoethyl group, a trifluoromethyl group, a 2,2,2-trifluoroethyl group, a pentafluoroethyl group, a 3,3,3-trifluoropropyl group, a 2,2,3,3-tetrafluoropropyl group, a 4,4,4-trifluorobutyl group, a 2,2,3,3,4,4,4-heptafluorobutyl group, a hydroxymethyl group, a hydroxyethyl group, a cyanomethyl group, a 2-cyanoethyl group, a methoxycarbonylmethyl group, an ethoxycarbonylmethyl group, an n-propoxycarbonylmethyl group, an isopropoxycarbonylmethyl group, an n-butoxycarbonylmethyl group, an isobutoxycarbonylmethyl group, a sec-butoxycarbonylmethyl group, a t-butoxycarbonylmethyl group, a methoxycarbonylethyl group, an ethoxycarbonylethyl group, an n-propoxycarbonylethyl group, an isopropoxycarbonylethyl group, an n-butoxycarbonylethyl group, an isobutoxycarbonylethyl group, a sec-butoxycarbonylethyl group, a t-butoxycarbonylethyl group, a methylcarbonylmethyl group, an ethylcarbonylmethyl group, an n-propylcarbonylmethyl group, an isopropylcarbonylmethyl group, an n-butylcarbonylmethyl group, an isobutylcarbonylmethyl group, a sec-butylcarbonylmethyl group, a t-butylcarbonylmethyl group, a methylcarbonylethyl group,- an ethylcarbonylethyl group, an n-propylcarbonylethyl group, an isopropylcarbonylethyl group, an n-butylcarbonylethyl group, an isobutylcarbonylethyl group, a sec-butylcarbonylethyl group, a t-butylcarbonylethyl group, a methylsulfonylmethyl group, an ethylsulfonylmethyl group, an n-propylsulfonylmethyl group, an isopropylsulfonylmethyl group, an n-butylsulfonylmethyl group, an isobutylsulfonylmethyl group, a sec-butylsulfonylmethyl group, a t-butylsulfonylmethyl group, a methylsulfonylethyl group, an ethylsulfonylethyl group, an n-propylsulfonylethyl group, an isopropylsulfonylethyl group, an n-butylsulfonylethyl group, an isobutylsulfonylethyl group, a sec-butylsulfonylethyl group, a t-butylsulfonylethyl group, a dimethylaminomethyl group, a diethylaminomethyl group, a di(n-propyl)aminomethyl group, a di(n-butyl)aminomethyl group, a dimethylaminoethyl group, a diethylaminoethyl group, a di(n-propyl)aminoethyl group, a di(n-butyl)aminoethyl group and the like;
substituted or unsubstituted aralkyl groups having 7 to 16 carbon atoms such as a benzyl group, an α-methylbenzyl group, an α,α-dimethylbenzyl group, a 1-naphthylmethyl group, a 2-naphthylmethyl group, a furfuryl group, a 2-methylbenzyl group, a 3-methylbenzyl group, a 4-methylbenzyl group, a 4-ethylbenzyl group, a 4-isopropylbenzyl group, a 4-tert-butylbenzyl group, a 4-n-hexylbenzyl group, a 4-n-nonylbenzyl group, a 3,4-dimethylbenzyl group, a 3-methoxybenzyl group, a 4-methoxybenzyl group, a 4-ethoxybenzyl group, a 4-n-butyloxybenzyl group, a 4-n-hexyloxybenzyl group, a 4-n-nonyloxybenzyl group, a 2-fluorobenzyl group, a 3-fluorobenzyl group, a 4-fluorobenzyl group, a 2-chlorobenzyl group, a 3-chlorobenzyl group, a 4-chlorobenzyl group, a 2-bromobenzyl group, a 3-bromobenzyl group, a 4-bromobenzyl group, a 2-nitrobenzyl group, a 3-nitrobenzyl group, a 4-nitrobenzyl group and the like;
substituted or unsubstituted aryl groups having 6 to 20 carbon atoms such as a phenyl group, a 4-methylphenyl group, a 3-methylphenyl group, a 2-methylphenyl group, a 4-ethylphenyl group, a 3-ethylphenyl group, a 2-ethylphenyl group, a 4-n-propylphenyl group, a 4-isopropylphenyl group, a 2-isopropylphenyl group, a 4-n-butylphenyl group, a 4-isobutylphenyl group, a 4-sec-butylphenyl group, a 2-sec-butylphenyl group, a 4-tert-butylphenyl group, a 3-tert-butylphenyl group, a 2-tert-butylphenyl group, a 4-n-pentylphenyl group, a 4-isopentylphenyl group, a 4-neopentylphenyl group, a 4-tert-pentylphenyl group, a 4-n-hexylphenyl group, a 4-(2'-ethylbutyl)phenyl group, a 4-n-heptylphenyl group, a 4-n-octylphenyl group, a 4-(2'-ethylhexyl)phenyl group, a 4-n-nonylphenyl group, a 4-n-decylphenyl group, a 4-n-undecylphenyl group, a 4-n-dodecylphenyl group, a 4-n-tetradecylphenyl group, a 4-cyclohexylphenyl group, a 4-(4'-methylcyclohexyl)phenyl group, a 4-(4'-tert-butylcyclohexyl)phenyl group, a 3-cyclohexylphenyl group, a 2-cyclohexylphenyl group, a 2,3-dimethylphenyl group, a 2,4-dimethylphenyl group, a 2,5-dimethylphenyl group, a 2,6-dimethylphenyl group, a 3,4-dimethylphenyl group, a 3,5-dimethylphenyl group, a-3,4,5-trimethylphenyl group, a 2,3,5,6-tetramethylphenyl group, a 2,4-diethylphenyl group, a 2,6-diethylphenyl group, a 2,5-diisopropylphenyl group, a 2,6-diisopropylphenyl group, a 2,6-diisobutylphenyl group, a 2,4-di-tert-butylphenyl group, a 2,5-di-tert-butylphenyl group, a 4,6-di-tert-butyl-2-methylphenyl group, a 5-tert-butyl-2-methylphenyl group, a 4-tert-butyl-2,6-dimethylphenyl group, a 1-naphthyl group, a 2-naphthyl group, a 1,2,3,4-tetrahydro-5-naphthyl group, a 1,2,3,4-tetrahydro-6-naphthyl group, a 4-ethyl-1-naphthyl group, a 6-n-butyl-2-naphthyl group, a 5-indanyl group, a 4-methoxyphenyl group, a 3-methoxyphenyl group, a 2-methoxyphenyl group, a 4-ethoxyphenyl group, a 3-ethoxyphenyl group, a 2-ethoxyphenyl group, a 4-n-propyloxyphenyl group, a 3-n-propyloxyphenyl group, a 4-isopropyloxyphenyl group, a 2-isopropyloxyphenyl group, a 4-n-butyloxyphenyl group, a 4-isobutyloxyphenyl group, a 2-sec-butyloxyphenyl group, a 4-n-pentyloxyphenyl group, a 4-isopentyloxyphenyl group, a 2-isopentyloxyphenyl group, a 4-neopentyloxyphenyl group, a 2-neopentyloxyphenyl group, a 4-n-hexyloxyphenyl group, a 4-(2'-ethylbutyl)oxyphenyl group, a 4-n-heptyloxyphenyl group, a 4-n-octyloxyphenyl group, a 4-n-nonyloxyphenyl group, a 4-n-decyloxyphenyl group, a 4-n-undecyloxyphenyl group, a 4-n-dodecyloxyphenyl group, a 4-n-tetradecyloxyphenyl group, a 4-cyclohexyloxyphenyl group, a 2-cyclohexyloxyphenyl group, a 2,3-dimethoxyphenyl group, a 2,4-dimethoxyphenyl group, a 2,5-dimethoxyphenyl group, a 3,4-dimethoxyphenyl group, a 3,5-dimethoxyphenyl group, a 3,5-diethoxyphenyl group, a 2-methoxy-4-methylphenyl group, a 2-methoxy-5-methylphenyl group, a 2-methyl-4-methoxyphenyl group, a 3-methyl-4-methoxyphenyl group, a 3-methyl-5-methoxyphenyl group, a 2-methoxy-1-naphthyl group, a 4-methoxy-1-naphthyl group, a 4-n-butyloxy-1-naphthyl group, a 5-ethoxy-1-naphthyl group, a 6-methoxy-2-naphthyl group, a 6-ethoxy-2-naphthyl group, a 6-n-butyloxy-2-naphthyl group, a 6-n-hexyloxy-2-naphthyl group, a 7-methoxy-2-naphthyl group, a 7-n-butyloxy-2-naphthyl group, a 4-phenylphenyl group, a 3-phenylphenyl group, a 2-phenylphenyl group, a 4-(4'-methylphenyl)phenyl group, a 4-(3'-methylphenyl)phenyl group, a 4-(4'-ethylphenyl)phenyl group, a 4-(4'-isopropylphenyl)phenyl group, a 4-(4'-tert-butylphenyl)phenyl group, a 4-(4'-n-hexylphenyl)phenyl group, a 4-(4'-n-octylphenyl)phenyl group, a 4-(4'-methoxyphenyl)phenyl group, a 4-(4'-n-butyloxyphenyl)phenyl group, a 2-(2'-methoxyphenyl)phenyl group, a 4-(4'-chlorophenyl)phenyl group, a 3-methyl-4-phenylphenyl group, a 3-methoxy-4-phenylphenyl group, a 9-phenyl-2-fluorenyl group, a 9,9-diphenyl-2-fluorenyl group, a 9-methyl-9-phenyl-2-fluorenyl group, a 9-ethyl-9-phenyl-2-fluorenyl group, a 4-fluorophenyl group, a 3-fluorophenyl group, a 2-fluorophenyl group, a 4-chlorophenyl group, a 3-chlorophenyl group, a 2-chlorophenyl group, a 4-bromophenyl group, a 2-bromophenyl group, a 4-trifluoromethylphenyl group, a 2,3-difluorophenyl group, a 2,4-difluorophenyl group, a 2,5-difluorophenyl group, a 2,6-difluorophenyl group, a 3,4-difluorophenyl group, a 3,5-difluorophenyl group,- a 2,3-dichlorophenyl group, a 2,4-dichlorophenyl group, a 2,5-dichlorophenyl group, a 3,4-dichlorophenyl group, a 3,5-dichlorophenyl group, a 2,5-dibromophenyl group, a 2,4,6-trichlorophenyl group, a 2-fluoro-4-methylphenyl group, a 2-fluoro-5-methylphenyl group, a 3-fluoro-2-methylphenyl group, a 3-fluoro-4-methylphenyl group, a 2-methyl-4-fluorophenyl group, a 2-methyl-5-fluorophenyl group, a 3-methyl-4-fluorophenyl group, a 2-chloro-4-methylphenyl group, a 2-chloro-5-methylphenyl group, a 2-chloro-6-methylphenyl group, a 3-chloro-4-methylphenyl group, a 2-methyl-3-chlorophenyl group, a 2-methyl-4-chlorophenyl group, a 3-methyl-4-chlorophenyl group, a 2-chloro-4,6-dimethylphenyl group, a 2,4-dichloro-1-naphthyl group, a 1,6-dichloro-2-naphthyl group, a 2-methoxy-4-fluorophenyl group, a 3-methoxy-4-fluorophenyl group, a 2-fluoro-4-methoxyphenyl group, a 2-fluoro-4-ethoxyphenyl group, a 2-fluoro-6-methoxyphenyl group, a 3-fluoro-4-methoxyphenyl group, a 3-fluoro-4-ethoxyphenyl group, a 2-chloro-4-methoxyphenyl group, a 3-chloro-4-methoxyphenyl group, a 2-methoxy-5-chlorophenyl group, a 3-methoxy-4-chlorophenyl group, a 3-methoxy-6-chlorophenyl group, a 5-chloro-2,4-dimethoxyphenyl group, a 2-hydroxyphenyl group, a 3-hydroxyphenyl group, a 4-hydroxyphenyl group, a 2-nitrophenyl group, a 3-nitrophenyl group, a 4-nitrophenyl group, a 2-cyanophenyl group, a 3-cyanophenyl group, a 4-cyanophenyl group, a 2-methyl-5-nitrophenyl group, a 3,5-dinitrophenyl group, a 2-hydroxy-4-nitrophenyl group and the like;
substituted or unsubstituted hetero aryl groups having 5 to 10 carbon atoms such as a 4-pyridyl group, a 3-pyridyl group, a 2-pyridyl group, a 4-methyl-2-pyridyl group, a 5-methyl-2-pyridyl group, a 6-methyl-2-pyridyl group, a 4,6-dimethyl-2-pyridyl group, a 4-methyl-5-nitro-2-pyridyl group, a 3-hydroxy-2-pyridyl group, a 6-fluoro-3-pyridyl group, a 6-methoxy-3-pyridyl group, a 6-methoxy-2-pyridyl group, a 2-pyrimidyl group, a 4-pyrimidyl group, a 5-pyrimidyl group, a 2,6-dimethyl-4-pyrimidyl group, a 4-quinolyl group, a 3-quinolyl group, a 4-methyl-2-quinolyl group, a 3-pyrrolyl group, a 2-pyrrolyl group, a 1-methyl-3-pyrrolyl group, a 1-methyl-2-pyrrolyl group, a 3-furyl group, a 2-furyl group, a 3-thienyl group, a 2-thienyl group, a 4-methyl-3-thienyl group, a 5-methyl-2-thienyl group, a 3-methyl-2-thienyl group, a 2-oxazolyl group, a 3-isoxazolyl group, a 2-thiazolyl group, a 2-thiadiazolyl group, a 2-benzoxazolyl group, a 2-benzothiazolyl group, a 2-benzimidazolyl group and the like;
substituted or unsubstituted alkenyl groups having 2 to 6 carbon atoms such as a vinyl group, a propenyl group, a butenyl group, a pentenyl group, a hexenyl group, a cyclopentenyl group, a cyclohexenyl group and the like;
substituted or unsubstituted alkynyl groups having 2 to 9 carbon atoms such as an ethynyl group, a 2-tert-butylethynyl group, a phenylethynyl group, a 4-methylphenylethynyl group, a 4-methoxyphenylethynyl group and the like;
substituted or unsubstituted metalocenyl groups such as a ferrocenyl group, a cobaltocenyl group, a nickelocenyl group, a dichlorotitanocenyl group, a trichlorotitaniumcyclopentadienyl group, a bis(trifluoromethanesulfonato)titanocenyl group, a dichlorozirconocenyl group, a dimethylzirconocenyl group, a diethoxyzirconocenyl group, a bis(cyclopentadienyl)chromium group, a bis(cyclopentadienyl)dichloromolybdenum group, a bis(cyclopentadienyl)dichlorohafnium group, a bis(cyclopentadienyl)dichloroniobium group, a bis(cyclopentadienyl)ruthenium group, a bis(cyclopentadienyl)vanadium group, a bis(cyclopentadienyl)dichlorovanadium group, an octamethylferrocenyl group, an octamethylcobaltocenyl group, an octamethylnickelocenyl group and the like;
substituted or unsubstituted alkoxy groups having 1 to 8 carbon atoms such as a methoxy group, an ethoxy group, an n-propoxy group, an isopropoxy group, an n-butoxy group, a tert-butoxy group, a sec-butoxy group, an n-pentyloxy group, an iso-pentyloxy group, an n-hexyloxy group, an n-octyloxy group, a methoxymethoxy group, an ethoxyethoxy group, a 3-(iso-propyloxy)propyloxy group and the like;
substituted or unsubstituted aralkyloxy groups having 7 to 16 carbon atoms such as a benzyloxy group, a 4-nitrobenzyloxy group, a 4-cyanobenzyloxy group, a 4-hydroxybenzyloxy group, a 2-methylbenzyloxy group, a 3-methylbenzyloxy group, a 4-methylbenzyloxy group, a 4-trifluoromethylbenzyloxy group, a 1-naphthylmethoxy group, a 2-naphthylmethoxy group, a 4-cyano-1-naphthylmethoxy group, a 4-hydroxy-1-naphthylmethoxy group, a 6-hydroxy-2-naphthylmethoxy group, a 4-methyl-1-naphthylmethoxy group, a 6-methyl-2-naphthylmethoxy group, a 4-trifluoromethyl-1-naphthylmethoxy group, a fluorene-9-ylethoxy group and the like;
substituted or unsubstituted aryloxy groups having 6 to 10 carbon atoms such as a phenoxy group, a 2-methylphenoxy group, a 4-methylphenoxy group, a 4-tert-butylphenoxy group, a 2-methoxyphenoxy group, a 4-isopropylphenoxy group, a naphthyloxy group and the like;
substituted or unsubstituted hetero aryloxy groups having 5 to 10 carbon atoms such as a 4-pyridyloxy group, a 3-pyridyloxy group, a 2-pyridyloxy group, a 4-methyl-2-pyridyloxy group, a 4-methyl-5-nitro-2-pyridyloxy group, a 3-hydroxy-2-pyridyloxy group, a 6-fluoro-3-pyridyloxy group, a 6-methoxy-3-pyridyloxy group, a 2-pyrimidyloxy group, a 4-pyrimidyloxy group, a 5-pyrimidyloxy group, a 2,6-dimethyl-4-pyrimidyloxy group, a 4-quinolyloxy group, a 3-quinolyloxy group, a 4-methyl-2-quinolyloxy group, a 3-pyrrolyloxy group, a 2-pyrrolyloxy group, a 1-methyl-3-pyrrolyloxy group, a 1-methyl-2-pyrrolyloxy group, a 3-furyloxy group, a 2-furyloxy group, a 3-thienyloxy group, a 2-thienyloxy group, a 4-methyl-3-thienyloxy group, a 2-oxazolyloxy group, a 3-isoxazolyloxy group, a 2-thiazolyloxy group, a 2-thiadiazolyloxy group, a 2-benzoxazolyloxy group, a 2-benzothiazolyloxy group, a 2-benzimidazolyloxy group and the like;
substituted or unsubstituted acyloxy groups having 1 to 8 carbon atoms such as a formyloxy group, a methylcarbonyloxy group, an ethylcarbonyloxy group, an n-propylcarbonyloxy group, an isopropylcarbonyloxy group, an n-butylcarbonyloxy group, an isobutylcarbonyloxy group, a sec-butylcarbonyloxy group, a tert-butylcarbonyloxy group, an n-pentylcarbonyloxy group, an isopentylcarbonyloxy group, a neopentylcarbonyloxy group, a 2-methylbutylcarbonyloxy group, a 4-nitrobenzylcarbonyloxy group and the like;
substituted or unsubstituted arylcarbonyloxy groups having 7 to 11 carbon atoms such as a phenylcarbonyloxy group, a 4-methylphenylcarbonyloxy group, a 4-chlorophenylcarbonyloxy group, a 4-bromophenylcarbonyloxy group, a naphthylcarbonyloxy group and the like;
substituted or unsubstituted alkylthio groups having 1 to 6 carbon atoms such as a methylthio group, an ethylthio group, an n-propylthio group, a tert-butylthio group, a sec-butylthio group, an n-pentylthio group, an n-hexylthio group and the like;
substituted or unsubstituted aralkylthio groups having 7 to 16 carbon atoms such as a benzylthio group, a 4-cyanobenzylthio group, a 4-hydroxybenzylthio group, a 2-methylbenzylthio group, a 3-methylbenzylthio group, a 4-methylbenzylthio group, a 4-trifluoromethylbenzylthio group, a 1-naphthylmethylthio group, a 4-nitro-1-naphthylmethylthio group, a 4-cyano-1-naphthylmethylthio group, a 4-hydroxy-1-naphthylmethylthio group, a 4-methyl-1-naphthylmethylthio group, a 4-trifluoromethyl-1-naphthylmethylthio group, a fluorene-9-ylethylthio group and the like;
substituted or unsubstituted arylthio groups having 6 to 10 carbon atoms such as a phenylthio group, a 4-methylphenylthio group, a 2-methoxyphenylthio group, a 4-tert-butylphenylthio group, a naphthylthio group and the like;
substituted or unsubstituted hetero arylthio groups having 5 to 10 carbon atoms such as a 4-pyridylthio group, a 3-pyridylthio group, a 2-pyridylthio group, a 4-methyl-2-pyridylthio group, a 4-methyl-5-nitro-2-pyridylthio group, a 3-hydroxy-2-pyridylthio group, a 6-fluoro-3-pyridylthio group, a 6-methoxy-3-pyridylthio group, a 2-pyrimidylthio group, a 4-pyrimidylthio group, a 5-pyrimidylthio group, a 2,6-dimethyl-4-pyrimidylthio group, a 4-quinolylthio group, a 3-quinolylthio group, a 4-methyl-2-quinolylthio group, a 3-pyrrolylthio group, a 2-pyrrolylthio group, a 1-methyl-3-pyrrolylthio group, a 1-methyl-2-pyrrolylthio group, a 3-furylthio group, a 2-furylthio group, a 3-thienylthio group, a 2-thienylthio group, a 4-methyl-3-thienylthio group, a 2-oxazolylthio group, a 3-isoxazolylthio group, a 2-thiazolylthio group, a 2-thiadiazolylthio group, a 2-benzoxazolylthio group, a 2-benzothiazolylthio group, a 2-benzimidazolylthio group and the like;
substituted or unsubstituted acylthio groups having 2 to 8 carbon atoms such as a methylcarbonylthio group, an ethylcarbonylthio group, an n-propylcarbonylthio group, an isopropylcarbonylthio group, an n-butylcarbonylthio group, an isobutylcarbonylthio group, a sec-butylcarbonylthio group, a tert-butylcarbonylthio group, an n-pentylcarbonylthio group, an isopentylcarbonylthio group, a neopentylcarbonylthio group, a 2-methylbutylcarbonylthio group, a 4-nitrobenzylcarbonylthio group and the like;
substituted or unsubstituted arylcarbonylthio groups having 7 to 11 carbon atoms such as a phenylcarbonylthio group, a 4-methylphenylcarbonylthio group, a 4-chlorophenylcarbonylthio group, a 4-bromophenylcarbonylthio group, a naphthylcarbonylthio group and the like;
substituted or unsubstituted alkylsulfonyl groups having 2 to 7 carbon atoms such as a methylsulfonyl group, an ethylsulfonyl group, an n-propylsulfonyl group, an isopropylsulfonyl group, an n-butylsulfonyl group, a tert-butylsulfonyl group, a sec-butylsulfonyl group, an n-pentylsulfonyl group, an n-hexylsulfonyl group and the like;
substituted or unsubstituted arylsulfonyl groups having 6 to 10 carbon atoms such as a phenylsulfonyl group, a 2-methylphenylsulfonyl group, a 4-methylphenylsulfonyl group, a 4-tert-butylphenylsulfonyl group, a 2-methoxyphenylsulfonyl group, a 4-isopropylphenylsulfonyl group, a naphthylsulfonyl group and the like;
substituted or unsubstituted alkylamino groups having 1 to 8 carbon atoms such as a methylamino group, an ethylamino group, an n-propylamino group, an n-butylamino group, a dimethylamino group, a diethylamino group, a di-n-propylamino group, a di-n-butylamino group and the like;
substituted or unsubstituted arylamino groups having 6 to 14 carbon atoms such as a phenylamino group, a 4-methylphenylamino group, a 4-chlorophenylamino group, a 4-bromophenylamino group, a diphenylamino group, a di(4-methylphenyl)amino group, a di(4-chlorophenyl)amino group, a di(4-bromophenyl)amino group and the like;
substituted or unsubstituted hetero arylamino groups having 5 to 10 carbon atoms such as a 4-pyridylamino group, a 3-pyridylamino group, a 2-pyridylamino group, a 4-methyl-2-pyridylamino group, a 5-methyl-2-pyridylamino group, a 6-methyl-2-pyridylamino group, a 4,6-dimethyl-2-pyridylamino group, a 4-methyl-5-nitro-2-pyridylamino group, a 3-hydroxy-2-pyridylamino group, a 6-fluoro-3-pyridylamino group, a 6-methoxy-3-pyridylamino group, a 6-methoxy-2-pyridylamino group, a 2-pyrimidylamino group, a 4-pyrimidylamino group, a 5-pyrimidylamino group, a 2,6-dimethyl-4-pyrimidylamino group, a 4-quinolylamino group, a 3-quinolylamino group, a 4-methyl-2-quinolylamino group, a 3-furylamino group, a 2-furylamino group, a 3-thienylamino group, a 2-thienylamino group, a 4-methyl-3-thienylamino group, a 5-methyl-2-thienylamino group, a 3-methyl-2-thienylamino group, a 2-oxazolylamino group, a 3-isoxazolylamino group, a 2-thiazolylamino group, a 2-thiadiazolylamino group, a 2-benzoxazolylamino group, 2-benzothiazolylamino group, a 2-benzimidazolylamino group and the like;
substituted or unsubstituted alkylcarbonylamino groups having 2 to 7 carbon atoms such as a methylcarbonylamino group, an ethylcarbonylamino group, an n-propylcarbonylamino group, an isopropylcarbonylamino group, an n-butylcarbonylamino group, a tert-butylcarbonylamino group, a sec-butylcarbonylamino group, an n-pentylcarbonylamino group, an n-hexylcarbonylamino group and the like;
substituted or unsubstituted arylcarbonylamino groups having 7 to 11 carbon atoms such as a phenylcarbonylamino group, a 2-methylphenylcarbonylamino group, a 4-methylphenylcarbonylamino group, a 4-tert-butylphenylcarbonylamino group, a 2-methoxyphenylcarbonylamino group, a 4-isopropylphenylcarbonylamino group, a naphthylcarbonylamino group and the like; and
a group having a substituted or unsubstituted metalocenyl groups composed of a linking group and a substituted or unsubstituted metalocenyl group.

As the group having a substituted or unsubstituted metalocenyl group, preferred is a group represented by the general formula (7) below,

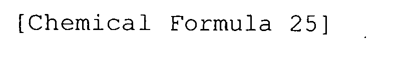

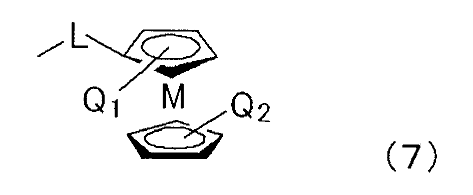

wherein, in the formula, L represents a linking group; Q₁ and Q₂ each independently represent a hydrogen atom, a halogen atom, an alkyl group having 1 to 4 carbon atoms, an alkoxy group having 1 to 4 carbon atoms, an aminoalkyl group having 1 to 4 carbon atoms or a diarylphosphino group; and M represents a divalent transition metal.

In the group represented by the general formula (7), Q₁ and Q₂ each independently represent a hydrogen atom, a halogen atom, an alkyl group having 1 to 4 carbon atoms, an alkoxy group having 1 to 4 carbon atoms, an aminoalkyl group having 1 to 4 carbon atoms or a diarylphosphino group.

Concrete examples of Q₁ and Q₂ include a hydrogen atom;
halogen atoms such as a fluorine atom, a chlorine atom, a bromine atom, an iodine atom and the like;
alkyl groups such as a methyl group, an ethyl group, an n-propyl group, an isopropyl group, an n-butyl group, an isobutyl group, a sec-butyl group, a tert-butyl group and the like;
alkoxy groups such as a methoxy group, an ethoxy group, an n-propyloxy group, an isopropyloxy group, an n-butyloxy group, an isobutyloxy group, a sec-butyloxy group, a tert-butyloxy group and the like;
aminoalkyl groups such as an aminomethyl group, an aminoethyl group, an aminopropyl group, an aminobutyl group and the like; and
diarylphosphino groups such as a diphenylphosphino group, a phenyl-3,5-xylylphosphino group and the like.

In the group represented by the general formula (7) , M represents a divalent transition metal, and concrete examples thereof include Fe, Co, Ni, Ru, Os, Mn, Cr, W, V, Sc, Y, La, Ce, Pr, Nd, Sm, Gd, Er, Tm, Yb and the like. Particularly preferred is Fe.

In the group represented by the general formula (7), L represents a linking group, and preferably a divalent residue which may contain a hetero atom having 1 to 10 carbon atoms.

When ring A may have substituents, as the substituent, preferred is a substituent having a fluorine atom and more preferred is a fluorine-substituted alkyl group from the viewpoints of the recording sensitivity and the stability against reproduction light.

Concrete examples of the fluorine-substituted alkyl group include a trifluoromethyl group, a 2,2,2-trifluoroethyl group, a pentafluoroethyl group, a 3,3,3-trifluoropropyl group, a 2,2,3,3-tetrafluoropropyl group, a 4,4,4-trifluorobutyl group, a 2,2,3,3,4,4,4-heptafluorobutyl group and the like.

In the linking group represented by the general formula (a), X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group.

X preferably represents a hydrogen atom, a hydroxyl group, an amino group, a carboxyl group, a substituted or unsubstituted alkoxycarbonyl group having 2 to 7 carbon atoms, a substituted or unsubstituted aralkyloxycarbonyl group having 8 to 16 carbon atoms, a substituted or unsubstituted aryloxycarbonyl group having 7 to 11 carbon atoms, a substituted or unsubstituted acyl group having 1 to 7 carbon atoms, a substituted or unsubstituted arylcarbonyl group having 7 to 11 carbon atoms, a substituted or unsubstituted hetero arylcarbonyl group having 6 to 11 carbon atoms, a substituted or unsubstituted alkyl group having 1 to 10 carbon atoms, a substituted or unsubstituted aralkyl group having 7 to 16 carbon atoms, a substituted or unsubstituted aryl group having 6 to 20 carbon atoms, a substituted or unsubstituted hetero aryl group having 5 to 10 carbon atoms, a substituted or unsubstituted alkenyl group having 2 to 6 carbon atoms, a substituted or unsubstituted alkynyl group having 2 to 9 carbon atoms, a substituted or unsubstituted metalocenyl group, a substituted or unsubstituted alkoxy group having 1 to 8 carbon atoms, a substituted or unsubstituted aralkyloxy group having 7 to 16 carbon atoms, a substituted or unsubstituted aryloxy group having 6 to 10 carbon atoms, a substituted or unsubstituted hetero aryloxy group having 5 to 10 carbon atoms, a substituted or unsubstituted acyloxy group having 1 to 8 carbon atoms, a substituted or unsubstituted alkylthio group having 1 to 6 carbon atoms, a substituted or unsubstituted aralkylthio group having 7 to 16 carbon atoms, a substituted or unsubstituted arylthio group having 6 to 10 carbon atoms, a substituted or unsubstituted hetero arylthio group having 5 to 10 carbon atoms, a substituted or unsubstituted alkylamino group having 1 to 8 carbon atoms, a substituted or unsubstituted arylamino group having 6 to 14 carbon atoms, a substituted or unsubstituted hetero arylamino group having 5 to 10 carbon atoms, a substituted or unsubstituted alkylcarbonylamino group having 2 to 7 carbon atoms, a substituted or unsubstituted arylcarbonylamino group having 7 to 11 carbon atoms, or a group having a substituted or unsubstituted metalocenyl group.

Concrete example of X include a hydrogen atom;
a hydroxyl group;
an amino group;
a carboxyl group;
concrete examples of the substituted or unsubstituted alkoxycarbonyl group having 2 to 7 carbon atoms as cited above; concrete examples of the substituted or unsubstituted aralkyloxycarbonyl group having 8 to 16 carbon atoms as cited above;
concrete examples of the substituted or unsubstituted aryloxycarbonyl group having 7 to 11 carbon atoms as cited above;
concrete examples of the substituted or unsubstituted acyl group having 1 to 7 carbon atoms as cited above;
concrete examples of the substituted or unsubstituted arylcarbonyl group having 7 to 11 carbon atoms as cited above;
concrete examples of the substituted or unsubstituted hetero arylcarbonyl group having 6 to 11 carbon atoms as cited above;
concrete examples of the substituted or unsubstituted alkyl group having 1 to 10 carbon atoms as cited above;
concrete examples of the substituted or unsubstituted aralkyl group having 7 to 16 carbon atoms as cited above;
concrete examples of the substituted or unsubstituted aryl group having 6 to 20 carbon atoms as cited above;
concrete examples of the substituted or unsubstituted hetero aryl group having 5 to 10 carbon atoms as cited above; concrete examples of the substituted or unsubstituted alkenyl group having 2 to 6 carbon atoms as cited above;
concrete examples of the substituted or unsubstituted alkynyl group having 2 to 9 carbon atoms as cited above;
concrete examples of the substituted or unsubstituted metalocenyl group as cited above;
concrete examples of the substituted or unsubstituted alkoxy group having 1 to 8 carbon atoms as cited above;
concrete examples of the substituted or unsubstituted aralkyloxy group having 7 to 16 carbon atoms as cited above;
concrete examples of the substituted or unsubstituted aryloxy group having 6 to 10 carbon atoms as cited above;
concrete examples of the substituted or unsubstituted hetero aryloxy group having 5 to 10 carbon atoms as cited above;
concrete examples of the substituted or unsubstituted acyloxy group having 1 to 8 carbon atoms as cited above;
concrete examples of the substituted or unsubstituted alkylthio group having 1 to 6 carbon atoms as cited above;
concrete examples of the substituted or unsubstituted aralkylthio group having 7 to 16 carbon atoms as cited above;
concrete examples of the substituted or unsubstituted arylthio group having 6 to 10 carbon atoms as cited above;
concrete examples of the substituted or unsubstituted hetero arylthio group having 5 to 10 carbon atoms as cited above;
concrete examples of the substituted or unsubstituted alkylamino group having 1 to 8 carbon atoms as cited above;
concrete examples of the substituted or unsubstituted arylamino group having 6 to 14 carbon atoms as cited above;
concrete examples of the substituted or unsubstituted hetero arylamino group having 5 to 10 carbon atoms as cited above;
concrete examples of the substituted or unsubstituted alkylcarbonylamino group having 2 to 7 carbon atoms as cited above;
concrete examples of the substituted or unsubstituted arylcarbonylamino group having 7 to 11 carbon atoms as cited above; and
concrete examples of the group having a substituted or unsubstituted metalocenyl group as cited above.

In the compound represented by the general formula (1), R₁ and R₂ each independently represent a hydrogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, a substituted or unsubstituted hetero arylcarbonyl group, a substituted or unsubstituted alkylaminothiocarbonyl group, a substituted or unsubstituted arylaminothiocarbonyl group, a substituted or unsubstituted alkylthiocarbonyl group, a substituted or unsubstituted arylthiocarbonyl group, a substituted or unsubstituted hetero arylthiocarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted arylcarbonyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted acylthio group, a substituted or unsubstituted arylcarbonylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group, and may be bonded with each other to form a hydrocarbon ring which may have a substituent or a heterocyclic ring which may have a substituent.

R₁ and R₂ are each preferably a hydrogen atom, a hydroxyl group, an amino group, a nitro group, a cyano group, a carboxyl group, a substituted or unsubstituted alkoxycarbonyl group having 2 to 7 carbon atoms, a substituted or unsubstituted aralkyloxycarbonyl group having 8 to 16 carbon atoms, a substituted or unsubstituted aryloxycarbonyl group having 7 to 11 carbon atoms, a substituted or unsubstituted alkylaminocarbonyl group having 2 to 9 carbon atoms, a substituted or unsubstituted arylaminocarbonyl group having 7 to 15 carbon atoms, a substituted or unsubstituted acyl group having 1 to 7 carbon atoms, a substituted or unsubstituted arylcarbonyl group having 7 to 11 carbon atoms, a substituted or unsubstituted hetero arylcarbonyl group having 6 to 11 carbon atoms, a substituted or unsubstituted alkylaminothiocarbonyl group having 2 to 9 carbon atoms, a substituted or unsubstituted arylaminothiocarbonyl group having 7 to 15 carbon atoms, a substituted or unsubstituted alkylthiocarbonyl group having 2 to 7 carbon atoms, a substituted or unsubstituted arylthiocarbonyl group having 7 to 11 carbon atoms, a substituted or unsubstituted hetero arylthiocarbonyl group having 6 to 11 carbon atoms, a substituted or unsubstituted alkyl group having 1 to 10 carbon atoms, a substituted or unsubstituted aralkyl group having 7 to 16 carbon atoms, a substituted or unsubstituted aryl group having 6 to 20 carbon atoms, a substituted or unsubstituted hetero aryl group having 5 to 10 carbon atoms, a substituted or unsubstituted alkenyl group having 2 to 6 carbon atoms, a substituted or unsubstituted alkynyl group having 2 to 9 carbon atoms, a substituted or unsubstituted metalocenyl group, a substituted or unsubstituted alkoxy group having 1 to 8 carbon atoms, a substituted or unsubstituted aralkyloxy group having 7 to 16 carbon atoms, a substituted or unsubstituted aryloxy group having 6 to 10 carbon atoms, a substituted or unsubstituted hetero aryloxy group having 5 to 10 carbon atoms, a substituted or unsubstituted acyloxy group having 1 to 8 carbon atoms, a substituted or unsubstituted arylcarbonyloxy group having 7 to 11 carbon atoms, a substituted or unsubstituted alkylthio group having 1 to 6 carbon atoms, a substituted or unsubstituted aralkylthio group having 7 to 16 carbon atoms, a substituted or unsubstituted arylthio group having 6 to 10 carbon atoms, a substituted or unsubstituted hetero arylthio group having 5 to 10 carbon atoms, a substituted or unsubstituted acylthio group having 2 to 8 carbon atoms, a substituted or unsubstituted arylcarbonylthio group having 7 to 11 carbon atoms, a substituted or unsubstituted alkylamino group having 1 to 8 carbon atoms, a substituted or unsubstituted arylamino group having 6 to 14 carbon atoms, a substituted or unsubstituted hetero arylamino group having 5 to 10 carbon atoms, a substituted or unsubstituted alkylcarbonylamino group having 2 to 7 carbon atoms, a substituted or unsubstituted arylcarbonylamino group having 7 to 11 carbon atoms, or a group having a substituted or unsubstituted metalocenyl group.

R₁ and R₂ may be bonded with each other to form a hydrocarbon ring which may have a substituent or a heterocyclic ring which may have a substituent.

Concrete examples of R₁ and R₂ include a hydrogen atom;
a hydroxyl group;
an amino group;
a nitro group;
a cyano group;
a carboxyl group;
concrete examples of the substituted or unsubstituted alkoxycarbonyl group having 2 to 7 carbon atoms as cited above;
concrete examples of the substituted or unsubstituted aralkyloxycarbonyl group having 8 to 16 carbon atoms as cited above;
concrete examples of the substituted or unsubstituted aryloxycarbonyl group having 7 to 11 carbon atoms as cited above;
concrete examples of the substituted or unsubstituted alkylaminocarbonyl group having 2 to 9 carbon atoms as cited above;
concrete examples of the substituted or unsubstituted alkylaminocarbonyl group having 7 to 15 carbon atoms as cited above;
concrete examples of the substituted or unsubstituted acyl group having 1 to 7 carbon atoms as cited above;
concrete examples of the substituted or unsubstituted arylcarbonyl group having 7 to 11 carbon atoms as cited above;
concrete examples of the substituted or unsubstituted hetero arylcarbonyl group having 6 to 11 carbon atoms as cited above;
concrete examples of the substituted or unsubstituted alkylaminothiocarbonyl group having 2 to 9 carbon atoms as cited above;
concrete examples of the substituted or unsubstituted arylaminothiocarbonyl group having 7 to 15 carbon atoms as cited above;
concrete examples of the substituted or unsubstituted alkylthiocarbonyl group having 2 to 7 carbon atoms as cited above;
concrete examples of the substituted or unsubstituted arylthiocarbonyl group having 7 to 11 carbon atoms as cited above;
concrete examples of the substituted or unsubstituted hetero arylthiocarbonyl group having 6 to 11 carbon atoms as cited above;
concrete examples of the substituted or unsubstituted alkyl group having 1 to 10 carbon atoms as cited above;
concrete examples of the substituted or unsubstituted aralkyl group having 7 to 16 carbon atoms as cited above;
concrete examples of the substituted or unsubstituted aryl group having 6 to 20 carbon atoms as cited above;
concrete examples of the substituted or unsubstituted hetero aryl group having 5 to 10 carbon atoms as cited above;
concrete examples of the substituted or unsubstituted alkenyl group having 2 to 6 carbon atoms as cited above;
concrete examples of the substituted or unsubstituted alkynyl group having 2 to 9 carbon atoms as cited above;
concrete examples of the a substituted or unsubstituted metalocenyl group as cited above;
concrete examples of the substituted or unsubstituted alkoxy group having 1 to 8 carbon atoms as cited above;
concrete examples of the substituted or unsubstituted aralkyloxy group having 7 to 16 carbon atoms as cited above;
concrete examples of the substituted or unsubstituted aryloxy group having 6 to 10 carbon atoms as cited above;
concrete examples of the substituted or unsubstituted hetero aryloxy group having 5 to 10 carbon atoms as cited above;
concrete examples of the substituted or unsubstituted acyloxy group having 1 to 8 carbon atoms as cited above;
concrete examples of the substituted or unsubstituted arylcarbonyloxy group having 7 to 11 carbon atoms as cited above;
concrete examples of the substituted or unsubstituted alkylthio group having 1 to 6 carbon atoms as cited above;
concrete examples of the substituted or unsubstituted aralkylthio group having 7 to 16 carbon atoms as cited above;
concrete examples of the substituted or unsubstituted arylthio group having 6 to 10 carbon atoms as cited above;
concrete examples of the substituted or unsubstituted hetero arylthio group having 5 to 10 carbon atoms as cited above;
concrete examples of the substituted or unsubstituted acylthio group having 2 to 8 carbon atoms as cited above;
concrete examples of the substituted or unsubstituted arylcarbonylthio group having 7 to 11 carbon atoms as cited above;
concrete examples of the substituted or unsubstituted alkylamino group having 1 to 8 carbon atoms as cited above;
concrete examples of the substituted or unsubstituted arylamino group having 6 to 14 carbon atoms as cited above;
concrete examples of the substituted or unsubstituted hetero arylamino group having 5 to 10 carbon atoms as cited above;
concrete examples of the substituted or unsubstituted alkylcarbonylamino group having 2 to 7 carbon atoms as cited above;
concrete examples of the substituted or unsubstituted arylcarbonylamino group having 7 to 11 carbon atoms as cited above; and
concrete examples of the group having a substituted or unsubstituted metalocenyl group as cited above.

When R₁ and R₂ are bonded with each other to form a hydrocarbon ring which may have a substituent or a heterocyclic ring which may have a substituent, preferred are a substituted or unsubstituted aliphatic ring, a substituted or unsubstituted carbocyclic aromatic ring and a substituted or unsubstituted heterocyclic aromatic ring, more preferred are a substituted or unsubstituted aliphatic ring having 3 to 26 carbon atoms, a substituted or unsubstituted carbocyclic aromatic ring having 6 to 26 carbon atoms and a substituted or unsubstituted heterocyclic aromatic ring having 3 to 26 carbon atoms, and further preferred are a substituted or unsubstituted aliphatic ring having 3 to 20 carbon atoms, a substituted or unsubstituted carbocyclic aromatic ring having 6 to 20 carbon atoms and a substituted or unsubstituted heterocyclic aromatic ring having 3 to 20 carbon atoms.

Particularly preferred are a 6-membered cyclic hydrocarbon ring which may have a substituent, a 6-membered cyclic heterocyclic ring which may have a substituent, a 5-membered cyclic hydrocarbon ring which may have a substituent, a 5-membered cyclic heterocyclic ring which may have a substituent and the like.

In the above, concrete examples of the atom constituting each ring include C, N, O, S and the like. Furthermore, when ring has substituents, concrete examples of the substituent include the same as those of the substituent when ring A may have substituents.

Concrete examples of the 6-membered cyclic hydrocarbon ring which may have a substituent, 6-membered cyclic heterocyclic ring which may have a substituent, 5-membered cyclic hydrocarbon ring which may have a substituent and 5-membered cyclic heterocyclic ring which may have a substituent include the formulae below,

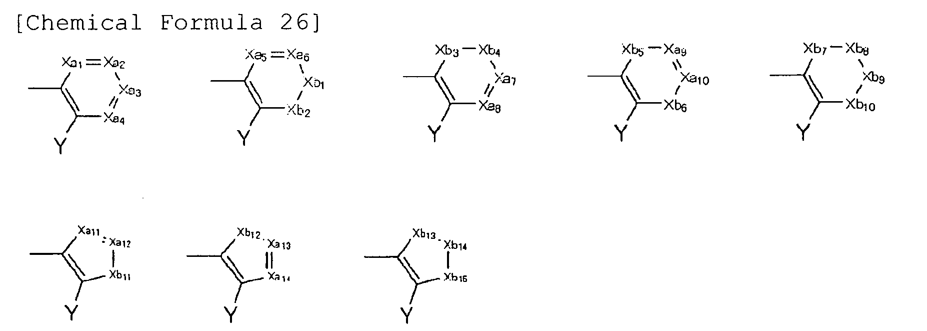

wherein, in the formula, Xₐ₁ to Xₐ₁₄ each independently represent -CZ₁= or -N=; and X_{b1} to X_{b15} each independently represent -CZ₁Z₂-, -C(=O)-, -C(=S)-, -NZ₁-, -O- or -S-, may be the same or different from each other, and adjacent ones thereof may be bonded with each other to form a ring;
Z₁ and Z₂ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylsulfonyl group, a substituted or unsubstituted arylsulfonyl group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group, and
Z₁ and Z₂ may be bonded with each other to form a 5 to 6-membered ring; and Y represents the same as those described above.

More preferable examples of the 6-membered cyclic hydrocarbon ring which may have a substituent, 6-membered cyclic heterocyclic ring which may have a substituent, 5-membered cyclic hydrocarbon ring which may have a substituent and 5-membered cyclic heterocyclic ring which may have a substituent include the general formulae - (C-1), to (C-7) bellow,

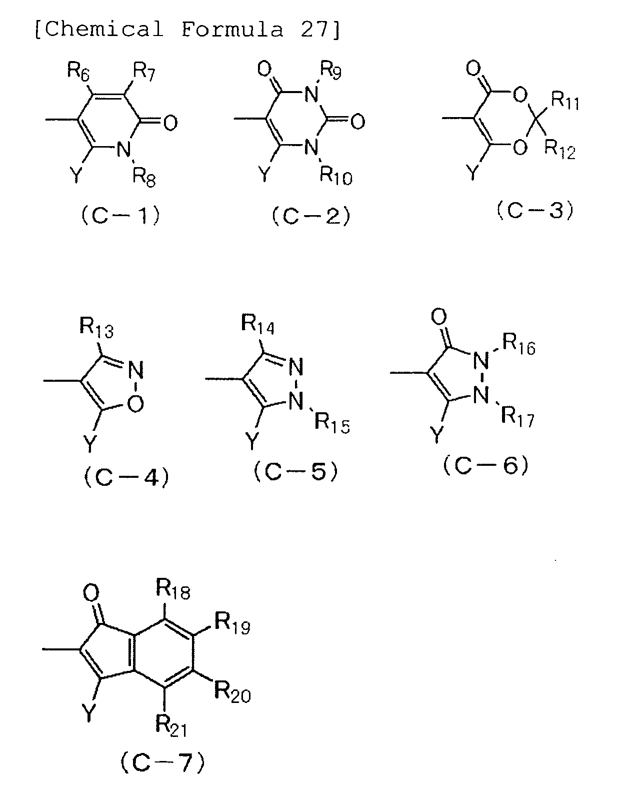

wherein, in the formula, R₆ to R₂₁ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylsulfonyl group, a substituted or unsubstituted arylsulfonyl group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
R₁₁ and R₁₂ may be bonded with each other to form a 5 to 6-membered ring; and Y represents the same as those described above.

Examples of R₆ to R₂₁ of the general formulae (C-1) to (C-7) include a hydrogen atom, a halogen atom, a hydroxyl group, an amino group, a nitro group, a cyano group, a carboxyl group, a substituted or unsubstituted alkoxycarbonyl group having 2 to 7 carbon atoms, a substituted or unsubstituted aralkyloxycarbonyl group having 8 to 16 carbon atoms, a substituted or unsubstituted aryloxycarbonyl group having 7 to 11 carbon atoms, a substituted or unsubstituted acyl group having 1 to 7 carbon atoms, a substituted or unsubstituted arylcarbonyl group having 7 to 11 carbon atoms, a substituted or unsubstituted hetero arylcarbonyl group having 6 to 11 carbon atoms, a substituted or unsubstituted alkyl group having 1 to 10 carbon atoms, a substituted or unsubstituted aralkyl group having 7 to 16 carbon atoms, a substituted or unsubstituted aryl group having 6 to 20 carbon atoms, a substituted or unsubstituted hetero aryl group having 5 to 10 carbon atoms, a substituted or unsubstituted alkenyl group having 2 to 6 carbon atoms, a substituted or unsubstituted alkynyl group having 2 to 9 carbon atoms, a substituted or unsubstituted metalocenyl group, a substituted or unsubstituted alkoxy group having 1 to 8 carbon atoms, a substituted or unsubstituted aralkyloxy group having 7 to 16 carbon atoms, a substituted or unsubstituted aryloxy group having 6 to 10 carbon atoms, a substituted or unsubstituted hetero aryloxy group having 5 to 10 carbon atoms, a substituted or unsubstituted acyloxy group having 1 to 8 carbon atoms, a substituted or unsubstituted alkylthio group having 1 to 6 carbon atoms, a substituted or unsubstituted aralkylthio group having 7 to 16 carbon atoms, a substituted or unsubstituted arylthio group having 6 to 10 carbon atoms, a substituted or unsubstituted hetero arylthio group having 5 to 10 carbon atoms, a substituted or unsubstituted alkylsulfonyl group having 2 to 7 carbon atoms, a substituted or unsubstituted arylsulfonyl group having 6 to 10 carbon atoms, a substituted or unsubstituted alkylamino group having 1 to 8 carbon atoms, a substituted or unsubstituted arylamino group having 6 to 14 carbon atoms, a substituted or unsubstituted hetero arylamino group having 5 to 10 carbon atoms, a substituted or unsubstituted alkylcarbonylamino group having 2 to 7 carbon atoms, a substituted or unsubstituted arylcarbonylamino group having 7 to 11 carbon atoms, and a group having a substituted or unsubstituted metalocenyl group, and R₁₁ and R₁₂ are preferably bonded with each other to form a 5 to 6-membered ring.

Concrete examples of R₆ to R₂₁ include a hydrogen atom;
halogen atoms such as a fluorine atom, a chlorine atom, a bromine atom, an iodine atom and the like;
a hydroxyl group;
an amino group;
a nitro group;
a cyano group;
a carboxyl group;
concrete examples of the substituted or unsubstituted alkoxycarbonyl group having 2 to 7 carbon atoms as cited above;
concrete examples of the substituted or unsubstituted aralkyloxycarbonyl group having 8 to 16 carbon atoms as cited above;
concrete examples of the substituted or unsubstituted aryloxycarbonyl group having 7 to 11 carbon atoms as cited above;
concrete examples of the substituted or unsubstituted acyl group having 1 to 7 carbon atoms as cited above;
concrete examples of the substituted or unsubstituted arylcarbonyl group having 7 to 11 carbon atoms as cited above;
concrete examples of the substituted or unsubstituted hetero arylcarbonyl group having 6 to 11 carbon atoms as cited above;
concrete examples of the substituted or unsubstituted alkyl group having 1 to 10 carbon atoms as cited above;
concrete examples of the substituted or unsubstituted aralkyl group having 7 to 16 carbon atoms as cited above;
concrete examples of the substituted or unsubstituted aryl group having 6 to 20 carbon atoms as cited above;
concrete examples of the substituted or unsubstituted hetero aryl group having 5 to 10 carbon atoms as cited above;
concrete examples of the substituted or unsubstituted alkenyl group having 2 to 6 carbon atoms as cited above;
concrete examples of the substituted or unsubstituted alkynyl group having 2 to 9 carbon atoms as cited above;
concrete examples of the substituted or unsubstituted metalocenyl group as cited above;
concrete examples of the substituted or unsubstituted alkoxy group having 1 to 8 carbon atoms as cited above;
concrete examples of the substituted or unsubstituted aralkyloxy group having 7 to 16 carbon atoms as cited above;
concrete examples of the substituted or unsubstituted aryloxy group having 6 to 10 carbon atoms as cited above;
concrete examples of the substituted or unsubstituted hetero aryloxy group having 5 to 10 carbon atoms as cited above;
concrete examples of the substituted or unsubstituted acyloxy group having 1 to 8 carbon atoms as cited above;
concrete examples of the substituted or unsubstituted alkylthio group having 1 to 6 carbon atoms as cited above;
concrete examples of the substituted or unsubstituted aralkylthio group having 7 to 16 carbon atoms as cited above;
concrete examples of the substituted or unsubstituted arylthio group having 6 to 10 carbon atoms as cited above;
concrete examples of the substituted or unsubstituted hetero arylthio group having 5 to 10 carbon atoms as cited above;
concrete examples of the substituted or unsubstituted alkylsulfonyl group having 2 to 7 carbon atoms as cited above;
concrete examples of the substituted or unsubstituted arylsulfonyl group having 6 to 10 carbon atoms as cited above;
concrete examples of the substituted or unsubstituted alkylamino group having 1 to 8 carbon atoms as cited above;
concrete examples of the substituted or unsubstituted arylamino group having 6 to 14 carbon atoms as cited above;
concrete examples of the substituted or unsubstituted hetero arylamino group having 5 to 10 carbon atoms as cited above;
concrete examples of the substituted or unsubstituted alkylcarbonylamino group having 2 to 7 carbon atoms as cited above;
concrete examples of the substituted or unsubstituted arylcarbonylamino group having 7 to 11 carbon atoms as cited above; and
concrete examples of the group having a substituted or unsubstituted metalocenyl group as cited above.

Examples of the group when R₁₁ and R₁₂ are bonded with each other to form a 5 to 6-membered ring include -(CH₂)₄-, -CH₂CH(CH₃) (CH₂)₂-, -CH₂O(CH₂)₂-, -(CH₂)₅-, -CH₂CH(CH₃) (CH₂)₃-, -CH₂O(CH₂)₃- and the like.

In the compound represented by the general formula (1) , when the hydrocarbon ring or the heterocyclic ring formed by bonding R₁ and R₂ has substituents, as the substituent and R₆ to R₂₁ in the general formulae (C-1) to (C-7), preferred is a fluorine-substituted substituent, and more preferred is a fluorine-substituted alkyl group, from the viewpoints of the recording sensitivity and stability against reproduction light.

Concrete examples of the fluorine-substituted alkyl group include a trifluoromethyl group, a 2,2,2-trifluoroethyl group, a pentafluoroethyl group, a 3,3,3-trifluoropropyl group, a 2,2,3,3-tetrafluoropropyl group, a 4,4,4-trifluorobutyl group, a 2,2,3,3,4,4,4-heptafluorobutyl group and the like.

In the compound represented by the general formula (1), when the hydrocarbon ring or the heterocyclic ring formed by bonding ring A, and R₁ and R₂ has substituents, a plurality of substituents in each ring may be bonded with each other to form a ring. That is, when ring A has substituents, a plurality of substituents in the ring A are bonded with each other to form a ring. When the hydrocarbon ring or the heterocyclic ring formed by bonding R₁ and R₂ has substituents, a plurality of substituents in the hydrocarbon ring or the heterocyclic ring formed by bonding R₁ and R₂ may be bonded with each other to form a ring.

Herein, when the ring A has substituents, or the hydrocarbon ring or the heterocyclic ring formed by bonding R₁ and R₂ has substituents (may be either adjacent to each other, or not), substituents may form a ring structure via a linking group together with a carbon atom which is substituted. Examples of the linking group at this time include hetero atoms such as a nitrogen atom, an oxygen atom, a sulfur atom and the like, carbon atoms, and groups in proper combination of these atoms. Preferable examples of the linking group include -O-, -S-, -C(=O)- and a methylene group, a methine group, an ethinylene group, a phenylene group, an amino group, an imino group or the like which may be substituted, and these groups can be properly combined to form a desired ring structure.

In the compound represented by the general formula (1), Y represents a group having an active hydrogen.
Concrete examples of Y include groups each having an active hydrogen such as -SH, -SO₂H, -SO₃H, -NH₂, -NHR, -OH, -COOH, -B(OH)₂, -PO(OH)₂, -NHCOH, -NHCOR, -NHSO₂R and the like. Preferable examples of Y include -SO₃H, -NH₂, -NHR, -OH, -COOH, -NHCOH and -NHSO₂R, and the most preferable examples of Y include -NH₂, -NHR and -OH. Herein, R represents an alkyl group having 1 to 6 carbon atoms such as a methyl group, an ethyl group, an n-propyl group, an isopropyl group, an n-butyl group, a tert-butyl group, a sec-butyl group, an n-pentyl group, an n-hexyl group and the like, or a phenyl group. These groups may be substituted with halogen atoms such as a fluorine atom, a chlorine atom, a bromine atom and the like.

In the present invention, concrete examples of the metal forming a chelate compound and azo compounds include Mg, Al, Ti, V, Cr, Mn, Fe, Co, Ni, Cu, Zn, Zr, Ru, Rh, Pd, In, Sn, Hf, Os, Pt or Hg, and particularly the azo metal chelate compound of Co, Ni or Cu is preferable because it is excellent in optical properties as an optical recording material.

In the optical recording medium of the present invention, the aforementioned recording layer containing a metal chelate compound of azo compounds represented by the general formula (2) below and a metal is a preferred embodiment,

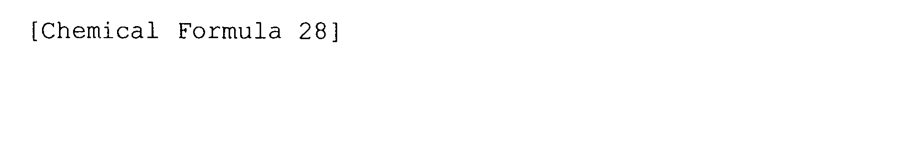

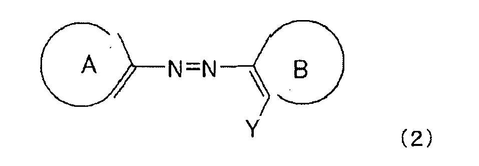

wherein, in the formula, ring A represents a carbocyclic aromatic ring which may have a substituent or a heterocyclic aromatic ring which may have a substituent, and two adjacent atoms in the ring are bonded with each other via a linking group represented by the general formula (a) and form at least one imide ruing;
ring B represents a hydrocarbon ring which may have a substituent or a heterocyclic ring which may have a substituent;
when rings A and B have substituents, substituents may be bonded with each other to form a ring; and
Y represents a group having an active hydrogen.

In the general formula (2), ring A represents a carbocyclic aromatic ring which may have a substituent or a heterocyclic aromatic ring which may have a substituent, and two adjacent atoms in the ring are bonded with each other via a linking group represented by the general formula (a) below and form at least one imide ring,

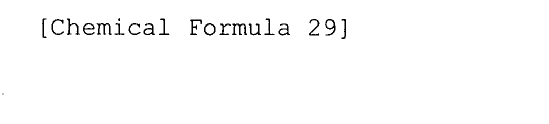

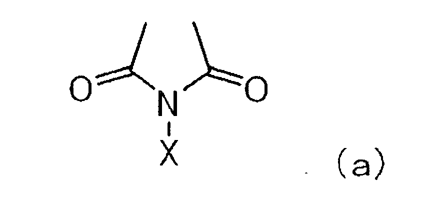

wherein, in the formula, X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group.

In the general formula (2), examples of ring A and examples of the substituent when ring A may have substituents include the same as those rings and substituents cited in the general formula (1), and the same as those preferred embodiments and concrete examples cited in the general formula (1).

In the general formula (2), ring B represents a hydrocarbon ring which may have a substituent or a heterocyclic ring which may have a substituent, and examples thereof include the same preferred embodiments and concrete examples as those cited in the case where, in the general formula (1), R₁ and R₂ are bonded with each other to form a hydrocarbon ring which may have a substituent or a heterocyclic ring which may have a substituent.

In the general formula (2), Y represents a group having an active hydrogen, and concrete examples thereof include the same as those described in Y of the general formula (1).

In the optical recording medium of the present invention, the aforementioned recording layer containing a metal chelate compound of azo compounds represented by the general formula (3) and a metal, or a metal chelate compound of azo compounds represented by the general formula (4) and a metal can be cited as a more preferred embodiment,

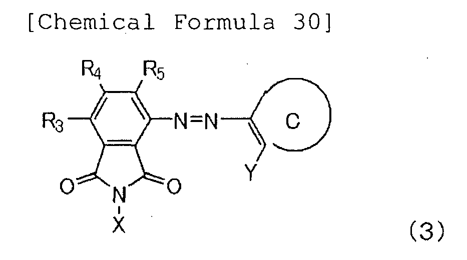

wherein, in the formula, X represents the same as those described above;
R₃ to R₅ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
Y represents a group having an active hydrogen; and
ring C represents the general formulae (C-1) to (C-7) below,

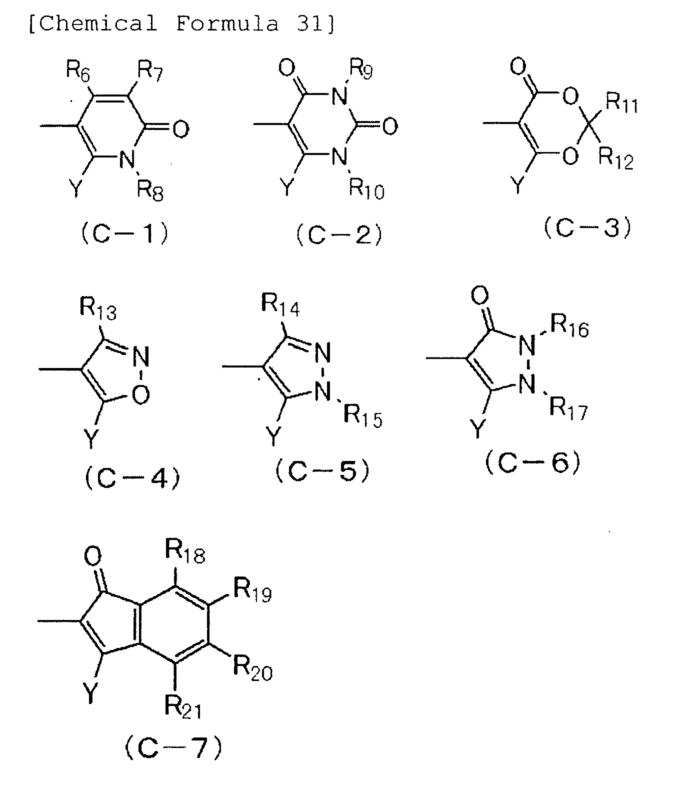

wherein, in the formula, R₆ to R₂₁ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylsulfonyl group, a substituted or unsubstituted arylsulfonyl group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
R₁₁ and R₁₂ may be bonded with each other to form a 5 to 6-membered ring; and Y represents the same as those described above,

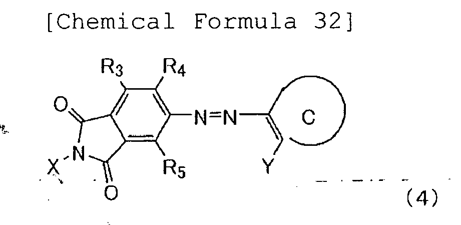

wherein, in the formula, X, R₃ to R₅, Y and ring C represent the same as those described above.

In the general formula (3) or (4) , X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group.

Examples of X in the general formula (3) or (4) include the same preferred embodiments and concrete examples as those cited in X of the general formula (a) of the general formula (1).

In the general formula (3) or (4), R₃ to R₅ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group.

Preferable examples of R₃ to R₅ include a hydrogen atom, a halogen atom, a hydroxyl group, an amino group, a nitro group, a cyano group, a carboxyl group, a substituted or unsubstituted alkoxycarbonyl group having 2 to 7 carbon atoms, a substituted or unsubstituted aralkyloxycarbonyl group having 8 to 16 carbon atoms, a substituted or unsubstituted aryloxycarbonyl group having 7 to 11 carbon atoms, a substituted or unsubstituted acyl group having 1 to 7 carbon atoms, a substituted or unsubstituted arylcarbonyl group having 7 to 11 carbon atoms, a substituted or unsubstituted hetero arylcarbonyl group having 6 to 11 carbon atoms, a substituted or unsubstituted alkyl group having 1 to 10 carbon atoms, a substituted or unsubstituted aralkyl group having 7 to 16 carbon atoms, a substituted or unsubstituted aryl group having 6 to 20 carbon atoms, a substituted or unsubstituted hetero aryl group having 5 to 10 carbon atoms, a substituted or unsubstituted alkenyl group having 2 to 6 carbon atoms, a substituted or unsubstituted alkynyl group having 2 to 9 carbon atoms, a substituted or unsubstituted metalocenyl group, a substituted or unsubstituted alkoxy group having 1 to 8 carbon atoms, a substituted or unsubstituted aralkyloxy group having 7 to 16 carbon atoms, a substituted or unsubstituted aryloxy group having 6 to 10 carbon atoms, a substituted or unsubstituted hetero aryloxy group having 5 to 10 carbon atoms, a substituted or unsubstituted acyloxy group having 1 to 8 carbon atoms, a substituted or unsubstituted alkylthio group having 1 to 6 carbon atoms, a substituted or unsubstituted aralkylthio group having 7 to 16 carbon atoms, a substituted or unsubstituted arylthio group having 6 to 10 carbon atoms, a substituted or unsubstituted hetero arylthio group having 5 to 10 carbon atoms, a substituted or unsubstituted alkylamino group having 1 to 8 carbon atoms, a substituted or unsubstituted arylamino group having 6 to 14 carbon atoms, a substituted or unsubstituted hetero arylamino group having 5 to 10 carbon atoms, a substituted or unsubstituted alkylcarbonylamino group having 2 to 7 carbon atoms, a substituted or unsubstituted arylcarbonylamino group having 7 to 11 carbon atoms, and a group having a substituted or unsubstituted metalocenyl group.

Concrete examples of R₃ to R₅ include a hydrogen atom;
halogen atoms such as a fluorine atom, a chlorine atom, a bromine atom, an iodine atom and the like;
a hydroxyl group;
an amino group;
a nitro group;
a cyano group;
a carboxyl group;
concrete examples of the substituted or unsubstituted alkoxycarbonyl group having 2 to 7 carbon atoms as cited above;
concrete examples of the substituted or unsubstituted aralkyloxycarbonyl group having 8 to 16 carbon atoms as cited above;
concrete examples of the substituted or unsubstituted aryloxycarbonyl group having 7 to 11 carbon atoms as cited above;
concrete examples of the substituted or unsubstituted acyl group having 1 to 7 carbon atoms as cited above;
concrete examples of the substituted or unsubstituted arylcarbonyl group having 7 to 11 carbon atoms as cited above;
concrete examples of the substituted or unsubstituted hetero arylcarbonyl group having 6 to 11 carbon atoms as cited above;
concrete examples of the substituted or unsubstituted alkyl group having 1 to 10 carbon atoms as cited above;
concrete examples of the substituted or unsubstituted aralkyl group having 7 to 16 carbon atoms as cited above;
concrete examples of the substituted or unsubstituted aryl group having 6 to 20 carbon atoms as cited above;
concrete examples of the substituted or unsubstituted hetero aryl group having 5 to 10 carbon atoms as cited above;
concrete examples of the substituted or unsubstituted alkenyl group having 2 to 6 carbon atoms as cited above;
concrete examples of the substituted or unsubstituted alkynyl group having 2 to 9 carbon atoms as cited above;
concrete examples of the substituted or unsubstituted metalocenyl group as cited above;
concrete examples of the substituted or unsubstituted alkoxy group having 1 to 8 carbon atoms as cited above;
concrete examples of the substituted or unsubstituted aralkyloxy group having 7 to 16 carbon atoms as cited above;
concrete examples of the substituted or unsubstituted aryloxy group having 6 to 10 carbon atoms as cited above;
concrete examples of the substituted or unsubstituted hetero aryloxy group having 5 to 10 carbon atoms as cited above;
concrete examples of the substituted or unsubstituted acyloxy group having 1 to 8 carbon atoms as cited above;
concrete examples of the substituted or unsubstituted alkylthio group having 1 to 6 carbon atoms as cited above;
concrete examples of the substituted or unsubstituted aralkylthio group having 7 to 16 carbon atoms as cited above;
concrete examples of the substituted or unsubstituted arylthio group having 6 to 10 carbon atoms as cited above;
concrete examples of the substituted or unsubstituted hetero arylthio group having 5 to 10 carbon atoms as cited above;
concrete examples of the substituted or unsubstituted alkylamino group having 1 to 8 carbon atoms as cited above;
concrete examples of the substituted or unsubstituted arylamino group having 6 to 14 carbon atoms as cited above;
concrete examples of the substituted or unsubstituted hetero arylamino group having 5 to 10 carbon atoms as cited above;
concrete examples of the substituted or unsubstituted alkylcarbonylamino group having 2 to 7 carbon atoms as cited above;
concrete examples of the substituted or unsubstituted arylcarbonylamino group having 7 to 11 carbon atoms as cited above; and
concrete examples of the group having a substituted or unsubstituted metalocenyl group as cited above.

As R₃ to R₅, preferred is a fluorine-substituted substituent, more preferred is a fluorine-substituted alkyl group, from the viewpoints of the recording sensitivity and stability against reproduction light.
Concrete examples of the fluorine-substituted alkyl group include a trifluoromethyl group, a 2,2,2-trifluoroethyl group, a pentafluoroethyl group, a 3,3,3-trifluoropropyl group, a 2,2,3,3-tetrafluoropropyl group, a 4,4,4-trifluorobutyl group, a 2,2,3,3,4,4,4-heptafluorobutyl group and the like.

In the general formula (3) or (4), Y represents a group having an active hydrogen. Examples of Y in the general formula (3) or (4) include the same concrete examples as those described in Y of the general formula (1).

In the general formula (3) or (4), ring C represents the general formulae (C-1) to (C-7).
The general formulae (C-1) to (C-7) are the same as those cited as preferred examples of ring B in the general formula (1).

In the optical recording medium of the present invention, the aforementioned recording layer containing a metal chelate compound of azo compounds represented by the general formula (5) and a metal, or a metal chelate compound of azo compounds represented by the general formula (6) and a metal is a more preferred embodiment,

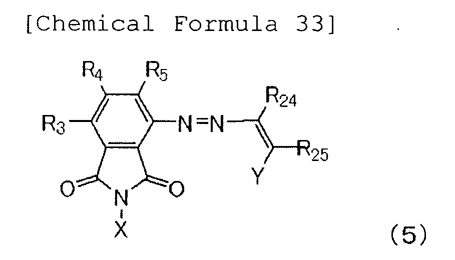

wherein, in the formula, X, R₃ to R₅ and Y represent the same as those described above;
R₂₄ represents a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, or a substituted or unsubstituted hetero arylcarbonyl group; and
R₂₅ represents a hydroxyl group, an amino group, a substituted or unsubstituted alkyl group, a substituted or unsubstituted aralkyl group, a substituted or unsubstituted aryl group, a substituted or unsubstituted hetero aryl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted alkylamino group, a substituted or unsubstituted arylamino group, or a substituted or unsubstituted hetero arylamino group,

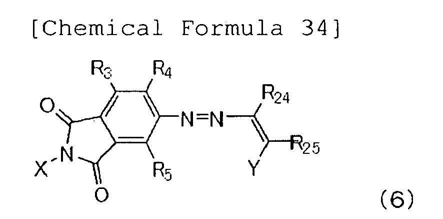

wherein, in the formula, X, R₃ to R₅, Y, R₂₄ and R₂₅ represent the same as those described above.

In the general formula (5) or (6), X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group.

In the general formula (5) or (6), examples of X include the same preferred embodiments and concrete examples as those cited in X in the general formula (a) of the general formula (1).

In the general formula (5) or (6), R₃ to R₅ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group. Concrete examples thereof include the same as those of R₃ to R₅ in the general formula (3) or (4).

In the general formula (5) or (6), R₂₄ represents a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, or a substituted or unsubstituted hetero arylcarbonyl group.

Herein, preferred examples of R₂₄ include a nitro group, a cyano group, a carboxyl group, a substituted or unsubstituted alkoxycarbonyl group having 2 to 7 carbon atoms, a substituted or unsubstituted aralkyloxycarbonyl group having 8 to 16 carbon atoms, a substituted or unsubstituted aryloxycarbonyl group having 7 to 11 carbon atoms, a substituted or unsubstituted alkylaminocarbonyl group having 2 to 9 carbon atoms, a substituted or unsubstituted arylaminocarbonyl group having 7 to 15 carbon atoms, a substituted or unsubstituted acyl group having 1 to 7 carbon atoms, a substituted or unsubstituted arylcarbonyl group having 7 to 11 carbon atoms, and a substituted or unsubstituted hetero arylcarbonyl group having 6 to 11 carbon atoms.

Concrete examples of R₂₄ include a nitro group;
a cyano group;
a carboxyl group;
concrete examples of the substituted or unsubstituted alkoxycarbonyl group having 2 to 7 carbon atoms as cited above;
concrete examples of the substituted or unsubstituted aralkyloxycarbonyl group having 8 to 16 carbon atoms as cited above;
concrete examples of the substituted or unsubstituted aryloxycarbonyl group having 7 to 11 carbon atoms as cited above;
concrete examples of the substituted or unsubstituted alkylaminocarbonyl group having 2 to 9 carbon atoms as cited above;
concrete examples of the substituted or unsubstituted arylaminocarbonyl group having 7 to 15 carbon atoms as cited above;
concrete examples of the substituted or unsubstituted acyl group having 1 to 7 carbon atoms as cited above;
concrete examples of the substituted or unsubstituted arylcarbonyl group having 7 to 11 carbon atoms as cited above; and
concrete examples of the substituted or unsubstituted hetero arylcarbonyl group having 6 to 11 carbon atoms as cited above.

In the general formula (5) or (6), R₂₅ represents a hydroxyl group, an amino group, a substituted or unsubstituted alkyl group, a substituted or unsubstituted aralkyl group, a substituted or unsubstituted aryl group, a substituted or unsubstituted hetero aryl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted alkylamino group, a substituted or unsubstituted arylamino group, or a substituted or unsubstituted hetero arylamino group.

Preferable examples of R₂₅ include a hydroxyl group, an amino group, a substituted or unsubstituted alkyl group having 1 to 10 carbon atoms, a substituted or unsubstituted aralkyl group having 7 to 16 carbon atoms, a substituted or unsubstituted aryl group having 6 to 20 carbon atoms, a substituted or unsubstituted hetero aryl group having 5 to 10 carbon atoms, a substituted or unsubstituted alkoxy group having 1 to 8 carbon atoms, a substituted or unsubstituted aralkyloxy group having 7 to 16 carbon atoms, a substituted or unsubstituted aryloxy group having 6 to 10 carbon atoms, a substituted or unsubstituted hetero aryloxy group having 5 to 10 carbon atoms, a substituted or unsubstituted alkylamino group having 1 to 8 carbon atoms, a substituted or unsubstituted arylamino group having 6 to 14 carbon atoms, and a substituted or unsubstituted hetero arylamino group having 5 to 10 carbon atoms.

Concrete examples of R₂₅ include a hydroxyl group;
an amino group;
concrete examples of the substituted or unsubstituted alkyl group having 1 to 10 carbon atoms as cited above;
concrete examples of the substituted or unsubstituted aralkyl group having 7 to 16 carbon atoms as cited above;
concrete examples of the substituted or unsubstituted aryl group having 6 to 20 carbon atoms as cited above;
concrete examples of the substituted or unsubstituted hetero aryl group having 5 to 10 carbon atoms as cited above;
concrete examples of the substituted or unsubstituted alkoxy group having 1 to 8 carbon atoms as cited above;
concrete examples of the substituted or unsubstituted aralkyloxy group having 7 to 16 carbon atoms as cited above;
concrete examples of the substituted or unsubstituted aryloxy group having 6 to 10 carbon atoms as cited-above;
concrete examples of the substituted or unsubstituted hetero aryloxy group having 5 to 10 carbon atoms as cited above;
concrete examples of the substituted or unsubstituted alkylamino group having 1 to 8 carbon atoms as cited above;
concrete examples of the substituted or unsubstituted arylamino group having 6 to 14 carbon atoms as cited above; and
concrete examples of the substituted or unsubstituted hetero arylamino group having 5 to 10 carbon atoms as cited above.

Concrete preferable examples of the aforementioned metal chelate compound of azo compounds represented by the above general formula (1) and a metal of the present invention are illustrated in concrete examples (1) to (94), (2-1) to (2-80) and (3-1) to (3-40) of Tables 1-1 to 1-22.

**[Table 1-1]**

| | |
|---|---|
| Concrete Example (1) 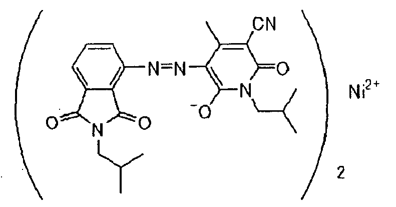 | Concrete Example (2) 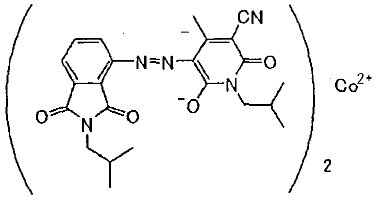 |
| Concrete Example (3) 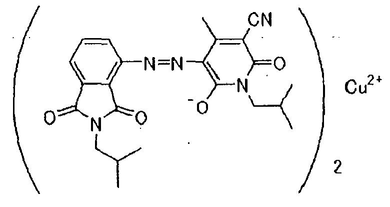 | Concrete Example (4) 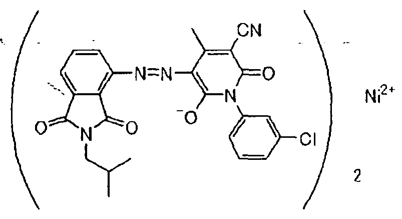 |
| Concrete Example (5) 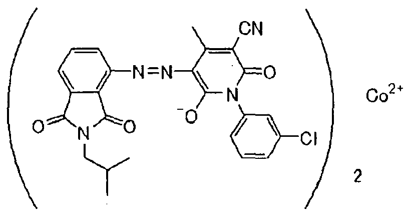 | Concrete Example (6) 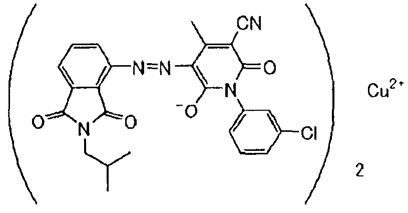 |
| Concrete Example (7) 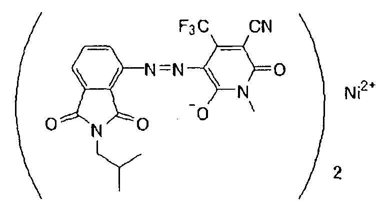 | Concrete Example (8) 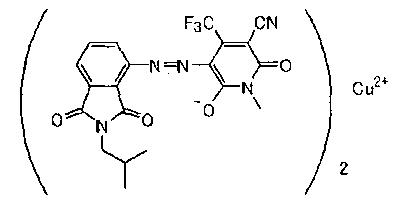 |
| Concrete Example (9) 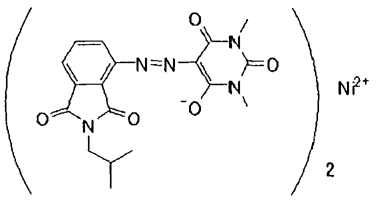 | Concrete Example (10) 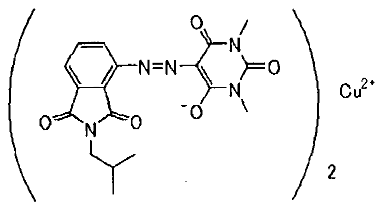 |

**[Table 1-2]**

| | |
|---|---|
| Concrete Example (11) 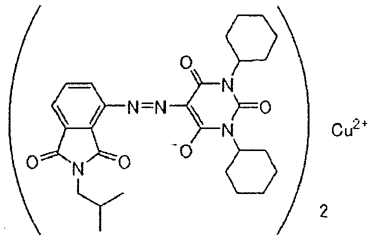 | Concrete Example (12) 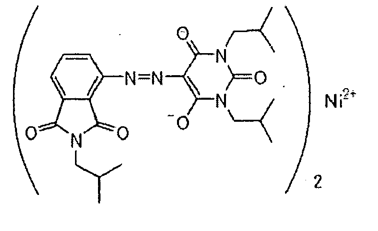 |
| Concrete Example (13) 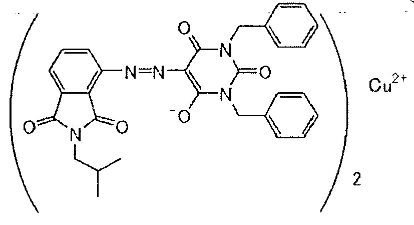 | Concrete Example (14) 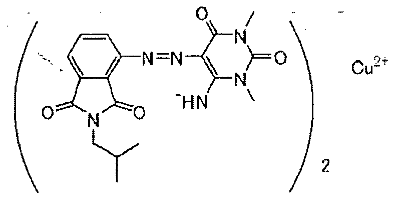 |
| Concrete Example (15) 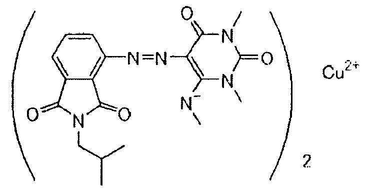 | Concrete Example (16) 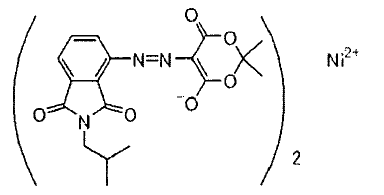 |
| Concrete Example (17) 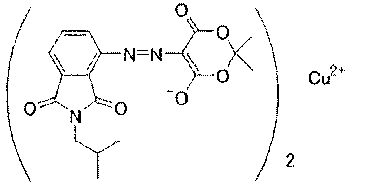 | Concrete Example (18) 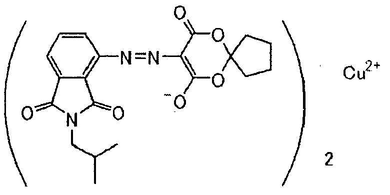 |
| Concrete Example (19) 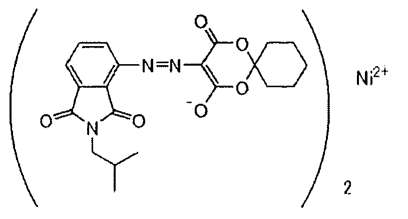 | Concrete Example (20) 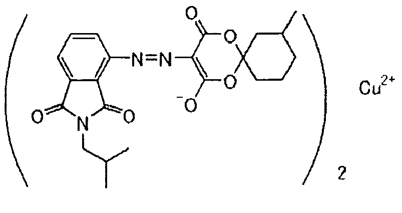 |

**[Table 1-3]**

| | |
|---|---|
| Concrete Example (21) 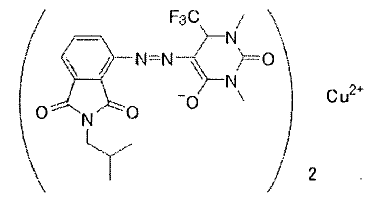 | Concrete Example (22) 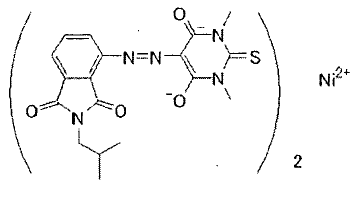 |
| Concrete Example (23) 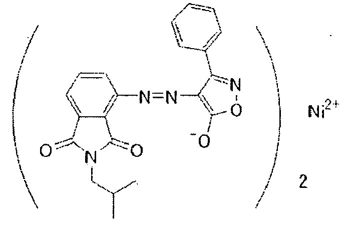 | Concrete Example (24) 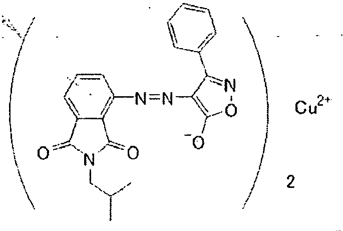 |
| Concrete Example (25) 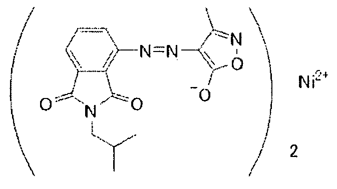 | Concrete Example (26) 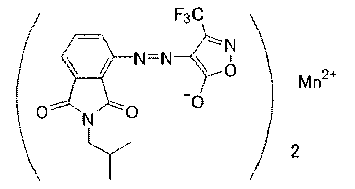 |
| Concrete Example (27) 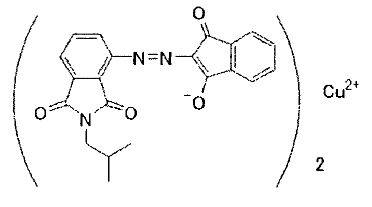 | Concrete Example (28) 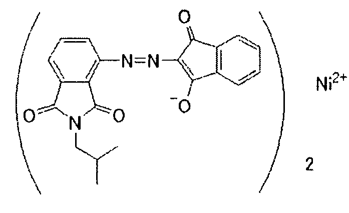 |
| Concrete Example (29) 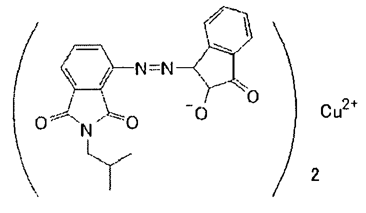 | Concrete Example (30) 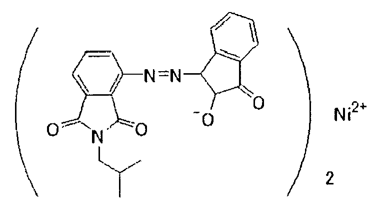 |

**[Table 1-4]**

| | | |
|---|---|---|
| Concrete Example (31) 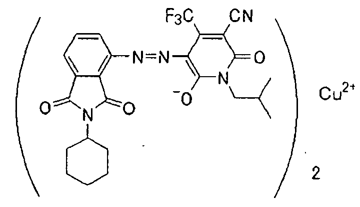 | | Concrete Example (32) 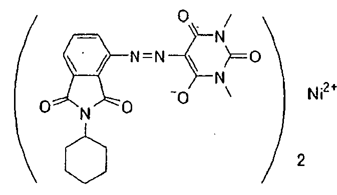 |
| Concrete Example (33) 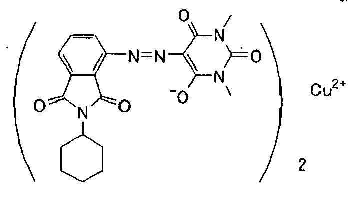 | | Concrete Example (34) 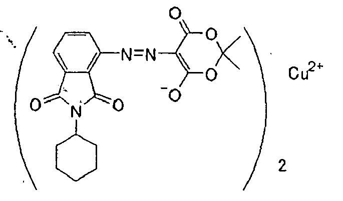 |
| Concrete Example (35) 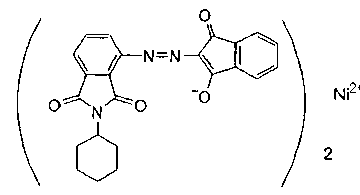 | | Concrete Example (36) 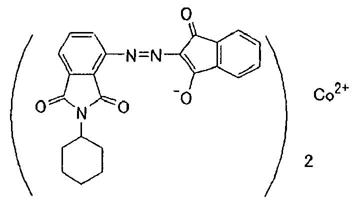 |
| Concrete Example (37) 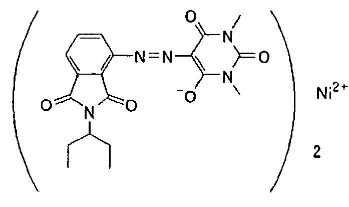 | | Concrete Example (38) 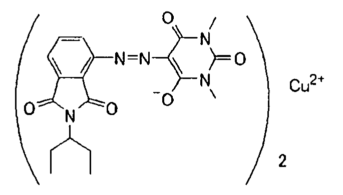 |
| Concrete Example (39) 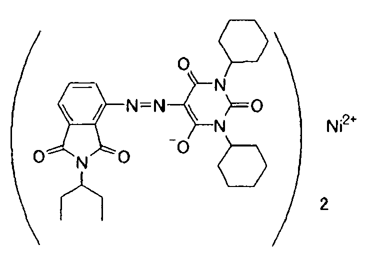 | | Concrete Example (40) 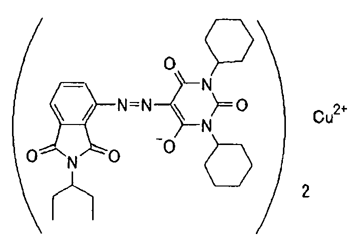 |

**[Table 1-5]**

| | |
|---|---|
| Concrete Example (41) 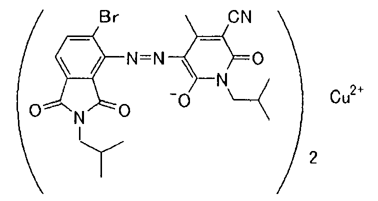 | Concrete Example (42) 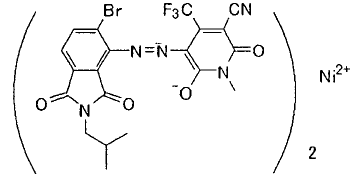 |
| Concrete Example (43) 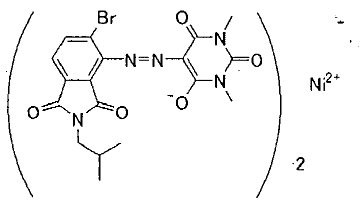 | Concrete Example (44) 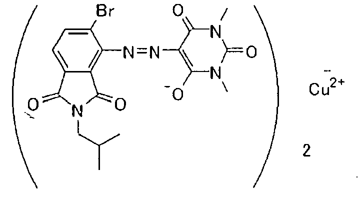 |
| Concrete Example (45) 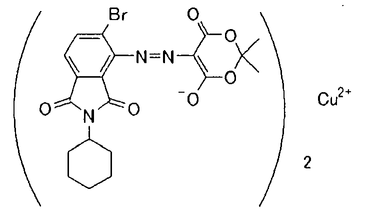 | Concrete Example (46) 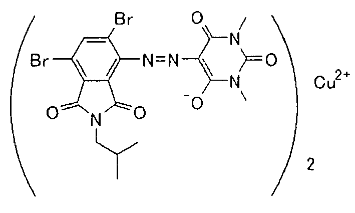 |
| Concrete Example (47) 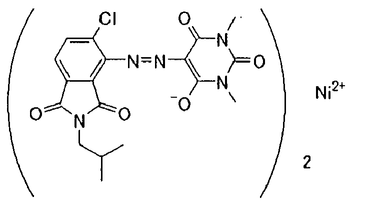 | Concrete Example (48) 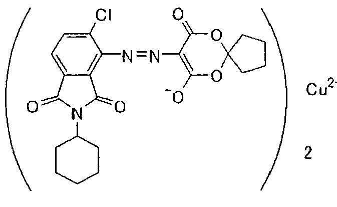 |
| Concrete Example (49) 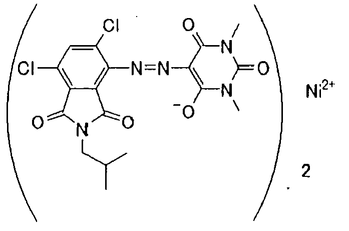 | Concrete Example (50) 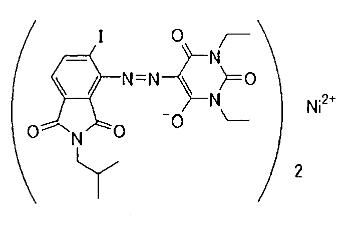 |

**[Table 1-6]**

| | |
|---|---|
| Concrete Example (51) 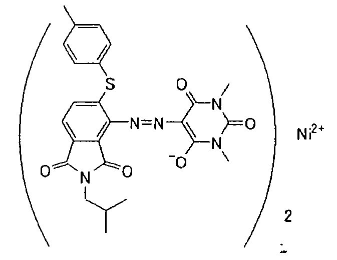 | Concrete Example (52) 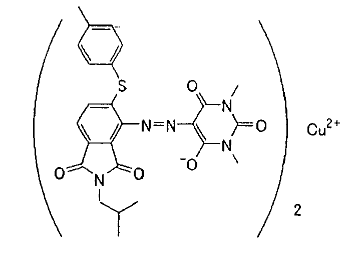 |
| Concrete Example (53) 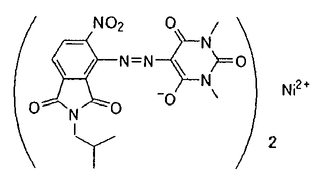 | Concrete Example (54) 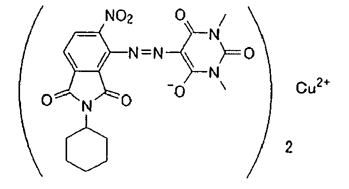 |
| Concrete Example (55) 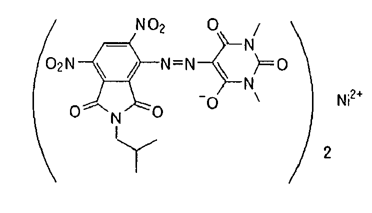 | Concrete Example (56) 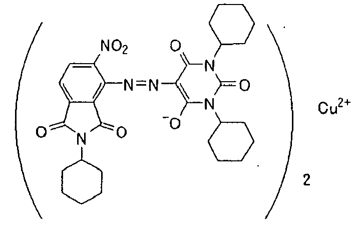 |
| Concrete Example (57) 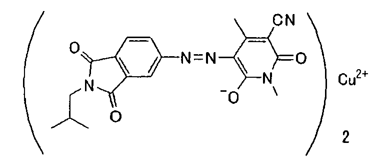 | Concrete Example (58) 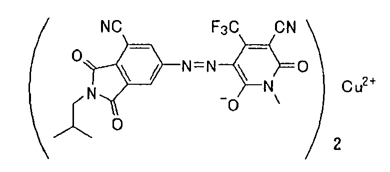 |
| Concrete Example (59) 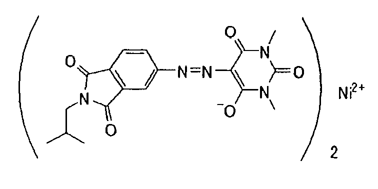 | Concrete Example (60) 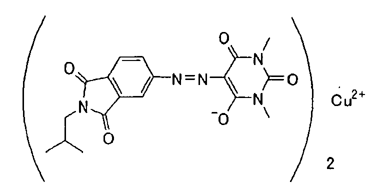 |

**[Table 1-7]**

| | |
|---|---|
| Concrete Example (61) 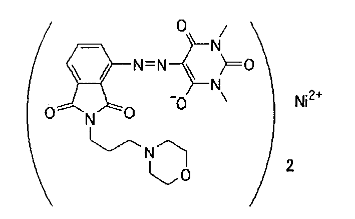 | Concrete Example (62) 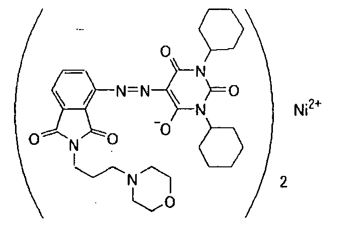 |
| Concrete Example (63) 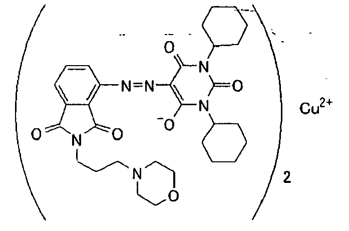 | Concrete Example (64) 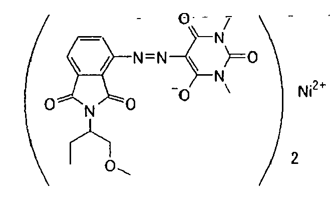 |
| Concrete Example (65) 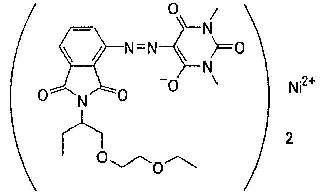 | Concrete Example (66) 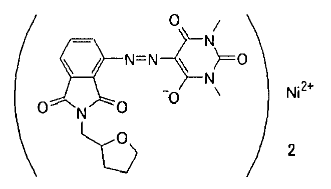 |
| Concrete Example (67) 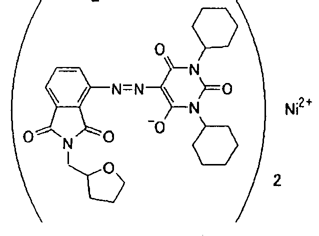 | Concrete Example (68) 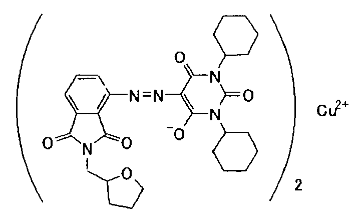 |
| Concrete Example (69) 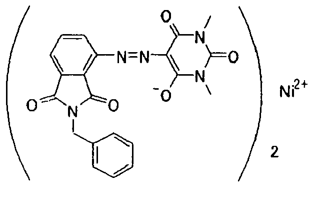 | Concrete Example (70) 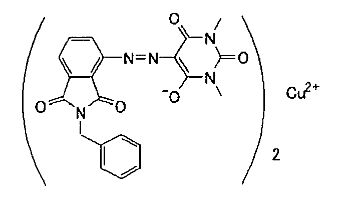 |

**[Table 1-8]**

| | |
|---|---|
| Concrete Example (71) 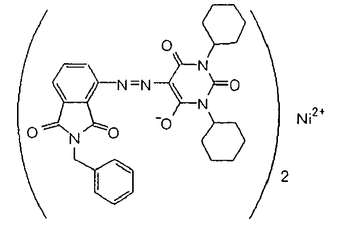 | Concrete Example (72) 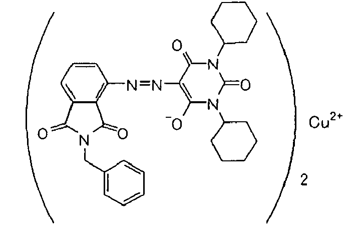 |
| Concrete Example (73) 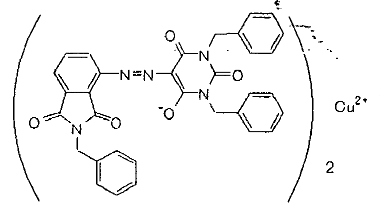 | Concrete Example (74) 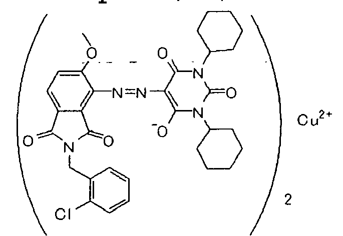 |
| Concrete Example (75) 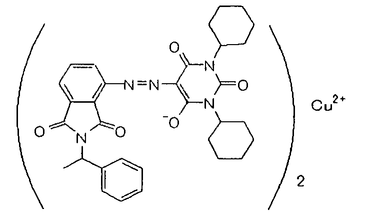 | Concrete Example (76) 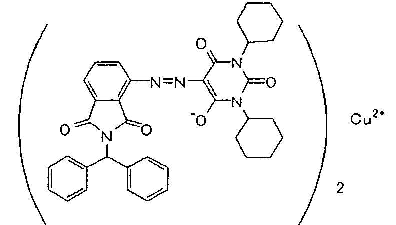 |
| Concrete Example (77) 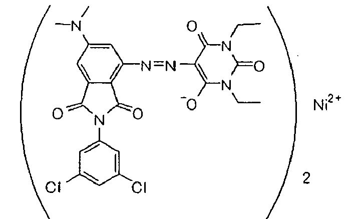 | Concrete Example (78) 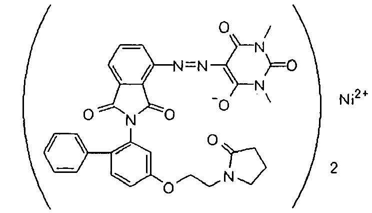 |
| Concrete Example (79) 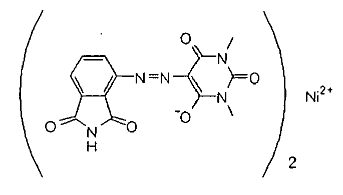 | Concrete Example (80) 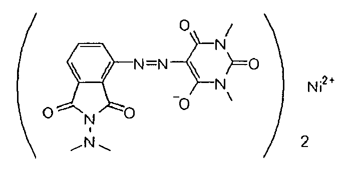 |

**[Table 1-9]**

| | |
|---|---|
| Concrete Example (81) 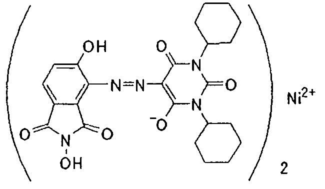 | Concrete Example (82) 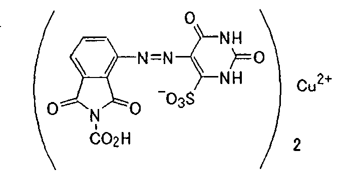 |
| Concrete Example (83) 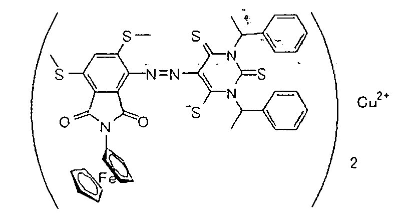 | Concrete Example (84) 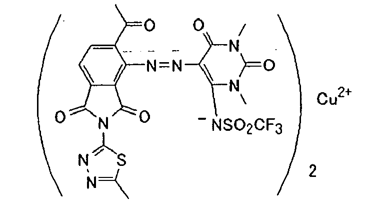 |
| Concrete Example (85) 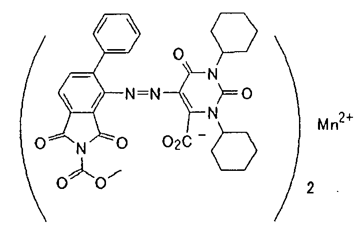 | Concrete Example (86) 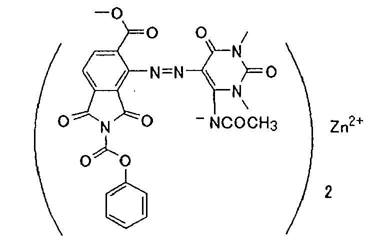 |
| Concrete Example (87) 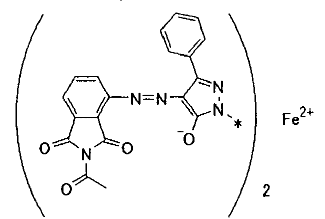 | Concrete Example (88) 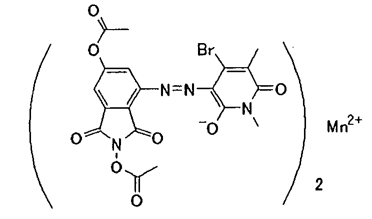 |
| Concrete Example (89) 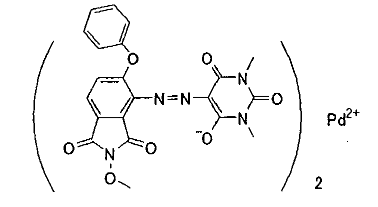 | Concrete Example (90) 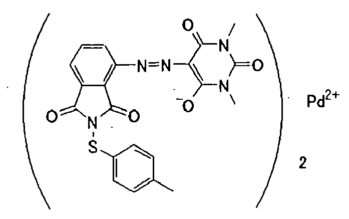 |

**[Table 1-10]**

| | |
|---|---|
| Concrete Example (91) 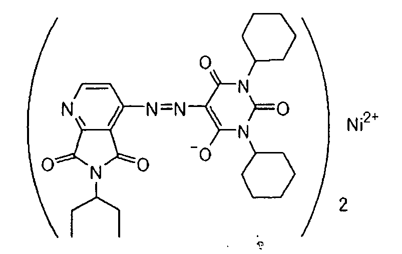 | Concrete Example (92) 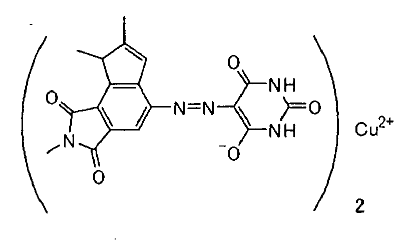 |
| Concrete Example (93) 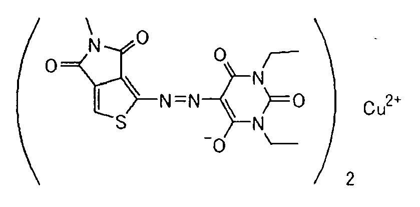 | Concrete Example (94) 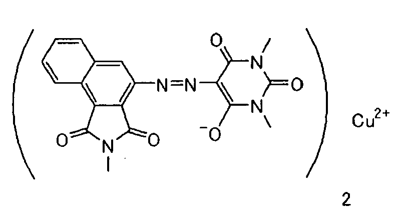 |

**[Table 1-11]**

| | |
|---|---|
| Concrete Example (2-1) 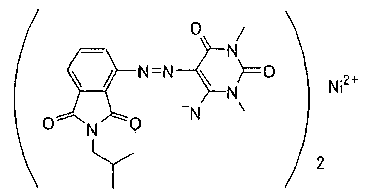 | Concrete Example (2-2) 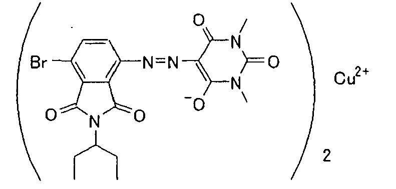 |
| Concrete Example (2-3) 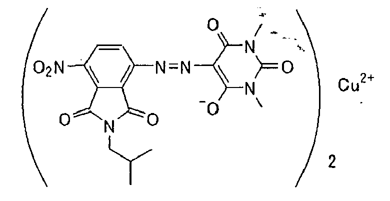 | Concrete Example (2-4) 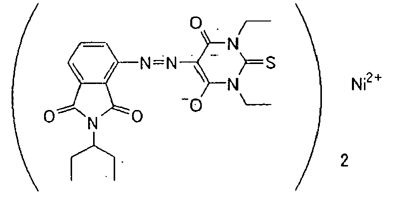 |
| Concrete Example (2-5) 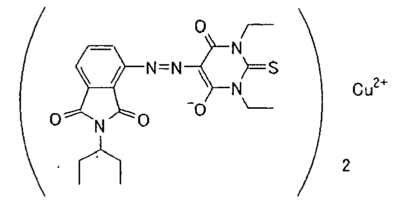 | Concrete Example (2-6) 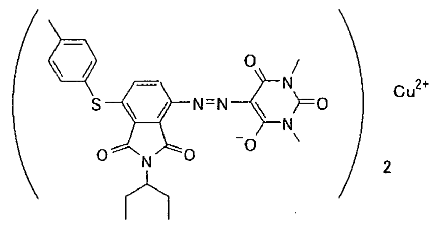 |
| Concrete Example (2-7) 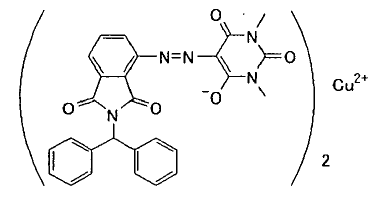 | Concrete Example (2-8) 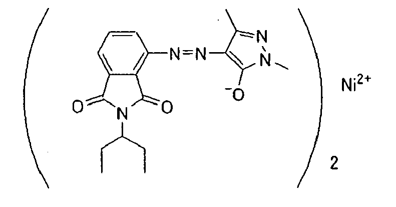 |
| Concrete Example (2-9) 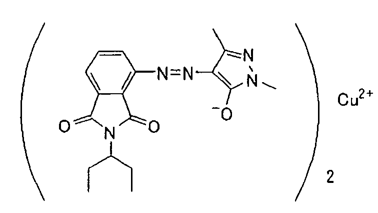 | Concrete Example (2-10) 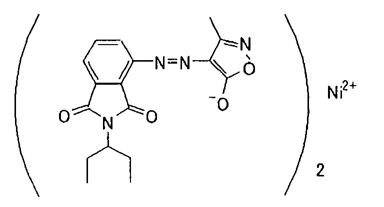 |

**[Table 1-12]**

| | |
|---|---|
| Concrete Example (2-11) 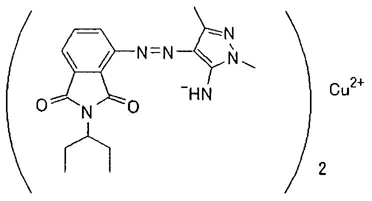 | Concrete Example (2-12) 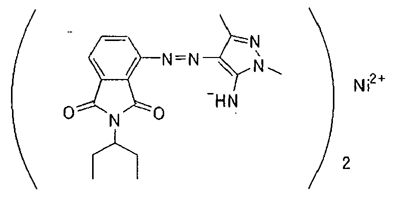 |
| Concrete Example (2-13) 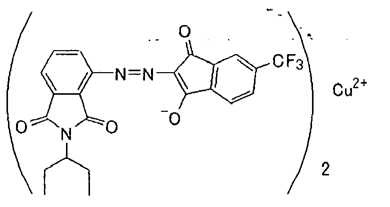 | Concrete Example (2-14) 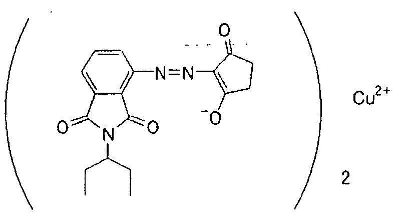 |
| Concrete Example (2-15) 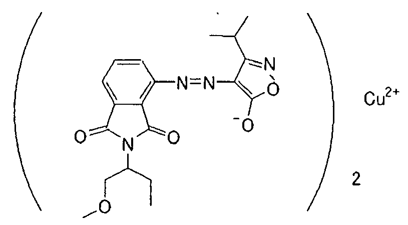 | Concrete Example (2-16) 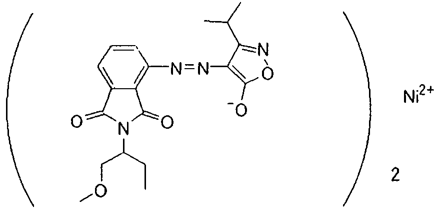 |
| Concrete Example (2-17) 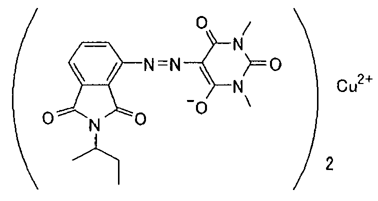 | Concrete Example (2-18) 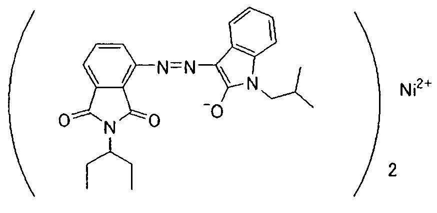 |
| Concrete Example (2-19) 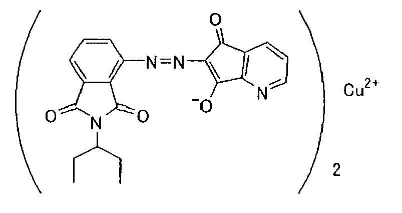 | Concrete Example (2-20) 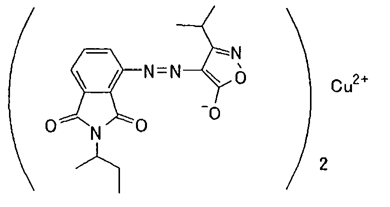 |

**[Table 1-13]**

| | |
|---|---|
| Concrete Example (2-21) 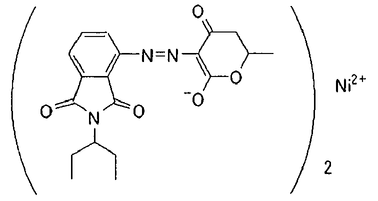 | Concrete Example (2-22) 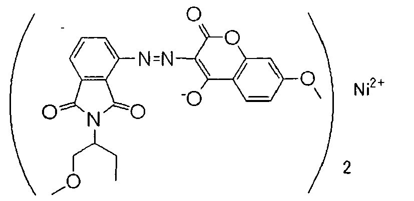 |
| Concrete Example (2-23) 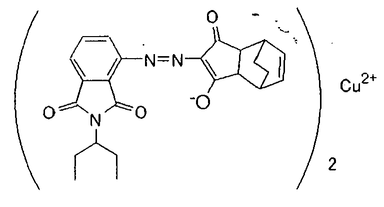 | Concrete Example (2-24) 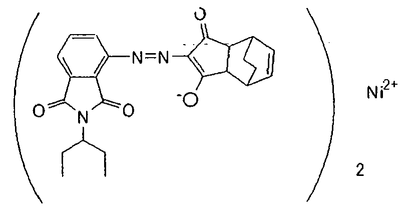 |
| Concrete Example (2-25) 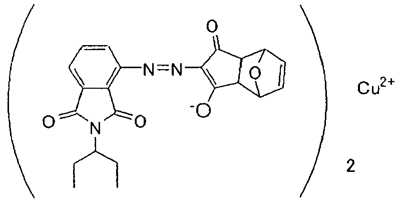 | Concrete Example (2-26) 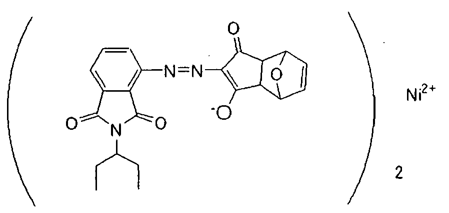 |
| Concrete Example (2-27) 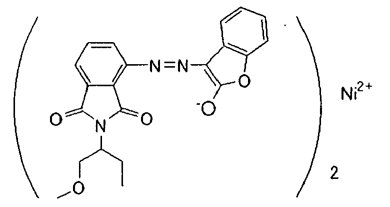 | Concrete Example (2-28) 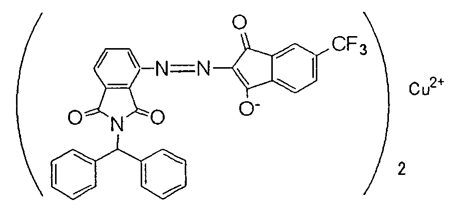 |
| Concrete Example (2-29) 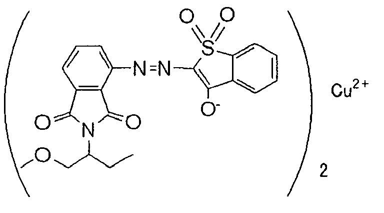 | Concrete Example (2-30) 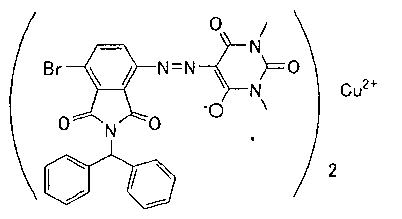 |

**[Table 1-14]**

| | |
|---|---|
| Concrete Example (2-31) 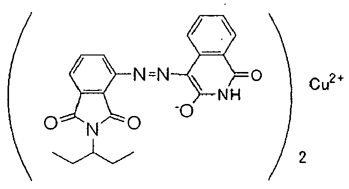 | Concrete Example (2-32) 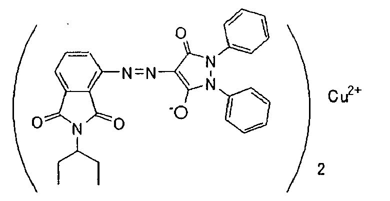 |
| Concrete Example (2-33) 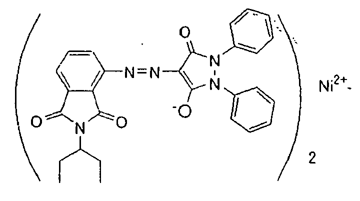 | Concrete Example (2-34) 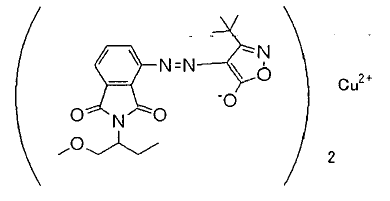 |
| Concrete Example (2-35) 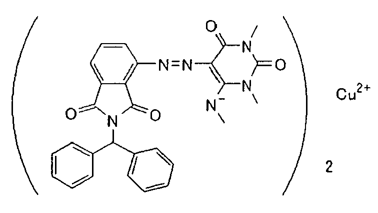 | Concrete Example (2-36) 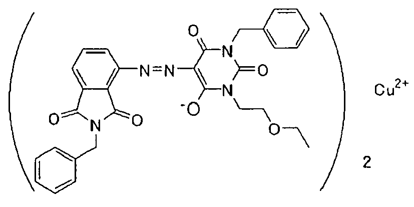 |
| Concrete Example (2-37) 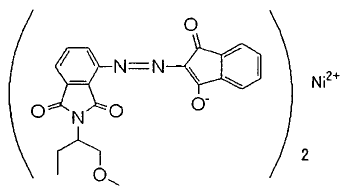 | Concrete Example (2-38) 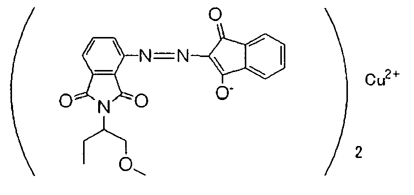 |
| Concrete Example (2-39) 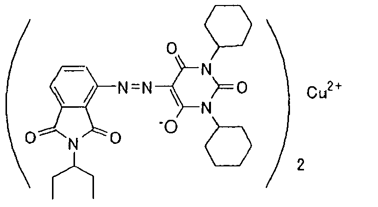 | Concrete Example (2-40) 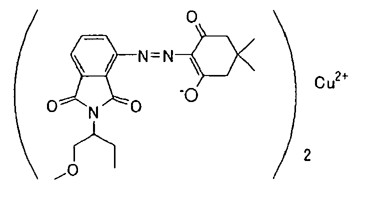 |

**[Table 1-15]**

| | |
|---|---|
| Concrete Example (2-41) 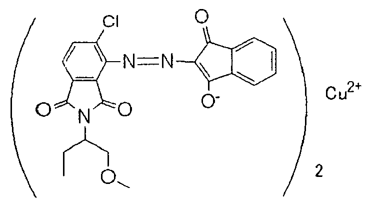 | Concrete Example (2-42) 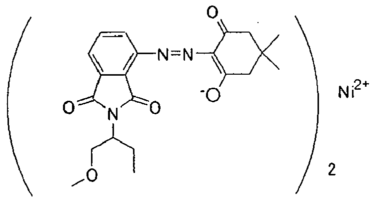 |
| Concrete Example (2-43) 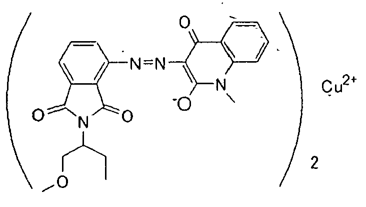 | Concrete Example (2-44) 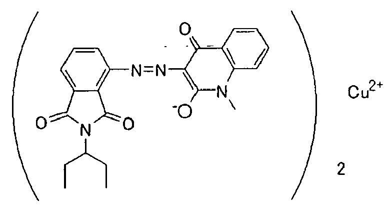 |
| Concrete Example (2-45) 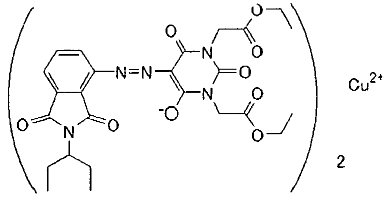 | Concrete Example (2-46) 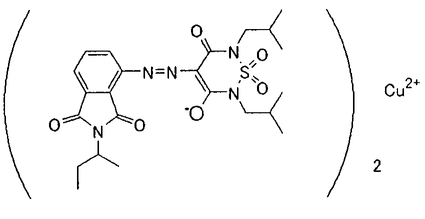 |
| Concrete Example (2-47) 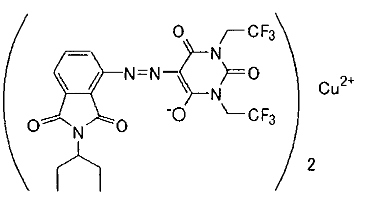 | Concrete Example (2-48) 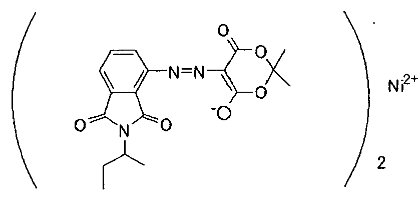 |
| Concrete Example (2-49) 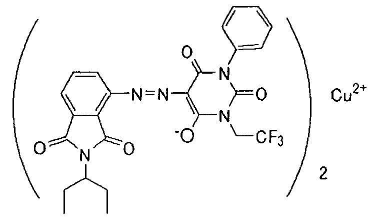 | Concrete Example (2-50) 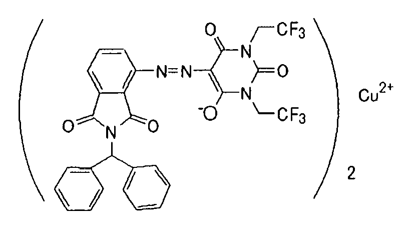 |

**[Table 1-16]**

| | |
|---|---|
| Concrete Example (2-51) 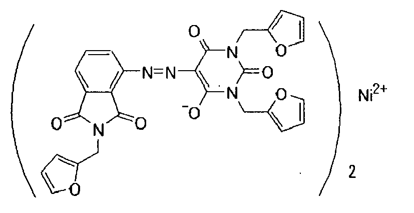 | Concrete Example (2-52) 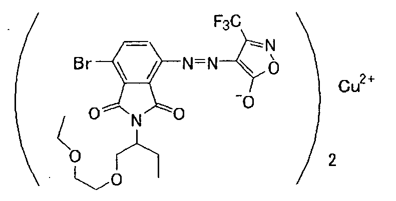 |
| Concrete Example (2-53) 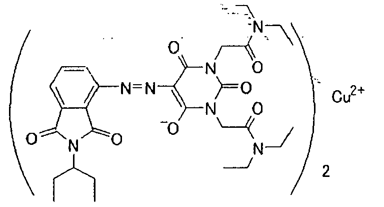 | Concrete Example (2-54) 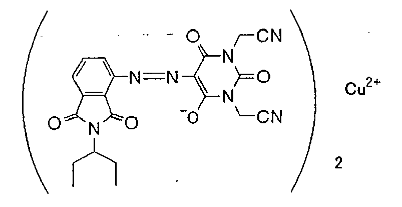 |
| Concrete Example (2-55) 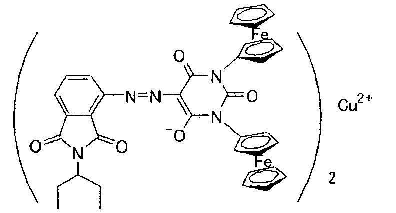 | Concrete Example (2-56) 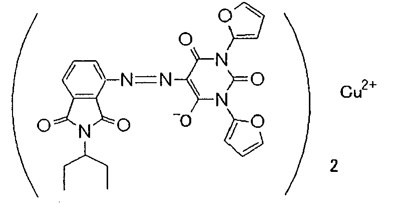 |
| Concrete Example (2-57) 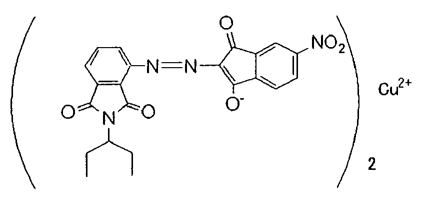 | Concrete Example (2-58) 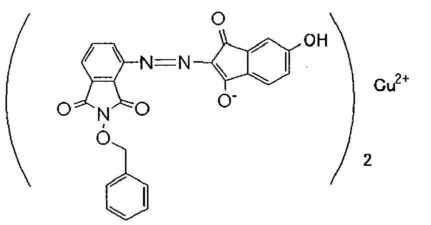 |
| Concrete Example (2-59) 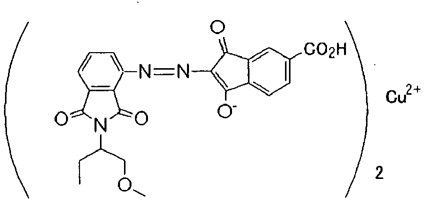 | Concrete Example (2-60) 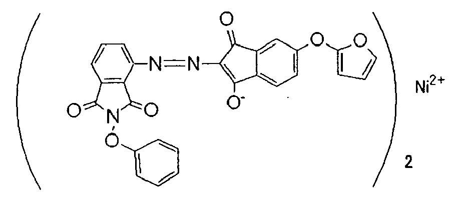 |

**[Table 1-17]**

| | |
|---|---|
| Concrete Example (2-61) 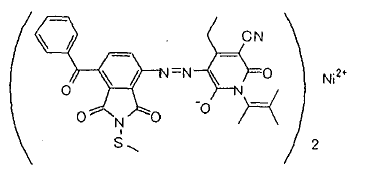 | Concrete Example (2-62) 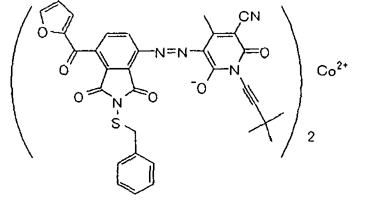 |
| Concrete Example (2-63) 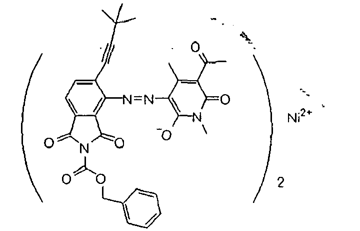 | Concrete Example (2-64) 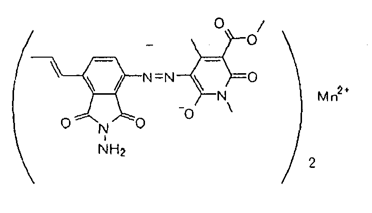 |
| Concrete Example (2-65) 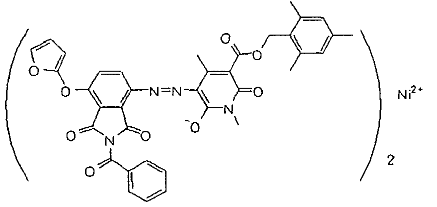 | Concrete Example (2-66) 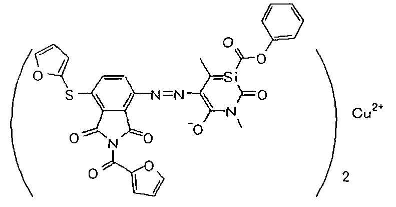 |
| Concrete Example (2-67) 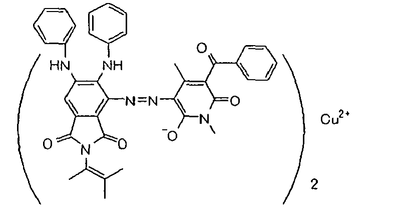 | Concrete Example (2-68) 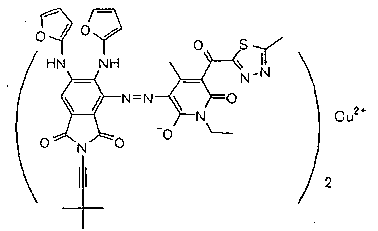 |
| Concrete Example (2-69) 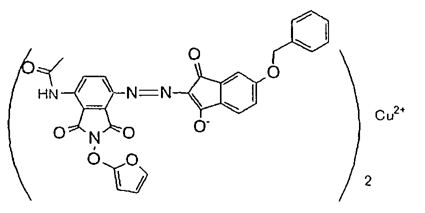 | Concrete Example (2-70) 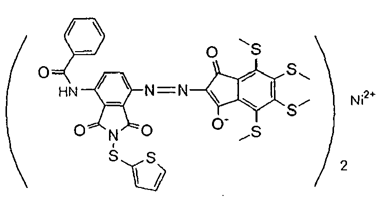 |

**[Table 1-18]**

| | |
|---|---|
| Concrete Example (2-71) 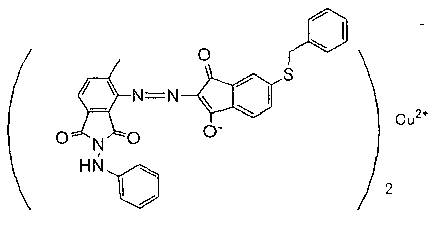 | Concrete Example (2-72) 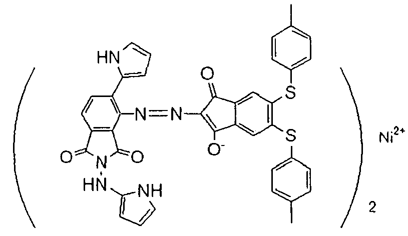 |
| Concrete Example (2-73) 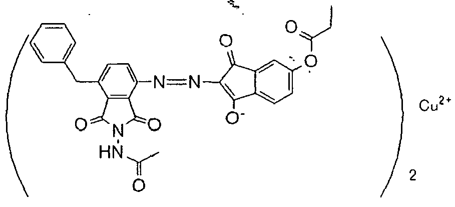 | Concrete Example (2-74) 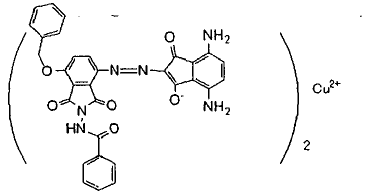 |
| Concrete Example (2-75) 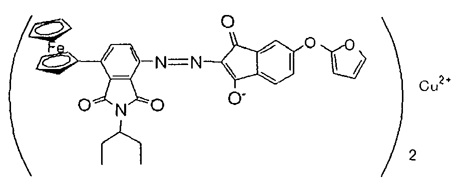 | Concrete Example (2-76) 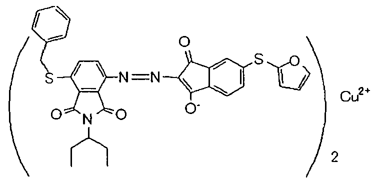 |
| Concrete Example (2-77) 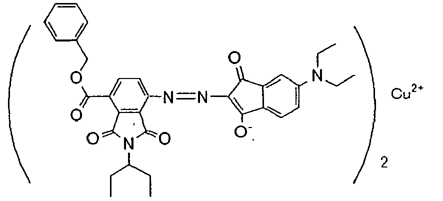 | Concrete Example (2-78) 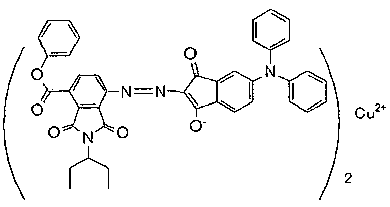 |
| Concrete Example (2-79) 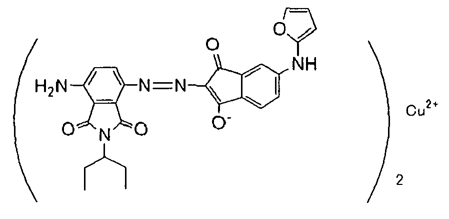 | Concrete Example (2-80) 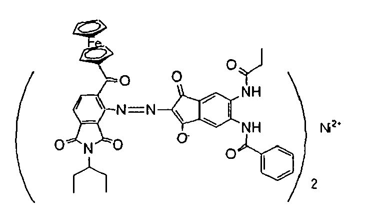 |

**[Table 1-19]**

| | |
|---|---|
| Concrete Example (3-1) 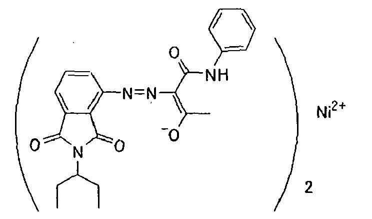 | Concrete Example (3-2) 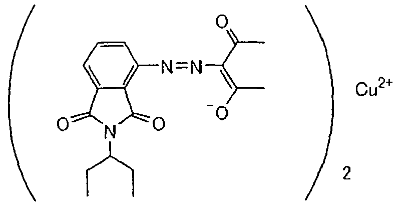 |
| Concrete Example (3-3) 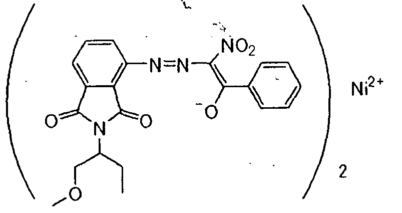 | Concrete Example (3-4) 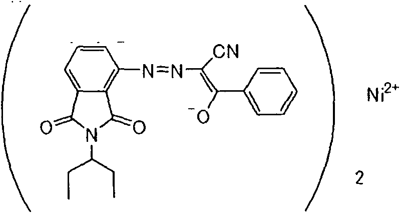 |
| Concrete Example (3-5) 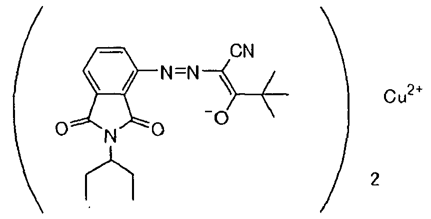 | Concrete Example (3-6) 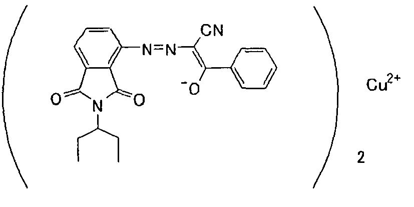 |
| Concrete Example (3-7) 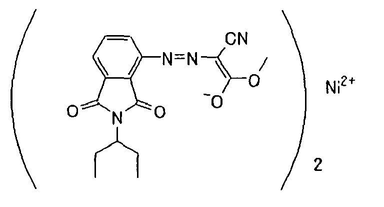 | Concrete Example (3-8) 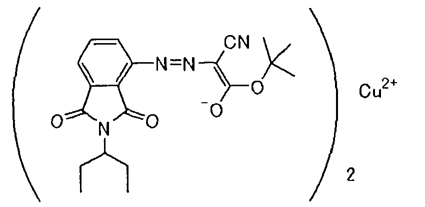 |
| Concrete Example (3-9) 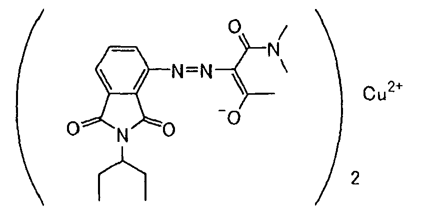 | Concrete Example (3-10) 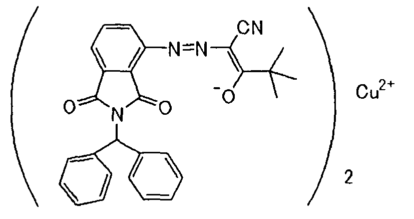 |

**[Table 1-20]**

| | |
|---|---|
| Concrete Example (3-11) 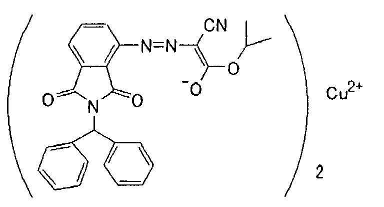 | Concrete Example (3-12) 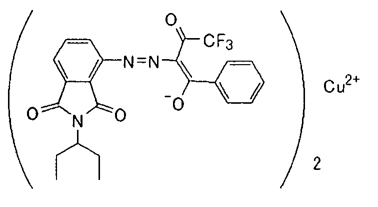 |
| Concrete Example (3-13) 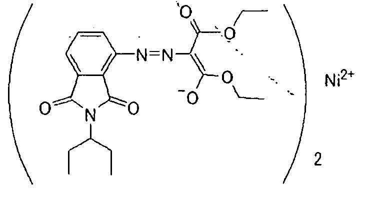 | Concrete Example (3-14) 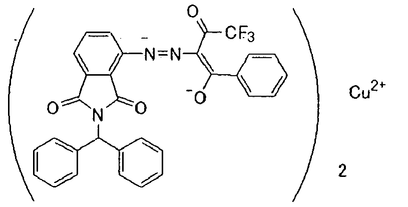 |
| Concrete Example (3-15) 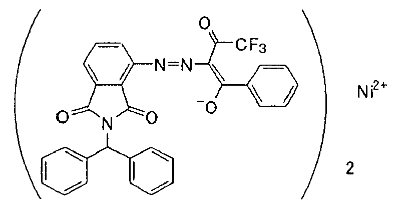 | Concrete Example (3-16) 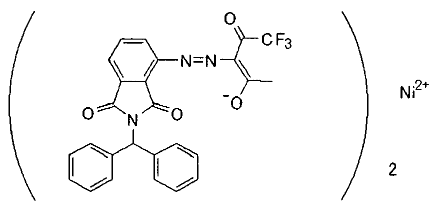 |
| Concrete Example (3-17) 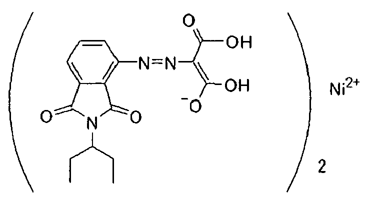 | Concrete Example (3-18) 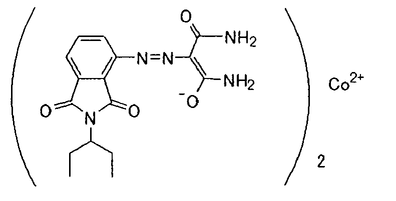 |
| Concrete Example (3-19) 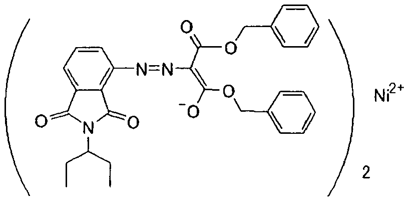 | Concrete Example (3-20) 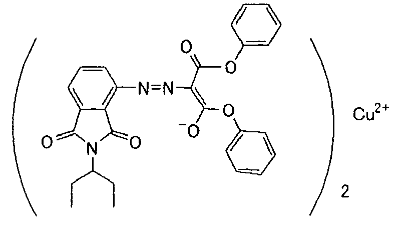 |

**[Table 1-21]**

| | |
|---|---|
| Concrete Example (3-21) 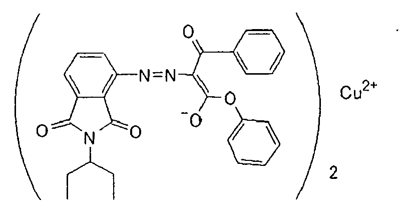 | Concrete Example (3-22) 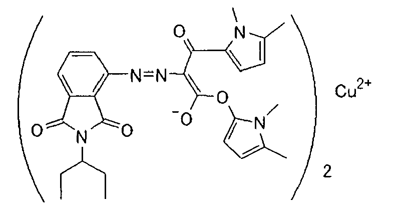 |
| Concrete Example (3-23) 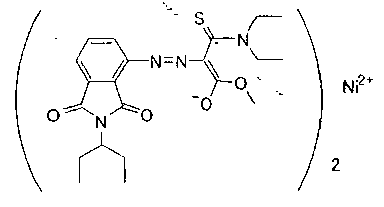 | Concrete Example (3-24) 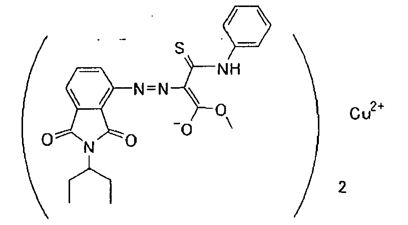 |
| Concrete Example (3-25) 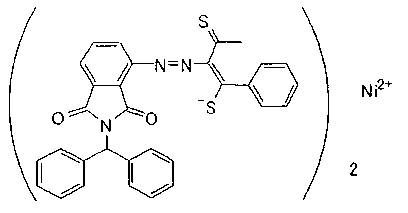 | Concrete Example (3-26) 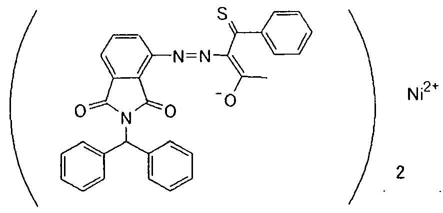 |
| Concrete Example (3-27) 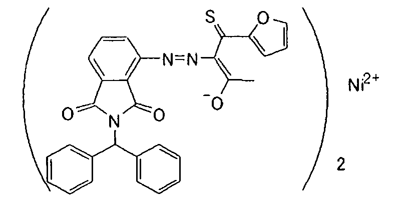 | Concrete Example (3-28) 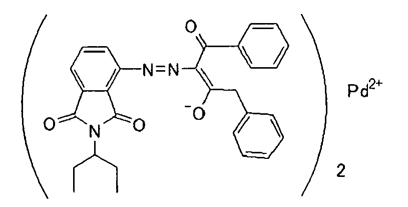 |
| Concrete Example (3-29) 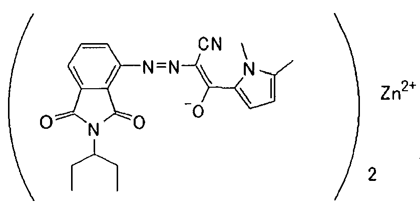 | Concrete Example (3-30) 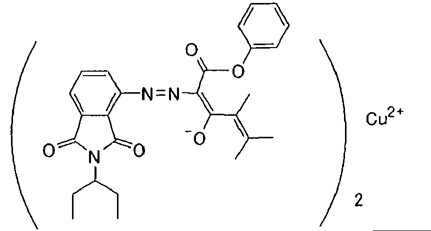 |

**[Table 1-22]**

| | |
|---|---|
| Concrete Example (3-31) 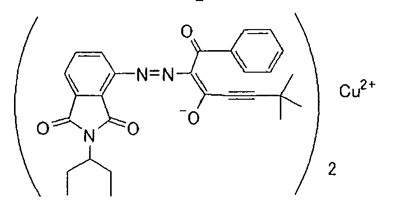 | Concrete Example (3-32) 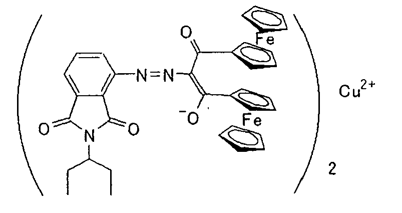 |
| Concrete Example (3-33) 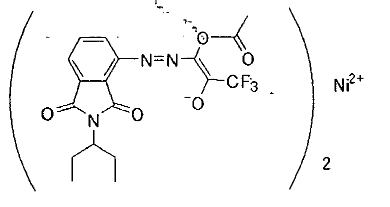 | Concrete Example (3-34) 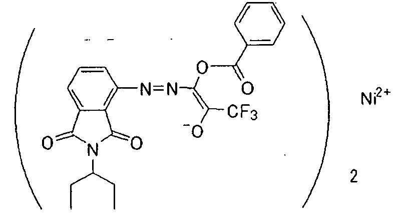 |
| Concrete Example (3-35) 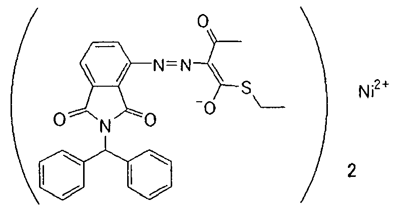 | Concrete Example (3-36) 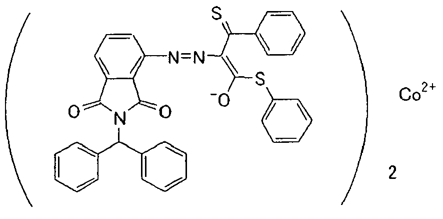 |
| Concrete Example (3-37) 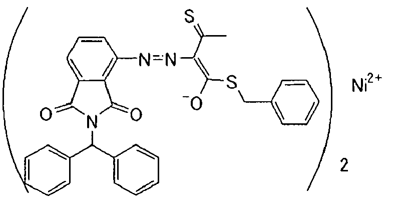 | Concrete Example (3-38) 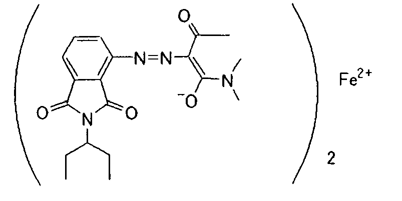 |
| Concrete Example (3-39) 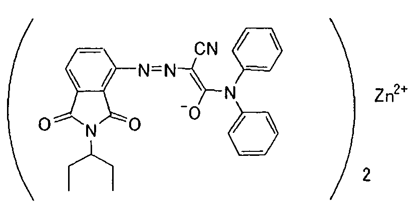 | Concrete Example (3-40) 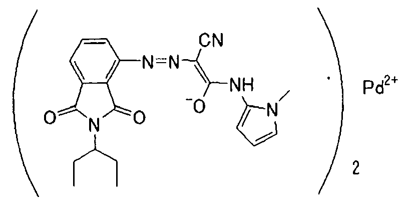 |

The metal chelate compound formed from azo compounds represented by the general formula (1) and a divalent or higher valent metal salt of the present invention, that is, an azo metal chelate compound is obtained, for example, by adding a methanol solution or an aqueous solution of a metal compound in an organic solvent such as methyl alcohol, ethyl alcohol, tetrahydrofuran, acetone or the like to azo compounds represented by the general formula (1) which is synthesized by diazotizing an amino compound represented by the general formula (8) according to a known method and reacting the diazotized compound with a coupling component represented by the general formula (9),

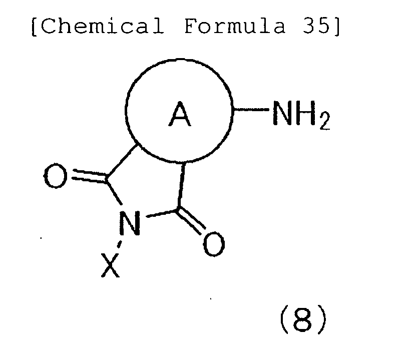

wherein, in the formula, A and X represent the same as those described above,

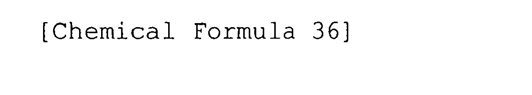

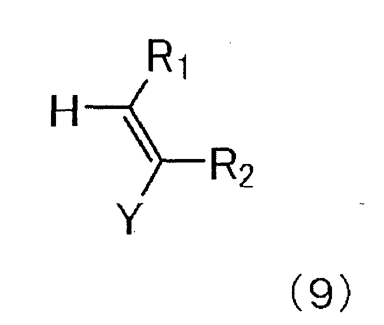

wherein, R₁, R₂ and Y represent the same as those described above

The metal chelate compound formed from azo compounds represented by the general formula (2) and a divalent or higher valent metal salt of the present invention, the metal chelate compound formed from azo compounds represented by the general formula (3) and a divalent or higher valent metal salt, the metal chelate compound formed from azo compounds represented by the general formula (4) and a divalent or higher valent metal salt, the metal chelate compound formed from azo compounds represented by the general formula (5) and a divalent or higher valent metal salt, and the metal chelate compound formed from azo compounds represented by the general formula (6) and a divalent or higher valent metal salt are also obtained in the same manner as in the metal chelate compound formed from azo compounds represented by the above general formula (1) and a divalent or higher valent metal salt.

The azo compound of the present invention is compound represented by the aforementioned general formula (2), and is the same as the compound represented by the general formula (2) explained in the aforementioned metal chelate compound.
As the preferred embodiment of the azo compound, the general formulae (10) and (11) can be cited,

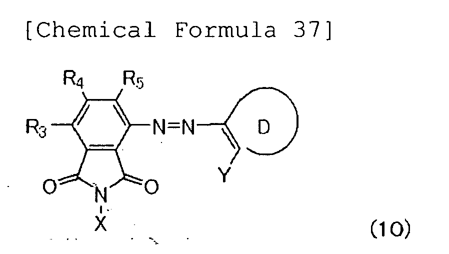

wherein, in the formula, X, R₃ to R₅ and Y represent the same as those described above, and ring D represents the general formulae (D-1) to (D-5) below,

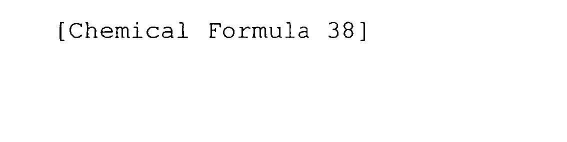

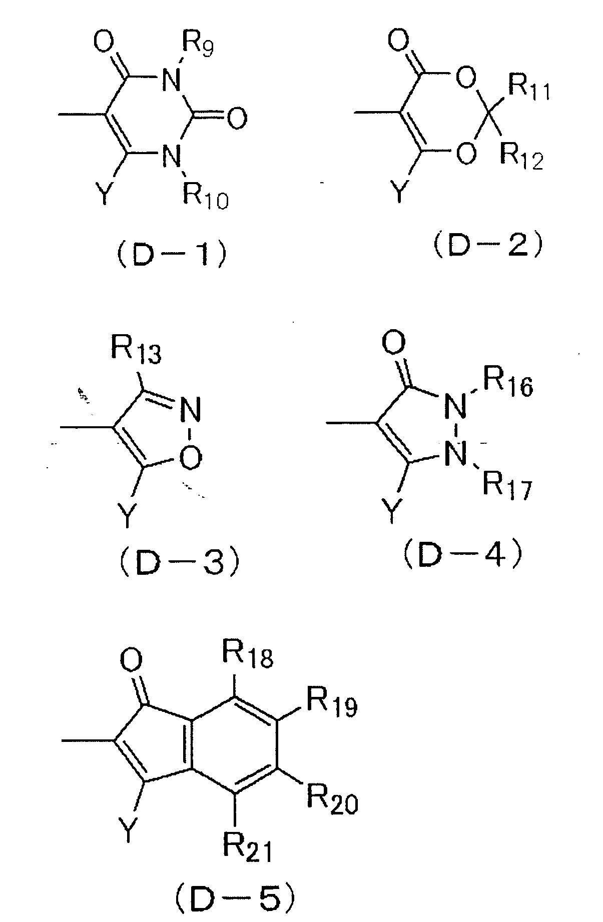

wherein, in the formula, R₉ to R₁₃ and R₁₆ to R₂₁ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylsulfonyl group, a substituted or unsubstituted arylsulfonyl group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group; and
R₁₁ and R₁₂ may be bonded with each other to form a 5 to 6-membered ring; and Y represents the same as those described above,

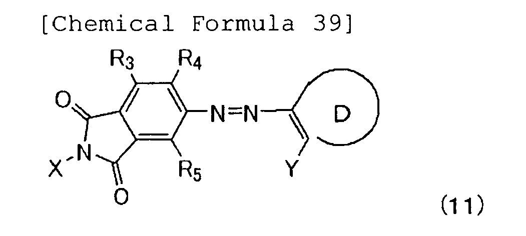

wherein, in the formula, X, R₃ to R₅, Y and ring D represent the same as those described above.

Examples of R₃ to R₅ and X in the general formulae (10) and (11), include the same preferred embodiments and concrete examples as those cited in the general formulae (3) and (4).
Examples of Y in the general formulae (10) and (11) include the same concrete examples as those cited in the general formulae (33) and (4).

Ring D represents the aforementioned general formulae (D-1) to (D-5). Examples of R₉ to R₁₃ and R₁₆ to R₂₁ in the general formulae (D-1) to (D-5) include the same preferred embodiments and concrete examples as those cited in the aforementioned general formulae (C-2), (C-3), (C-4), (C-6) and (C-7).

In the optical recording medium of the present invention, a recording layer is formed on a substrate, but the recording layer contains at least one kind of metal chelate compounds formed from azo compounds represented by the general formula (1) and a divalent or higher valent metal salt. The optical recording medium of the present invention is capable of performing recording and reproducing at the recording laser wavelength and the reproducing laser wavelength selected from wavelengths of 300 nm to 900 nm. Good C/N ratio can be attained at the recording laser wavelength and the reproducing laser wavelength selected from a wavelength range of 390 nm to 430 nm, and furthermore a wavelength range of 400 nm to 410 nm. Furthermore, the stability against reproduction light is also good, and high quality signal characteristics are attained.

A dye in a recording layer constituting the optical recording medium of the present invention is substantially composed of one or more azo metal chelate compounds according to the present invention. However, the dye-may be mixed with a compound other than the above compounds which has an absorption maximum at a wavelength of 290 nm to 690 nm and a high refractive index at a wavelength of 300 nm to 700 nm. Concrete examples of such a compound include a cyanine-based compound, a squarylium-based compound, a naphthoquinone-based compound, an anthraquinone-based compound, a tetrapyraporphyrazine-based compound, an indophenol-based compound, a pyrylium-based compound, a thiopyrylium-based compound, an azulenium-based compound, a triphenylmethane-based compound, a xanthene-based compound, an indanthrene-based compound, an indigo-based compound, a thioindigo-based compound, a merocyanine-based compound, a thiazine-based compound, an acridine-based compound, an oxazine-based compound, a dipyromethene-based compound, an oxazole-based compound, an azaporphyrin-based compound, a porphyrin-based compound and the like. A plurality of compounds may be used in combination. These compounds are mixed in an amount of about 0.1 to 30 weight %.

In forming the recording layer, an additive such as a quencher, a compound thermal decomposition accelerator, an ultraviolet absorber, an adhesive, an endothermic or endothermically decomposable compound or a polymer capable of improving solubility can be mixed in the azo metal chelate compound according to the present invention as required.

As concrete examples of the quencher, preferred are metal complexes such as an acetylacetonato-based compound, a bisdithiol-based compound such as a bisdithio-α-diketone-based compound, a bisphenyldithiol-based compound or the like, a thiocatechonal-based compound, a salicylaldehydeoxime-based compound, a thiobisphenolate-based compound and the like.

The compound thermal decomposition accelerator is not particularly limited as long as its acceleration of thermal decomposition of a compound can be confirmed by thermal reduction analysis (TG analysis) or the like. Examples of the compound thermal decomposition accelerator include metal compounds such as a metallic antiknock agent, an acetylacetonato-based metal complex and the like.
Examples of the endothermic or endothermically decomposable compound include compounds described in Japanese Patent Laid-open No. 1998-291366 and compounds having substituents described in the publication.

The aforementioned various quenchers, compound thermal decomposition accelerators and endothermic or endothermically decomposable compounds may be used singly, or two or more kinds thereof may be used in combination as required.
Alternatively, compounds having a quenching ability, a compound thermal decomposition accelerating ability, an ultraviolet absorbing ability or an adhering ability can be introduced as substituents of the azo metal chelate compound.

Furthermore, in forming the recording layer, an additive substance such as a binder, a leveling agent an antifoaming agent or the like may be added to the azo metal chelate compound according to the present invention as required. Preferable examples of the binder include polyvinyl alcohol, polyvinyl pyrrolidone, nitrocellulose, cellulose acetate, a ketone resin, an acrylic resin, a polystyrene resin, a urethane resin, polyvinyl butyral, polycarbonate, polyolefin and the like.

In forming the recording layer on a substrate, a layer composed of an inorganic substance or polymer may be formed on the substrate so as to improve solvent resistance of the substrate, reflectance, recording sensitivity or the like.

Here, as the content of the azo metal chelate compound according to the present invention in the recording layer, any content which makes recording and reproduction possible can be selected. However, the content is usually not less than 30% and preferable not less than 60%. Incidentally, it is also preferable that the content is substantially 100%.

Examples of a method for forming the recording layer include coating methods such as spin coating, spray coating, cast coating, slide coating, curtain coating, extrusion coating, wire coating, gravure coating, spread coating, roller coating, knife coating, immersion coating and the like, sputtering, chemical vapor deposition and vacuum deposition. The spin coating method is preferred because it is easy to use.

When a coating method such as spin coating or the like is used, there is used a coating solution obtained by dissolving or dispersing the azo metal chelate compound according to the present invention in a solvent such that its content is from 1 to 40 weight % and preferably from 3 to 30 weight %. In this case, however, a solvent which causes no damage on the substrate is preferably selected as the solvent. Examples of such a solvent used for the coating method include alcohol solvents such as methanol, ethanol, isopropyl alcohol, allyl alcohol, methyl cellosolve, ethyl cellosolve, tetrafluoropropanol, octafluoropentanol and the like; aliphatic or alicyclic hydrocarbon solvents such as hexane, heptane, octane, decane, cyclohexane, methylcyclohexane, ethylcyclohexane, dimethylcyclohexane and the like; aromatic hydrocarbon solvents such as toluene, xylene, benzene and the like; halogenated hydrocarbon solvents such as carbon tetrachloride, chloroform, tetrachloroethane, dibromoethane and the like; ether solvents such as diethyl ether, dibutyl ether, diisopropyl ether, dioxane and the like; ketone solvents such as acetone, 3-hydroxy-3-methyl-2-butanone and the like; ester solvents such as ethyl acetate, methyl lactate and the like; and water or the like. These may be used singly, or two or more kinds may be used in combination.
In addition, as required, the compound in the recording layer can be dispersed in a thin polymer film or the like and used.
Further, when a solvent which causes no damage on the substrate cannot be selected, sputtering, chemical vapor deposition and vacuum deposition are effective.

The film thickness of the recording layer is from 10 nm to 1,000 nm and preferably from 20 nm to 300 nm. When the film thickness of the recording layer is smaller than 10 nm, recording cannot be performed due to a great degree of thermal diffusion, or distortion may occur in a recording signal and then amplitude of the signal may become small in some cases. On the other hand, when the film thickness is larger than 1,000 nm, reflectance may be lowered, thereby degrading reproduction signal properties in some cases.

Then, on the recording layer, a reflective layer having a thickness of preferably from 50 nm to 300 nm is formed. To enhance reflectance and improve adhesion, a reflection amplifying layer or an adhesive layer can be formed between the recording layer and the reflective layer. As a material of the reflective layer, those which exhibit a sufficiently high reflectance at a wavelength of reproduction light, for example, metals such as Au, Al, Ag, Cu, Ti, Cr, Ni, Pt, Ta and Pd can be used singly or as an alloy. Of these metals, Au, Ag and Al are suitable as a material of the reflective layer because of their high reflectance. When recording and reproduction are carried out by using a blue laser, Al or Ag is suitable. In addition to these metals, the following materials may be contained. For example, metals and metalloids such as Mg, Se, Hf, V, Nb, Ru, W, Mn, Re, Fe, Co, Rh, Ir, Zn, Cd, Ga, In, Si, Ge, Te, Pb, Po, Sn and Bi can be cited. Furthermore, materials composed essentially of Ag or Al are suitable because a reflective layer having a high reflectance can be easily obtained. A multilayer film can be formed by stacking a thin non-metal film having a low refractive index and a thin non-metal film having a high refractive index alternately and used as the reflective layer.

Examples of a method for forming the reflective layer include sputtering, ion plating, chemical vapor deposition, vacuum deposition and the like. Further, on the substrate or under the reflective layer, a known inorganic or organic intermediate layer or an adhesive layer can be formed so as to improve reflectance, recording characteristics, stability against reproduction light or adhesion.

Further, a material of a protective layer to be formed on the reflective layer is not particularly limited as long as the material protects the reflective layer from an external force. Examples of an inorganic material include SiO₂, Si₃N₄, MgF₂, AlN, SnO₂, TiO₂ and the like. Meanwhile, examples of an organic material include a thermoplastic resin, a thermosetting resin, an electron beam curable resin, an ultraviolet curable resin and the like. As for the thermoplastic resin, the thermosetting resin and the like, the protective layer can be formed by dissolving them in an appropriate solvent to prepare a coating solution, and then applying and drying the coating solution. As for the ultraviolet curable resin, the protective layer can be formed by dissolving the ultraviolet curable resin as it is or in an appropriate solvent to prepare a coating solution, applying the coating solution and then irradiating the applied coating solution with ultraviolet light to cure it. As the ultraviolet curable resin, an acrylate resin such as urethane acrylate, epoxy acrylate, polyester acrylate or the like can be used. These materials may be used singly, or two or more kinds may be used in combination, and may be formed into a multilayer film as well as a single layer.

As a method for forming the protective layer, a coating method such as spin coating or cast coating, or a method such as sputtering or chemical vapor deposition is used, as in the case of the recording layer. Of these methods, spin coating is preferred.
The film thickness of the protective layer is generally in the range of 0.1 nm to 100 µm. In the present invention, it is from 3 nm to 30 µm and more preferably from 5 nm to 20 µm.
Further, a label, a bar code or the like can also be printed on the protective layer.

In addition, means for adhering a protective sheet or a substrate onto the surface of the reflective layer or means for adhering one optical recording medium to the other optical recording medium such that the surfaces of reflective layers are opposed to each other may be used.
A mirror surface of the substrate may be coated with an ultraviolet curable resin, a thin inorganic film or the like so as to protect the surface and to prevent dust or the like from sticking to the surface.

Further, when an optical recording medium as shown in Fig. 3 is prepared, a reflective layer having a thickness of preferably from 1 nm to 300 nm is formed on a substrate. To enhance reflectance and improve adhesion, a reflection amplifying layer or an adhesive layer can be formed between the recording layer and the reflective layer. As a material of the reflective layer, those which exhibit a sufficiently high reflectance at a wavelength of reproduction light, for example, metals such as Al, Ag, Ni and Pt can be used alone or as an alloy. Of these metals, Ag and Al are suitable as a material of the reflective layer because of their high reflectance. In addition to these metals, the following materials may be contained as necessary. For example, metals and metalloids such as Mg, Se, Hf, V, Nb, Ru, W, Mn, Re, Fe, Co, Rh, Ir, Zn, Cd, Ga, In, Si, Ge, Te, Pb, Po, Sn, Bi, Au, Cu, Ti, Cr, Pd and Ta may also be cited. Materials composed essentially of Ag or Al are suitable because a reflective layer having a high reflectance can be easily obtained. A multilayer film can be formed by stacking a thin non-metal film having a low refractive index and a thin non-metal film having a high refractive index alternately and used as the reflective layer.

Examples of a method for forming the reflective layer include sputtering, ion plating, chemical vapor deposition, vacuum deposition and the like. Further, on the substrate or under the reflective layer, a known inorganic or organic intermediate layer or an adhesive layer can be formed so as to improve reflectance, recording characteristics, adhesion or the like.
Then, in forming the recording layer on the reflective layer, a layer composed of an inorganic material or polymer may be formed on the reflective layer so as to improve solvent resistance of the reflective layer, reflectance, recording sensitivity or the like.

Here, as the content of the azo metal chelate compound according to the present invention in the recording layer, any content which makes recording and reproduction possible can be selected. However, the content is usually not less than 30 weight % and preferably not less than 60 weight %. Incidentally, it is also preferred that the content is substantially 100 weight %.
Examples of a method for forming the recording layer include coating methods such as spin coating, spray coating, cast coating, slide coating, curtain coating, extrusion coating, wire coating, gravure coating, spread coating, roller coating, knife coating, immersion coating and the like; sputtering, chemical vapor deposition, vacuum deposition and the like. Spin coating is preferred because it is easy to use.

When a coating method such as spin coating or the like is used, there is used a coating solution obtained by dissolving or dispersing the azo metal chelate compound according to the present invention in a solvent such that its content is from 1 to 40 weight % and preferably from 3 to 30 weight %. In this case, however, a solvent which causes no damage on the reflective layer is preferably selected as the solvent. Examples of such a solvent used for the coating method include alcohol solvents such as methanol, ethanol, isopropyl alcohol, allyl alcohol, methyl cellosolve, ethyl cellosolve, tetrafluoropropanol, octafluoropentanol and the like; aliphatic or alicyclic hydrocarbon solvents such as hexane, heptane, octane, decane, cyclohexane, methylcyclohexane, ethylcyclohexane, dimethylcyclohexane and the like; aromatic hydrocarbon solvents such as toluene, xylene, benzene and the like; halogenated hydrocarbon solvents such as carbon tetrachloride, chloroform, tetrachloroethane, dibromoethane and the like; ether solvents such as diethyl ether, dibutyl ether, diisopropyl ether, dioxane and the like; ketone solvents such as acetone, 3-hydroxy-3-methyl-2-butanone and the like; ester solvents such as ethyl acetate, methyl lactate and the like; and water. These may be used singly, or two or more kinds may be used in combination.
In addition, as required, the compound in the recording layer can be dispersed in a thin polymer film or the like and used.
Meanwhile, when a solvent which causes no damage on the reflective layer cannot be selected, sputtering, chemical vapor deposition and vacuum deposition are effective.

The film thickness of the recording layer is usually from 1 nm to 1,000 nm and preferably from 5 nm to 300 nm. When the film thickness of the recording layer is smaller than 1 nm, recording cannot be performed, or distortion may occur in a recording signal and then amplitude of the signal may become small in some cases. On the other hand, when the film thickness is larger than 1,000 nm, reflectance may be lowered, thereby degrading reproduction signal properties in some case.

Further, a material of the protective layer to be formed on the recording layer is not particularly limited as long as the material protects the recording layer from being adversely affected from outside, for example, by an external force, atmosphere or the like. Examples of an inorganic material include SiO₂, Si₃N₄, MgF₂, AlN, SnO₂, TiO₂ and the like. Meanwhile, examples of an organic material include a thermoplastic resin, a thermosetting resin, an electron beam curable resin, an ultraviolet curable resin and the like. As for the thermoplastic resin and the thermosetting resin, the protective layer can be formed by dissolving them in an appropriate solvent to prepare a coating solution, and then applying and drying the coating solution. As for the ultraviolet curable resin, the protective layer can be formed by dissolving the ultraviolet curable resin as it is or in an appropriate solvent to prepare a coating solution, applying the coating solution and then irradiating the applied coating solution with ultraviolet light to cure it. As the ultraviolet curable resin, an acrylate resin such as urethane acrylate, epoxy acrylate, polyester acrylate or the like can be used. These materials may be used singly, or two or more kinds may be used in combination, and may be formed into a multilayer film as well as a single layer.

As a method for forming the protective layer, a coating method such as spin coating or cast coating, or a method such as sputtering or chemical vapor deposition is used, as in the case of the recording layer. Of these methods, spin coating is preferred. Incidentally, when a solvent which causes no damage on the recording layer cannot be selected, sputtering, chemical vapor deposition and vacuum deposition can be cited, as in the case of forming the recording layer. Furthermore, as described in Japanese Patent Laid-open No. 1999-273147, the protective layer can be formed through an adhesive layer such as a pressure-sensitive adhesion sheet, a dry photopolymer sheet or the like, or the pressure-sensitive adhesion sheet or the dry photopolymer sheet itself can be used as the protective layer.

Furthermore, in forming the protective layer on the recording layer, a layer composed of an inorganic material or polymer may be formed on the recording layer so as to improve solvent resistance of the recording layer, reflectance, recording sensitivity or the like.
The film thickness of the protective layer is generally in the range of 0.01 µm to 1,000 µm, but can be from 0.1 µm to 100 µm and furthermore from 1 µm to 20 µm in some cases.

In addition, means for adhering the protective sheet or the reflective layer onto the surface of the substrate or means for adhering one optical recording medium to the other optical recording medium such that the surfaces of substrates are opposed to each other may be used.
A surface of the protective layer may be coated with an ultraviolet curable resin, a thin inorganic film or the like so as to protect the surface and to prevent dust or the like from sticking to the surface.

In the optical recording medium of the present invention, for example, a case-type protective unit may be housed for protecting a disk as seen in a flexible disk or a magneto-optical disk so as to protect the whole medium. As a material of the unit, plastic or metal such as aluminum or the like can be used.

A material of the substrate may basically be one which is transparent at wavelengths of recording light and reproduction light. As a material of the supporting substrate, when irradiation is carried out with a blue-violet laser through a substrate 11 as shown in Fig. 4, there is used a transparent material of a polymer material such as an acrylic resin, a polyethylene resin, a polycarbonate resin, a polyolefin resin or an epoxy resin, or an inorganic material such as glass and the like. On the other hand, as shown in the configuration of Fig. 5, when irradiation is carried out with a laser from a surface of the light transmitting layer 15' opposite to the substrate 11' , a material of the substrate does not need to meet the optical requirements and can be widely selected from materials. From the viewpoints of mechanical properties required for the substrate and productivity of the substrate, preferred are materials capable of injection molding or cast molding such as an acrylic resin, a polycarbonate resin, a polyolefin resin and the like. These substrate materials may be molded into a substrate in the disc-like form by an injection molding method or the like.

Furthermore, a guide groove and/or pre-pit in submicron order may be formed spirally or in a concentric circle on a surface layer of the substrate, if necessary. The guide groove and pre-pit are preferably provided at the time of the formation of the substrate. They can be provided by injection molding using a disc stamper or by a thermal transfer method using a photopolymer. Incidentally, the guide groove and/or pre-pit may be formed in the light transmission layer 15' in Fig. 5, and these guide groove and pre-pit can be provided in the same way. The pitch and depth of the guide groove are preferably selected from a range of 0.25 to 0.80 µm for pitch and a range of 20 to 150 nm for depth in the case of HD-DVD-R to be recorded with the density higher than that of DVD.

When it is used as an optical disk, the optical recording medium of the present invention may usually be a disc shape of about 1.2 mm in thickness and about 80 to 120 mm in diameter. There may be a hole of about 15 mm in diameter in the center.
Here, as a laser for a wavelength of 300 nm to 500 nm mentioned in the present invention, there can be exemplified, for example, a dye laser which can select a wavelength from a wide visible radiation range, a gas laser such as a nitrogen laser (337 nm), ion lasers such as a helium cadmium laser for a wavelength of 445 nm and an argon laser for a wavelength of 457 or 488 nm, a GaN laser for a wavelength of 400 to 410 nm, a laser which generates the second harmonic of 430 nm of an infrared laser for a wavelength of 860 nm using Cr-doped LiSnAlF₆, as well as a semiconductor laser such as a visible semiconductor laser for a wavelength of 415 nm, 425 nm or the like. In the present invention, an appropriate laser can be selected from the above lasers according to a wavelength to which a recording layer for recording or reproduction is sensitive. High-density recording and reproduction are each possible at one wavelength or a plurality of wavelengths selected from the above lasers.

### EXAMPLES

Hereinafter, Examples of the present invention will be described. However, the present invention shall not be limited in any way by these Examples.

### Example 1

### Synthesis of Compound of Concrete Example (1)

In a mixed solution of 56 g of acetic acid and 28 g of propionic acid, was dissolved 8. 0 g of a compound represented by the following formula (8-a) and 12.24 g of 40% nitrosylsulfuric acid was added dropwise thereto at 0 to 5 degree centigrade. The resulting solution was stirred at the same temperature for 1 hour and diazotized. In 46 g of 3% aqueous caustic soda solution, were dissolved 7.2 g of a compound represented by the following formula (9-a) and 2.1 g of urea and, the obtained diazo solution was introduced dropwise thereto at 0 to 5 degree centigrade. The resulting solution was stirred at the same temperature for 2 hours, and then the precipitated crystal was filtered out, washed with water and dried to obtain 13.6 g of a compound represented by the following formula (1-a),

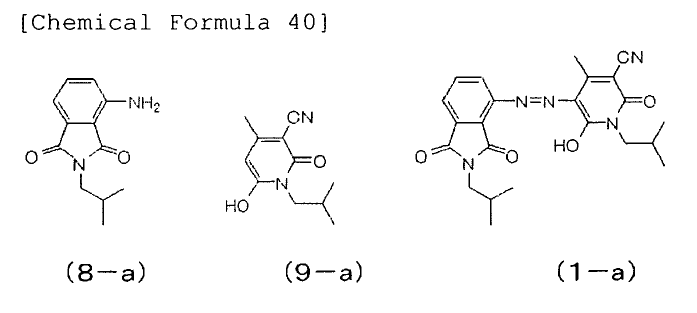

Then, 3.3 g of the compound represented by the formula (1-a) was dissolved in 165 g of methanol, and 1.03 g of nickel acetate tetrahydrate was added thereto at 50 to 55 degree centigrade. The resulting mixture was stirred at the same temperature for 3 hours. The precipitated crystal was filtered out, washed with water and dried to obtain 2.99 g of a compound of Concrete Example (1),

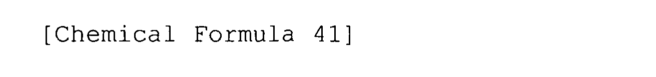

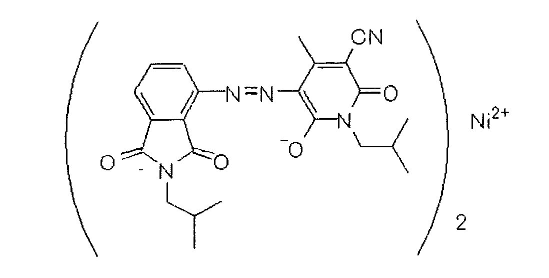

### Compound of Concrete Example (1)

. From the following analytical results, the obtained compound was confirmed as a desired product.
Elemental Analytical Value (C₄₆H₄₈N₁₀ O₈Ni)

**[Table 2]**

| | C | H | N |
|---|---|---|---|
| Calculated value (%) | 59.56 | 5.22 | 15.10 |
| Analytical value (%) | 59.54 | 5.19 | 15.14 |

MS (m/e) : 926 (M⁺)
The thus-obtained compound exhibited an absorption maximum at a wavelength of 479.5 nm in a chloroform solution and the gram extinction coefficient of the compound was 1.05 × 10⁵ ml/g·cm.

### Example 2

### Synthesis of Compound of Concrete Example (2)

In 165 g of methanol, was dissolved 3.3 g of the compound represented by the formula (1-a) and 1.04 g of cobalt acetate tetrahydrate was added thereto at 50 to 55 degree centigrade. The resulting mixture was stirred at the same temperature for 3 hours. The precipitated crystal was filtered out, washed with water and dried to obtain 3.09 g of a compound of Concrete Example (2),

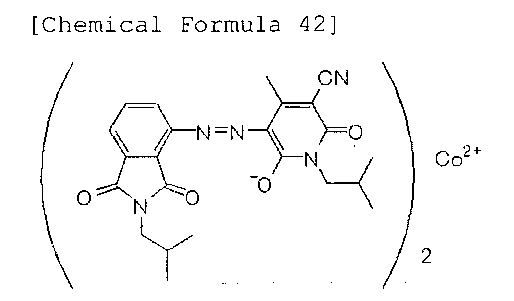

### Compound of Concrete Example (2)

From the following analytical results, the obtained compound was confirmed as a desired product.
Elemental Analytical Value (C₄₆H₄₈N₁₀ O₈Co)

**[Table 3]**

| | C | H | N |
|---|---|---|---|
| Calculated value (%) | 59.54 | 5.21 | 15.10 |
| Analytical value (%) | 59.54 | 5.23 | 15.12 |

MS (m/e) : 927 (M⁺)
The thus-obtained compound exhibited an absorption maximum at a wavelength of 462.0 nm in a chloroform solution and the gram extinction coefficient of the compound was 0.75 × 10⁵ ml/g·cm.

### Example 3

### Synthesis of Compound of Concrete Example (3)

In 165 g of methanol, was dissolved 3.3 g of the compound represented by the formula (1-a) and 0.76 g of copper acetate was added thereto at 50 to 55 degree centigrade. The resulting mixture was stirred at the same temperature for 3 hours. The precipitated crystal was filtered out, washed with water and dried to obtain 2.90 g of a compound of Concrete Example (3),

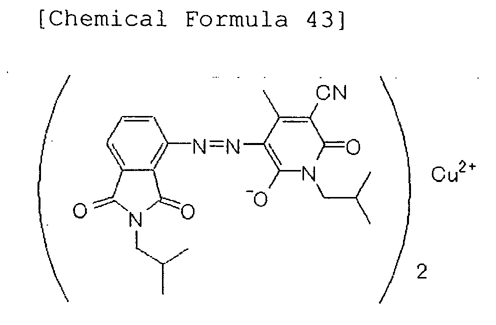

### Compound of Concrete Example (3)

From the following analytical results, the obtained compound was confirmed as a desired product.
Elemental Analytical Value (C₄₆H₄₈N₁₀ O₈Cu)

**[Table 4]**

| | C | H | N |
|---|---|---|---|
| Calculated value (%) | 59.25 | 5.19 | 15.02 |
| Analytical value (%) | 59.21 | 5.22 | 14.99 |

MS (m/e) : 931 (M⁺)
The thus-obtained compound exhibited an absorption maximum at a wavelength of 451.5 nm in a chloroform solution and the gram extinction coefficient of the compound was 0.75 × 10⁵ ml/g·cm.

### Example 4

### Synthesis of Compound of Concrete Example (4)

In a mixed solution of 56 g of acetic acid and 28 g of propionic acid, was dissolved 8.0 g of a compound represented by the following formula (8-a) and 12.24 g of 40% nitrosylsulfuric acid was added dropwise thereto at 0 to 5 degree centigrade. The resulting solution was stirred at the same temperature for 1 hour and diazotized. In 48 g of 3% aqueous caustic soda solution, were dissolved 9.37 g of a compound represented by the following formula (9-b) and 2.16 g of urea and, the obtained diazo solution was introduced dropwise thereto at 0 to 5 degree centigrade. The resulting solution was stirred at the same temperature for 3 hours, and then the precipitated crystal was filtered out, washed with water and dried to obtain 15.67 g of a compound represented by the following formula (1-b),

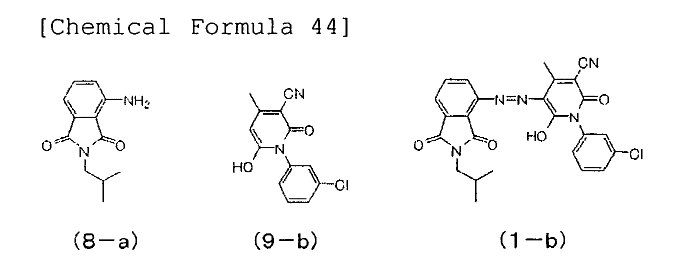

Then, 5.0 g of the compound represented by the formula (1-b) was dissolved in 250 g of methanol, and 1.40 g of nickel acetate tetrahydrate was added thereto at 50 to 55 degree centigrade. The resulting mixture was stirred at the same temperature for 3 hours. The precipitated crystal was filtered out, washed with water and dried to obtain 5.13 g of a compound of Concrete Example (4),

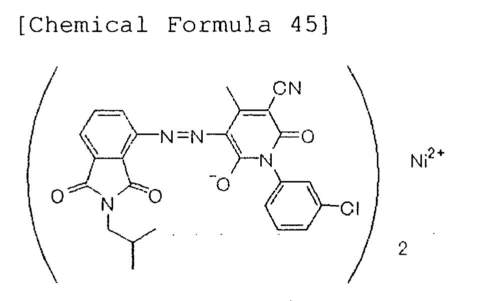

### Compound of Concrete Example (4)

From the following analytical results, the obtained compound was confirmed as a desired product.
Elemental Analytical Value (C₅₀H₃₈N₁₀ O₈Cl₂Ni)

**[Table 5]**

| | C | H | N |
|---|---|---|---|
| Calculated value (%) | 57.94 | 3.70 | 13.51 |
| Analytical value (%) | 57.92 | 3.75 | 13.48 |

MS(m/e): 1034(M⁺)
The thus-obtained compound exhibited an absorption maximum at a wavelength of 477.0 nm in a chloroform solution and the gram extinction coefficient of the compound was 0.80 × 10⁵ ml/g·cm.

### Example 5

### Synthesis of Compound of Concrete Example (14)

In a mixed solution of 36 g of acetic acid and 18 g of propionic acid, was dissolved 3.0 g of a compound represented by the following formula (8-a) and 4.65 g of 40% nitrosylsulfuric acid was added dropwise thereto at 0 to 5 degree centigrade. The resulting solution was stirred at the same temperature for 1 hour and diazotized. In 30 g of 3% aqueous caustic soda solution, were dissolved 2. 13 g of a compound represented by the following formula (9-c) and 0.75 g of urea and, the obtained diazo solution was introduced dropwise thereto at 0 to 5 degree centigrade. The resulting solution was stirred at the same temperature for 2 hours, and then the precipitated crystal was filtered out, washed with water and dried to obtain 4.79 g of a compound represented by the following formula (1-c),

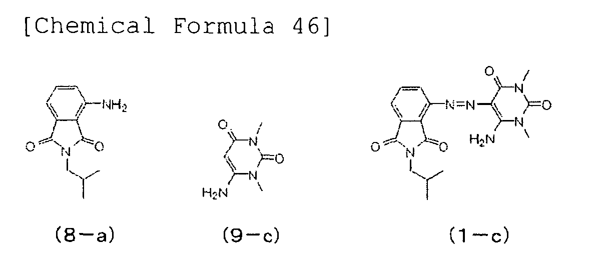

Then, 3.4 g of the compound represented by the formula (1-c) was dissolved in 240 g of 2-propanol, and 0.88 g of copper acetate was added thereto at 75 to 80 degree centigrade. The resulting mixture was stirred at the same temperature for 4 hours. The precipitated crystal was filtered out, washed with water and dried to obtain 2.75 g of a compound of Concrete Example (14),

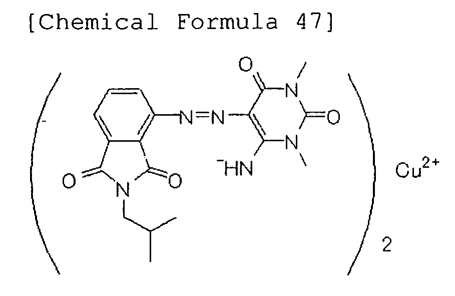

### Compound of Concrete Example (14)

From the following analytical results, the obtained compound was confirmed as a desired product.
Elemental Analytical Value (C₃₆H₃₆N₁₂ O₈Cu)

**[Table 6]**

| | C | H | N |
|---|---|---|---|
| Calculated value (%) | 52.20 | 4.38 | 20.29 |
| Analytical value (%) | 52.20 | 4.36 | 19.98 |

MS(m/e): 827 (M⁺)
The thus-obtained compound exhibited an absorption maximum at a wavelength of 421.0 nm in a chloroform solution and the gram extinction coefficient of the compound was 0.42 × 10⁵ ml/g·cm.

### Example 6

### Synthesis of Compound of Concrete Example (16)

In 40 g of acetic acid, was dissolved 4.0 g of a compound represented by the following formula (8-a) and 6.40 g of 40% nitrosylsulfuric acid was added dropwise thereto at 5 to 10 degree centigrade. The resulting solution was stirred at the same temperature for 1 hour and diazotized. In 30 g of methanol, were dissolved 2.64 g of a compound represented by the following formula (9-d) and 1.10 g of urea and, the obtained diazo solution was introduced dropwise thereto at 0 to 5 degree centigrade. The resulting solution was stirred at the same temperature for 2 hours, and then the precipitated crystal was filtered out, washed with water and dried to obtain 6.43 g of a compound represented by the following formula (1-d),

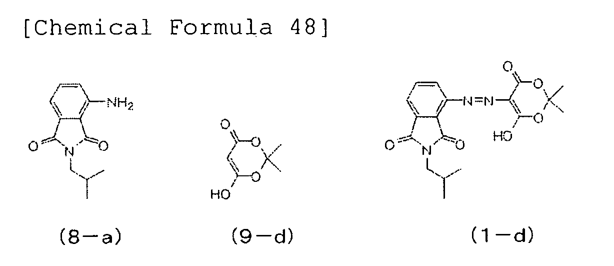

Then, 4.0 g of the compound represented by the formula (1-d) was dissolved in a mixed solvent of 120 g of methanol and 40 g of N,N-dimethylformamide, and 1.49 g of nickel acetate tetrahydrate was added thereto at 60 to 65 degree centigrade. The resulting mixture was stirred at the same temperature for 3 hours. The precipitated crystal was filtered out, washed with water and dried to obtain 3.87 g of a compound of Concrete Example (16),

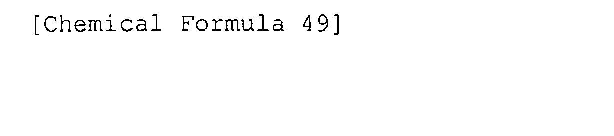

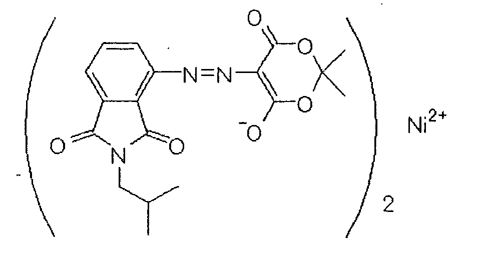

### Compound of Concrete Example (16)

From the following analytical results, the obtained compound was confirmed as a desired product.
Elemental Analytical Value (C₃₆H₃₆N₆ O₁₂Ni)

**[Table 7]**

| | C | H | N |
|---|---|---|---|
| Calculated value (%) | 53.82 | 4.52 | 10.46 |
| Analytical value (%) | 53.75 | 4.50 | 10.45 |

MS(m/e): 802(M⁺)
The thus-obtained compound exhibited an absorption maximum at a wavelength of 412.5 nm in a chloroform solution and the gram extinction coefficient of the compound was 0.52 × 10⁵ ml/g·cm.

### Example 7

### Synthesis of Compound of Concrete Example (23)

In a mixed solution of 36 g of acetic acid and 18 g of propionic acid, was dissolved 3.0 g of a compound represented by the following formula (8-a) and 4.65 g of 40% nitrosylsulfuric acid was added dropwise thereto at 0 to 5 degree centigrade. The resulting solution was stirred at the same temperature for 1 hour and diazotized. In 25 g of methanol, were dissolved 2.22 g of a compound represented by the following formula (9-e) and 0.83 g of urea and, the obtained diazo solution was introduced dropwise thereto at 0 to 5 degree centigrade. The resulting solution was stirred at the same temperature for 2 hours, and then the precipitated crystal was filtered out, washed with water and dried to obtain 4.40 g of a compound represented by the following formula (1-e),

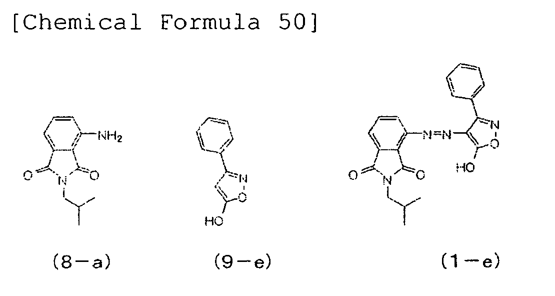

Then, 4.0 g of the compound represented by the formula (1-e) was dissolved in 160 g of methanol, and 1.40 g of nickel acetate tetrahydrate was added thereto at 60 to 65 degree centigrade. The resulting mixture was stirred at the same temperature for 5 hours. The precipitated crystal was filtered out, washed with water and dried to obtain 3.73 g of a compound of Concrete Example (23),

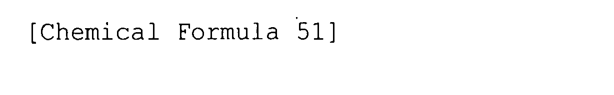

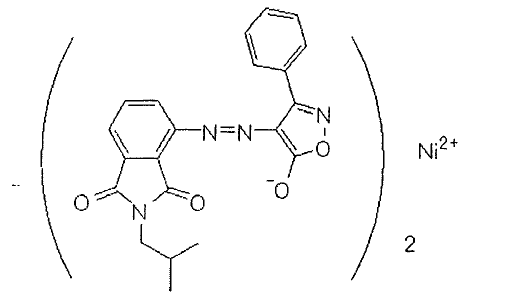

### Compound of Concrete Example (23)

From the following analytical results, the obtained compound was confirmed as a desired product.
Elemental Analytical Value (C₄₂H₃₄N₈ O₈Ni)

**[Table 8]**

| | C | H | N |
|---|---|---|---|
| Calculated value (%) | 60.24 | 4.09 | 13.38 |
| Analytical value (%) | 60.10 | 4.09 | 13.35 |

MS (m/e) : 836(M⁺)
The thus-obtained compound exhibited an absorption maximum at a wavelength of 427.0 nm in a chloroform solution and the gram extinction coefficient of the compound was 0.52 × 10⁵ ml/g·cm.

### Example 8

### Synthesis of Compound of Concrete Example (31)

In a mixed solution of 60 g of acetic acid and 30 g of propionic acid, was dissolved 6.0 g of a compound represented by the following formula (8-b) and 8.58 g of 40% nitrosylsulfuric acid was added dropwise thereto at 0 to 5 degree centigrade. The resulting solution was stirred at the same temperature for 1 hour and diazotized. In 50 g of 3% aqueous caustic soda solution, were dissolved 6.39 g of a compound represented by the following formula (9-f) and 1.48 g of urea and, the obtained diazo solution was introduced dropwise thereto at 0 to 5 degree centigrade. The resulting solution was stirred at the same temperature for 3 hours, and then the precipitated crystal was filtered out, washed with water and dried to obtain 11.90 g of a compound represented by the following formula (1-f),

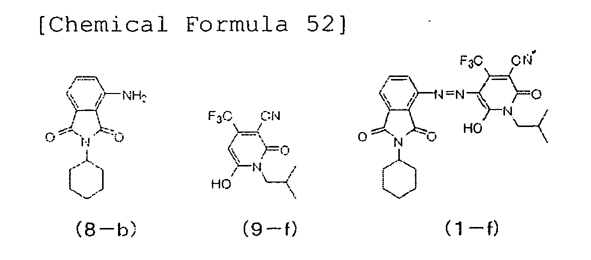

Then, 5.0 g of the compound represented by the formula (1-f) was dissolved in 200 g of methanol, and 0.97 g of copper acetate was added thereto at 50 to 55 degree centigrade. The resulting mixture was stirred at the same temperature for 2 hours. The precipitated crystal was filtered out, washed with water and dried to obtain 4.82 g of a compound of Concrete Example (31),

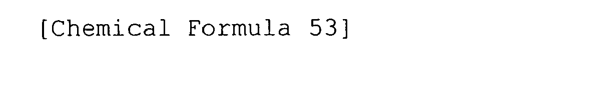

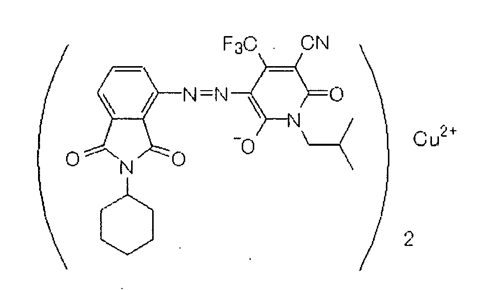

### Compound of Concrete Example (31)

From the following analytical results, the obtained compound was confirmed as a desired product.
Elemental Analytical Value (C₅₀H₄₆N₁₀ O₈F₆Cu)

**[Table 9]**

| | C | H | N |
|---|---|---|---|
| Calculated value (%) | 54.97 | 4.24 | 12.82 |
| Analytical value (%) | 54.99 | 4.20 | 12.80 |

MS(m/e): 1091(M⁺)
The thus-obtained compound exhibited an absorption maximum at a wavelength of 453.5 nm in a chloroform solution and the gram extinction coefficient of the compound was 0.64 × 10⁵ ml/g·cm.

### Example 9

### Synthesis of Compound of Concrete Example (38)

In 50 g of acetic acid, was dissolved 5.0 g of a compound represented by the following formula (8-c) and 7.18 g of 40% nitrosylsulfuric acid was added dropwise thereto at 5 to 10 degree centigrade. The resulting solution was stirred at the same temperature for 1 hour and diazotized. In 60 g of methanol, were dissolved 3.53 g of a compound represented by the following formula (9-g) and 1.29 g of urea and, the obtained diazo solution was introduced dropwise thereto at 0 to 5 degree centigrade. The resulting solution was stirred at the same temperature for 3 hours, and then the precipitated crystal was filtered out, washed with water and dried to obtain 7.57 g of a compound represented by the following formula (1-g),

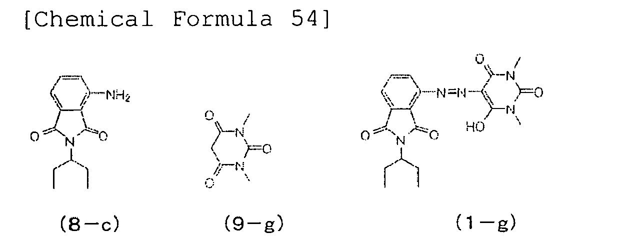

Then, 4.0 g of the compound represented by the formula (1-g) was dissolved in a mixed solvent of 100 g of methanol and 20 g of N,N-dimethylformamide, and 1.04 g of copper acetate was added thereto at 60 to 65 degree centigrade. The resulting mixture was stirred at the same temperature for 3 hours. The precipitated crystal was filtered out, washed with water and dried to obtain 4.17 g of a compound of Concrete Example (38),

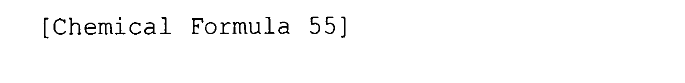

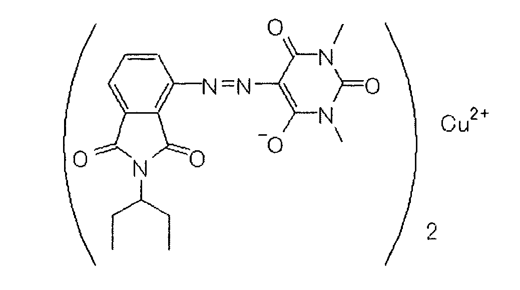

### Compound of Concrete Example (38)

From the following analytical results, the obtained compound was confirmed as a desired product.
Elemental Analytical Value (C₃₈H₄₀N₁₀O₁₀Cu)

**[Table 10]**

| | C | H | N |
|---|---|---|---|
| Calculated value (%) | 53.05 | 4.69 | 16.28 |
| Analytical value (%) | 53.10 | 4.68 | 16.22 |

MS(m/e): 859(M⁺)
The thus-obtained compound exhibited an absorption maximum at a wavelength of 405.0 nm in a chloroform solution and the gram extinction coefficient of the compound was 0.55 × 10⁵ ml/g·cm.

### Example 10

### Synthesis of Compound of Concrete Example (41)

In a mixed solution of 80 g of acetic acid and 20 g of propionic acid, was dissolved 5. 0 g of a compound represented by the following formula (8-d) and 5.45 g of 40% nitrosylsulfuric acid was added dropwise thereto at 0 to 5 degree centigrade. The resulting solution was stirred at the same temperature for 1 hour and diazotized. In 60 g of 3% aqueous caustic soda solution, were dissolved 3.47 g of a compound represented by the following formula (9-a) and 1.01 g of urea and, the obtained diazo solution was introduced dropwise thereto at 0 to 5 degree centigrade. The resulting solution was stirred at the same temperature for 2 hours, and then the precipitated crystal was filtered out, washed with water and dried to obtain 7.36 g of a compound represented by the following formula (1-h),

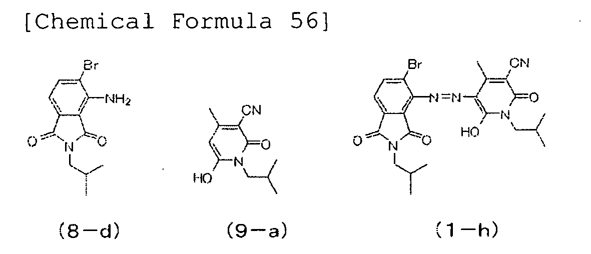

Then, 3.0 g of the compound represented by the formula (1-h) was dissolved in 90 g of methanol, and 0.64 g of copper acetate was added thereto at 50 to 55 degree centigrade. The resulting mixture was stirred at the same temperature for 4 hours. The precipitated crystal was filtered out, washed with water and dried to obtain 2.89 g of a compound of Concrete Example (41),

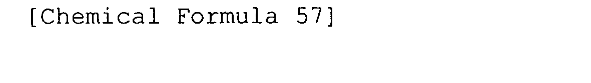

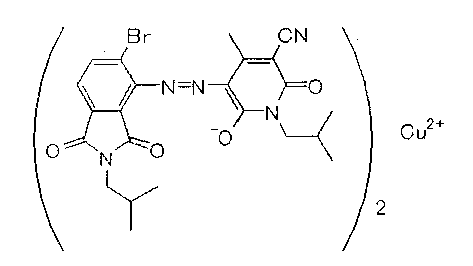

### Compound of Concrete Example (41)

From the following analytical results, the obtained compound was confirmed as a desired product.
Elemental Analytical Value (C₄₆H₄₆N₁₀ O₈Br₂Cu)

**[Table 11]**

| | C | H | N |
|---|---|---|---|
| Calculated value (%) | 50.67 | 4.25 | 12.85 |
| Analytical value (%) | 50.65 | 4.22 | 12.81 |

MS(m/e): 1087(M⁺)
The thus-obtained compound exhibited an absorption maximum at a wavelength of 446.5 nm in a chloroform solution and the gram extinction coefficient of the compound was 0.65 × 10⁵ ml/g·cm.

### Example 11

### Synthesis of Compound of Concrete Example (43)

In 50 g of acetic acid, was dissolved 5.0 g of a compound represented by the following formula (8-d) and 7.18 g of 40% nitrosylsulfuric acid was added dropwise thereto at 5 to 10 degree centigrade. The resulting solution was stirred at the same temperature for 1 hour and diazotized. In 50 g of methanol, were dissolved 2. 63 g of a compound represented by the following formula (9-g) and 1.01 g of urea and, the obtained diazo solution was introduced dropwise thereto at 0 to 5 degree centigrade. The resulting solution was stirred at the same temperature for 3 hours, and then the precipitated crystal was filtered out, washed with water and dried to obtain 7.27 g of a compound represented by the following formula (1-i),

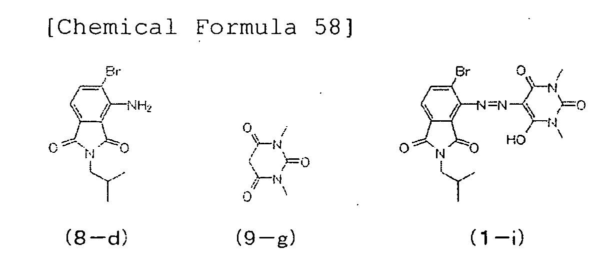

Then, 3.0 g of the compound represented by the formula (1-i) was dissolved in 120 g of ethanol, and 0.96 g of nickel acetate tetrahydrate was added thereto at 70 to 75 degree centigrade. The resulting mixture was stirred at the same temperature for 2 hours. The precipitated crystal was filtered out, washed with water and dried to obtain 2.64 g of a compound of Concrete Example (43),

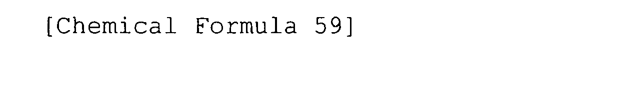

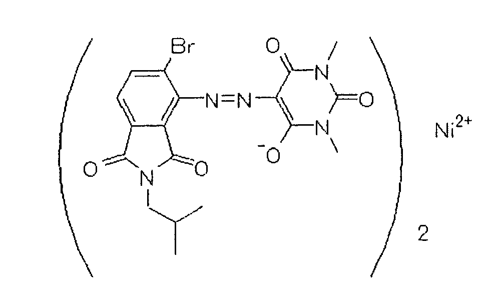

### Compound of Concrete Example (43)

From the following analytical results, the obtained compound was confirmed as a desired product.
Elemental Analytical Value (C₃₆H₃₄N₁₀O₁₀Br₂Ni)

**[Table 12]**

| | C | H | N |
|---|---|---|---|
| Calculated value (%) | 43.89 | 3.48 | 14.22 |
| Analytical value (%) | 43.91 | 3.44 | 14.21 |

MS(m/e): 982 (M⁺)
The thus-obtained compound exhibited an absorption maximum at a wavelength of 433.0 nm in a chloroform solution and the gram extinction coefficient of the compound was 0.56 × 10⁵ ml/g·cm.

### Example 12

### Synthesis of Compound of Concrete Example (53)

In a mixed solution of 80 g of acetic acid and 20 g of propionic acid, was dissolved 4.0 g of a compound represented by the following formula (8-e) and 4.92 g of 40% nitrosylsulfuric acid was added dropwise thereto at 0 to 5 degree centigrade. The resulting solution was stirred at the same temperature for 1 hour and diazotized. In 100 g of methanol, were dissolved 2.49 g of a compound represented by the following formula (9-g) and 0.91 g of urea and, the obtained diazo solution was introduced dropwise thereto at 0 to 5 degree centigrade. The resulting solution was stirred at the same temperature for 3 hours, and then the precipitated crystal was filtered out, washed with water and dried to obtain 6.21 g of a compound represented by the following formula (1-j),

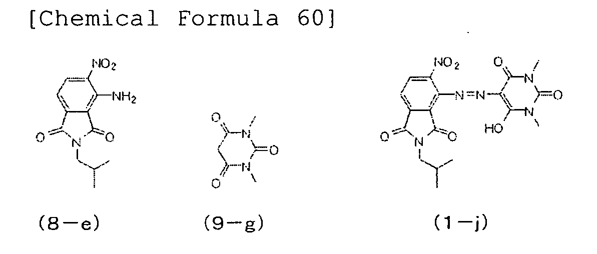

Then, 4.0 g of the compound represented by the formula (1-j) was dissolved in 120 g of 2-propanol, and 1.27 g of nickel acetate tetrahydrate was added thereto at 70 to 75 degree centigrade. The resulting mixture was stirred at the same temperature for 3 hours. The precipitated crystal was filtered out, washed with water and dried to obtain 3.84 g of a compound of Concrete Example (53),

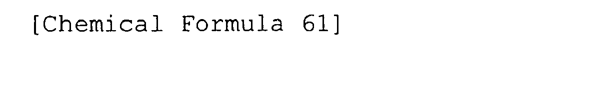

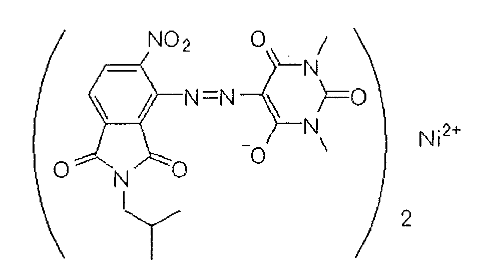

### Compound of Concrete Example (53)

From the following analytical results, the obtained compound was confirmed as a desired product.
Elemental Analytical Value (C₃₆H₃₄N₁₂O₁₄Ni)

**[Table 13]**

| | C | H | N |
|---|---|---|---|
| Calculated value (%) | 47.13 | 3.74 | 18.32 |
| Analytical value (%) | 47.20 | 3.68 | 18.22 |

MS(m/e): 916 (M⁺)
The thus-obtained compound exhibited an absorption maximum at a wavelength of 440.0 nm in a chloroform solution and the gram extinction coefficient of the compound was 0.61 × 10⁵ ml/g·cm.

### Example 13

### Synthesis of Compound of Concrete Example (59)

In a mixed solution of 80 g of acetic acid and 40 g of propionic acid, was dissolved 4.0 g of a compound represented by the following formula (8-f) and 5.82 g of 40% nitrosylsulfuric acid was added dropwise thereto at 0 to 5 degree centigrade. The resulting solution was stirred at the same temperature for 1 hour and diazotized. In 90 g of methanol, were dissolved 2.86 g of a compound represented by the following formula (9-g) and 1.10 g of urea and, the obtained diazo solution was introduced dropwise thereto at 0 to 5 degree centigrade. The resulting solution was stirred at the same temperature for 3 hours, and then the precipitated crystal was filtered out, washed with water and dried to obtain 6.78 g of a compound represented by the following formula (1-k),

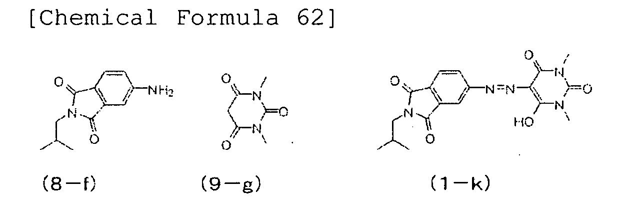

Then, 4.0 g of the compound represented by the formula (1-k) was dissolved in 120 g of methanol, and 1.55 g of nickel acetate tetrahydrate was added thereto at 60 to 65 degree centigrade. The resulting mixture was stirred at the same temperature for 3 hours. The precipitated crystal was filtered out, washed with water and dried to obtain 8.42 g of a compound of Concrete Example (59),

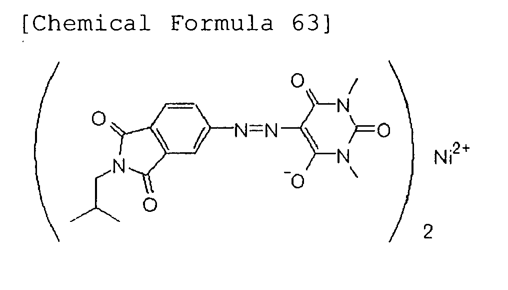

### Compound of Concrete Example (59)

From the following analytical results, the obtained compound was confirmed as a desired product.
Elemental Analytical Value (C₃₆H₃₆N₁₀O₁₀Ni)

**[Table 14]**

| | C | H | N |
|---|---|---|---|
| Calculated value (%) | 52.26 | 4.39 | 16.93 |
| Analytical value (%) | 52.25 | 4.40 | 16.99 |

MS(m/e): 826(M⁺)
The thus-obtained compound exhibited an absorption maximum at a wavelength of 396.5 nm in a chloroform solution and the gram extinction coefficient of the compound was 0.39 × 10⁵ ml/g·cm.

### Example 14

In 10 ml of 2, 2, 3, 3-tetrafluoro-1-propanol to prepare a compound solution, was dissolved 0.2 g of the compound of Concrete Example (3) .
On a disc polycarbonate resin substrate of 120 mmΦ in outer diameter and 0.6 mm in thickness having a continuous guide groove (track pitch: 0.40 µm), this compound solution was spin-coated at a rotating rate of 2,000 rpm, and the coated substrate was dried at 90 degree centigrade for 1 hour to form a recording layer. On this recording layer, silver is sputtered with a sputtering apparatus (CDI-900) manufactured by Balzars to form a reflective layer of 200 nm in thickness. Argon gas was used as sputter gas. Sputtering was carried out under conditions including a sputtering power of 3.75 kW and a sputtering gas pressure of 0.8 Pa (0.6 × 10⁻² Torr).

Further, after an ultraviolet curing resin SD-1700 (a product of DIC Corporation) was spin-coated on the reflective layer and, was irradiated with ultraviolet light to form a protective layer of 5 µm in thickness. Furthermore, after an ultraviolet curing resin DeSolite KZ-8681 (a product of JSR Corporation) was spin-coated on the protective layer, a polycarbonate resin substrate, which was the same as the above-mentioned substrate, was put on the protective layer, and these substrates were adhered together by irradiation with ultraviolet light to prepare an optical recording medium.

With the optical recording medium having a recording layer formed as described above, evaluation test was carried out as follows.
On an optical tester (Pulstec Industrial Co., Ltd., model No.: ODU-1000) equipped with a blue laser head having a wavelength of 405 nm and a numerical aperture of 0.65, recording was carried out at a recording frequency of 64.8 MHz, a linear velocity of 6.61 m/s and a shortest mark length of 0.204 µm. After recording, reproduction was carried out at a reproducing laser power of 0.4 mW and a linear velocity of 6.61 m/s on the same apparatus. Evaluation was carried out and as a result, a good signal waveform was obtained such that the recording sensitivity was 9.0 mW and the degree of modulation was 0.55. Reproduction was further repeatedly carried out 100,000 times and as a result, the degree of modulation was 0.54 and the stability against reproduction light was excellent.

### Examples 15 to 33

Optical recording media were prepared in the same manner as in Example 14, except that compounds of Concrete Examples illustrated in Table 2-1 among compounds of Concrete Examples illustrated in Tables 1-1 to 1-10 were properly used.
These optical recording media were evaluated in the same manner as in Example 14 and as a result, good recording characteristics and excellent stability against reproduction light could be confirmed for all media.

### Comparative Example 1

An optical recording medium was prepared in the same manner as in Example 14, except that a compound of the formula (a) was used, and the same evaluation was carried out with respect to the optical recording medium. A good signal waveform was obtained such that the recording sensitivity was 7.2 mW and the degree of modulation was 0.71. However, reproduction was repeatedly carried out 100,000 times and as a result, the degree of modulation was 0.32, the signal waveform became distorted, and the stability against reproduction light became remarkably worse,

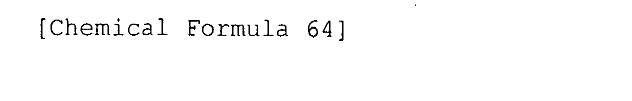

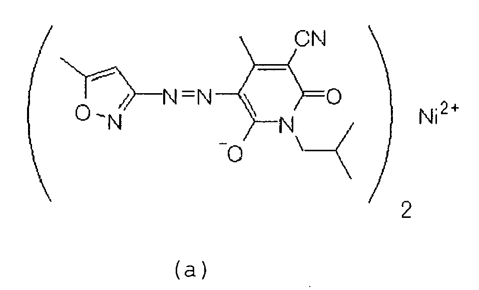

### Comparative Example 2

An optical recording medium was prepared in the same manner as in Example 14, except that a compound of the formula (b) was used, and the same evaluation was carried out with respect to the optical recording medium. The sensitivity was remarkably bad such that the recording sensitivity was not less than 16 mW, and a signal waveform was distorted such that the degree of modulation was 0.44. Furthermore, reproduction was repeatedly carried out 100,000 times and as a result, the degree of modulation was not more than 0.30 and the stability against reproduction light became remarkably worse,

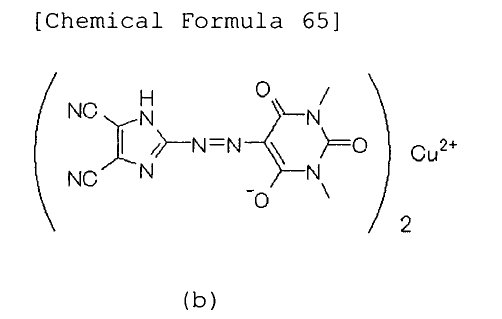

As for Examples 14 to 33, and Comparative Examples 1 and 2, the recording sensitivity, degree of modulation and degree of modulation after reproduction of 100,000 times were illustrated in Table 2-1. It could be confirmed that the optical recording media illustrated in the Examples exhibited good recording characteristics and excellent stability against reproduction light.

[Table 15]

**Table 2-1**

| | Compound No. | Recording Sensitivity (mW) | Degree of modulation | |
|---|---|---|---|---|
| | | | Initial | After reproduction of 100,000 times |
| Example 14 | 3 | 9.0 | 0.55 | 0.54 |
| Example 15 | 9 | 9.4 | 0.61 | 0.60 |
| Example 16 | 10 | 9.2 | 0.61 | 0.61 |
| Example 17 | 11 | 9.2 | 0.59 | 0.57 |
| Example 18 | 12 | 9.4 | 0.60 | 0.59 |
| Example 19 | 14 | 8.2 | 0.61 | 0.60 |
| Example 20 | 16 | 6.8 | 0.69 | 0.65 |
| Example 21 | 31 | 11.0 | 0.47 | 0.46 |
| Example 22 | 37 | 9.4 | 0.65 | 0.64 |
| Example 23 | 38 | 9.4 | 0.70 | 0.68 |
| Example 24 | 41 | 8.4 | 0.59 | 0.59 |
| Example 25 | 43 | 7.8 | 0.67 | 0.66 |
| Example 26 | 51 | 8.8 | 0.62 | 0.60 |
| Example 27 | 53 | 6.8 | 0.66 | 0.63 |
| Example 28 | 60 | 8.8 | 0.61 | 0.60 |
| Example 29 | 61 | 10.4 | 0.61 | 0.60 |
| Example 30 | 69 | 8.8 | 0.66 | 0.64 |
| Example 31 | 70 | 9.4 | 0.57 | 0.56 |
| Example 32 | 72 | 8.0 | 0.62 | 0.61 |
| Example 33 | 78 | 11.6 | 0.53 | 0.52 |
| Comparative Example 1 | Structural Formula (a) | 7.2 | 0.71 | 0.32 |
| Comparative Example 2 | Structural b) Formula (b) | 16.0 or more | 0.44 | 0.30 or less |

### Example 34

### Synthesis of Compound of Concrete Example (3-1)

In a mixed solution of 18 g of acetic acid and 9 g of propionic acid, was dissolved 1.82 g of a compound represented by the following formula (8-c) and 2.60 g of 40% nitrosylsulfuric acid was added dropwise thereto at 0 to 5 degree centigrade. The resulting solution was stirred at the same temperature for 1 hour and diazotized. In 40 g of 3% aqueous caustic soda solution, were dissolved 1.39 g of a compound represented by the following formula (9-h) and 0.47 g of urea and, the obtained diazo solution was introduced dropwise thereto at 0 to 5 degree centigrade. The resulting solution was stirred at the same temperature for 2 hours, and then the precipitated crystal was filtered out, washed with water and dried to obtain 2.9 g of a compound represented by the following formula (1-1),

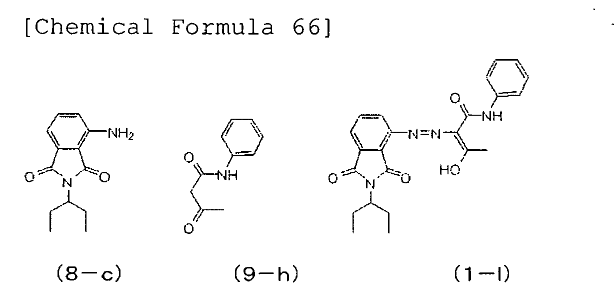

Then, 2.86 g of the compound represented by the formula (1-1) was dissolved in 86 g of methanol, and 1.02 g of nickel acetate tetrahydrate was added thereto at 50 to 55 degree centigrade. The resulting mixture was stirred at the same temperature for 2 hours. The precipitated crystal was filtered out, washed with water and then washed with methanol and dried to obtain 1.90 g of a compound of Concrete Example (3-1),

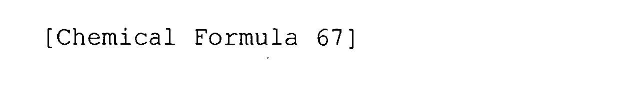

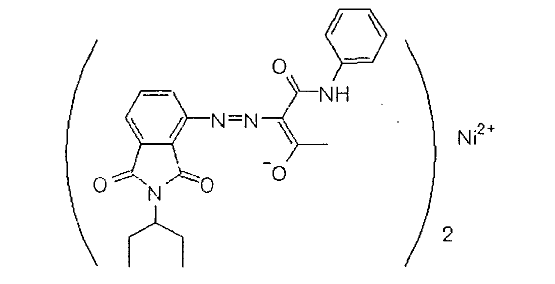

### Compound of Concrete Example (3-1)

From the following analytical results, the obtained compound was confirmed as a desired product.
Elemental Analytical Value (C₄₆H₄₆N₈O₈Ni)

**[Table 16]**

| | C | H | N |
|---|---|---|---|
| Calculated value (%) | 61.55 | 5.17 | 12.48 |
| Analytical value (%) | 61.52 | 5.22 | 12.40 |

MS(m/e): 896(M⁺)
The thus-obtained compound exhibited an absorption maximum at a wavelength of 405.0 nm in a chloroform solution and the gram extinction coefficient of the compound was 0.53 × 10⁵ ml/g·cm.

### Example 35

### Synthesis of Compound of Concrete Example (2-15)

In a mixed solution of 200 g of acetic acid and 50 g of propionic acid, was dissolved 29.0 g of a compound represented by the following formula (8-g) and 38.9 g of 40% nitrosylsulfuric acid was added dropwise thereto at 0 to 5 degree centigrade. The resulting solution was stirred at the same temperature for 2 hours and diazotized.
On the other hand, 18.5 g of methyl isobutyrylacetate was dissolved in a mixed solvent of 90 g of ethanol and 45 g of water, and 8.97 g of hydroxylamine hydrochloride was added dropwise thereto. In an ice water bath, was added dropwise 33.6 g of morpholine with cooling, and the resulting solution was stirred for 4 hours to obtain an aqueous ethanol solution of a compound represented by the following formula (9-i). The diazo solution was introduced dropwise thereto at 0 to 5 degree centigrade. The resulting solution was stirred at the same temperature for 2 hours, and then poured into 2,400 g of water. The precipitated crystal was filtered out, washed with water and dried to obtain 43.4 g of a compound represented by the general formula (1-m),

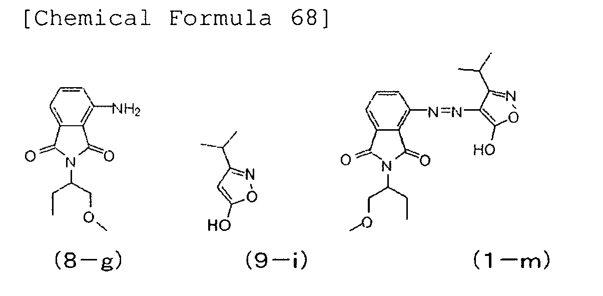

Then, 43.0 g of the compound represented by the formula (1-m) was dissolved in 400 g of 2-propanol, and 11.1 g of copper acetate was added thereto at 80 degree centigrade. The resulting mixture was stirred at the same temperature for 5 hours. The precipitated crystal was filtered out, washed with 2-propanol and dried to obtain 32.2 g of a compound of Concrete Example (2-15),

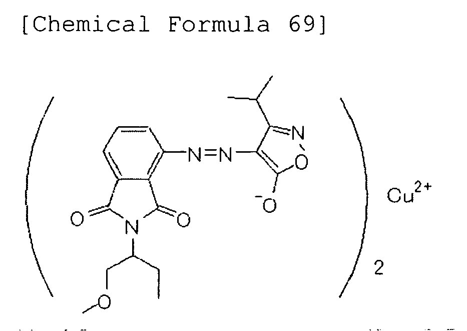

### Compound of Concrete Example (2-15)

From the following analytical results, the obtained compound was confirmed as a desired product.
Elemental Analytical Value (C₃₈H₄₂N₈O₁₀Cu)

**[Table 17]**

| | C | H | N |
|---|---|---|---|
| Calculated value (%) | 54.70 | 5.07 | 13.43 |
| Analytical value (%) | 54.55 | 5.11 | 13.40 |

MS(m/e): 833 (M⁺)
The thus-obtained compound exhibited an absorption maximum at a wavelength of 422.5 nm in a chloroform solution and the gram extinction coefficient of the compound was 0.54 × 10⁵ ml/g·cm.

### Example 36

### Synthesis of Compound of Concrete Example (2-16)

In 40 g of 2-propanol, was dissolved 5.29 g of a compound represented by the formula (1-m) and 3.47 g of nickel acetate tetrahydrate was added thereto at 80 to 85 degree centigrade. The resulting solution was stirred at the same temperature for 2 hours. The precipitated crystal was filtered out, washed with 2-propanol and dried to obtain 5.18 g of a compound of Concrete Example (2-16),

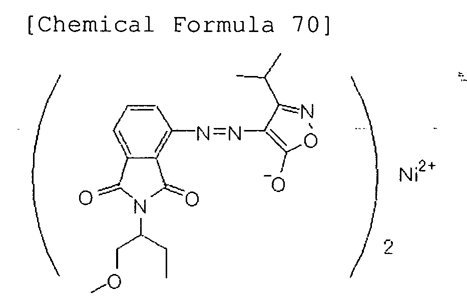

### Compound of Concrete Example (2-16)

From the following analytical results, the obtained compound was confirmed as a desired product.
Elemental Analytical Value (C₃₈H₄₂N₈O₁₀Ni)

**[Table 18]**

| | C | H | N |
|---|---|---|---|
| Calculated value (%) | 55.02 | 5.10 | 13.51 |
| Analytical value (%) | 55.00 | 5.14 | 13.48 |

MS(m/e): 828(M⁺)
The thus-obtained compound exhibited an absorption maximum at a wavelength of 438.0 nm in a chloroform solution and the gram extinction coefficient of the compound was 0.57 × 10⁵ ml/g·cm.

### Example 37

### Synthesis of Compound of Concrete Example (2-23)

In a mixed solution of 26 g of acetic acid and 13 g of propionic acid, was dissolved 2.63 g of a compound represented by the following formula (8-c) and 3.79 g of 40% nitrosylsulfuric acid was added dropwise thereto at 0 to 5 degree centigrade. The resulting solution was stirred at the same temperature for 2 hours and diazotized.
In 60 g of 3% aqueous caustic soda solution, were dissolved 2.00 g of a compound represented by the following formula (9-j) and 0.68 g of urea and, the obtained diazo solution was introduced dropwise thereto at 0 to 5 degree centigrade. The resulting solution was stirred at the same temperature for 2 hours, and then the precipitated crystal was filtered out, washed with water and dried to obtain 3.17 g of a compound represented by the following formula (1-n),

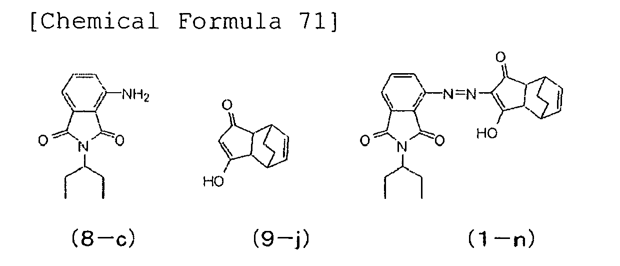

Then, 1.5 g of the compound represented by the formula (1-n) was dissolved in 45 g of methanol, and 0.39 g of copper acetate was added thereto at 60 degree centigrade. The resulting mixture was stirred at the same temperature for 2 hours. The reaction solution was cooled to room temperature, and then poured into 45 g of water. The precipitated solid was filtered out, washed with water and dried to obtain 0.91 g of a compound of Concrete Example (2-23),

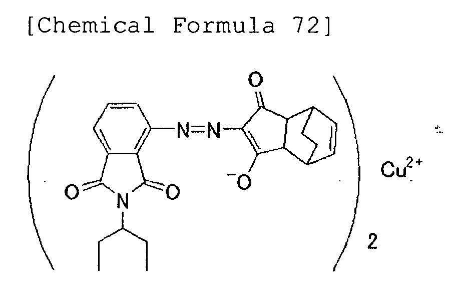

### Compound of Concrete Example (2-23)

From the following analytical results, the obtained compound was confirmed as a desired product.
Elemental Analytical Value (C₄₈H₄₈N₆O₈Cu)

**[Table 19]**

| | C | H | N |
|---|---|---|---|
| Calculated value (%) | 64.02 | 5.37 | |
| Analytical value (%) | 64.23 | 5.40 | 9.28 |

MS(m/e): 899(M⁺)
The thus-obtained compound exhibited an absorption maximum at a wavelength of 409.0 nm in a chloroform solution and the gram extinction coefficient of the compound was 0.33 × 10⁵ ml/g·cm.

### Example 38

### Synthesis of Compound of Concrete Example (2-24)

In 45 g of methanol, was dissolved 1.5 g of the compound represented by the formula (1-n) and 0.53 g of nickel acetate tetrahydrate was added dropwise thereto at 60 degree centigrade. The resulting solution was stirred at the same temperature for 2 hours. The reaction solution was cooled to room temperature, and then poured into 45 g of water. The precipitated solid was filtered out, washed with water and dried to obtain 0.79 g of a compound of Concrete Example (2-24),

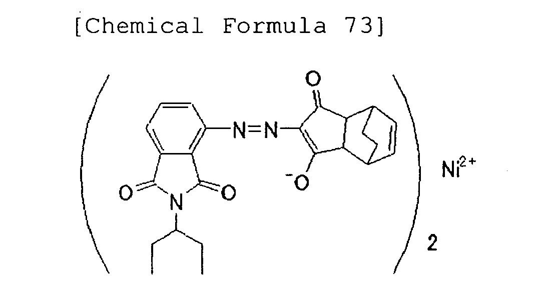

### Compound of Concrete Example (2-24)

From the following analytical results, the obtained compound was confirmed as a desired product.
Elemental Analytical Value (C₄₈H₄₈N₆O₈Ni)

**[Table 20]**

| | C | H | N |
|---|---|---|---|
| Calculated value (%) | 64.37 | 5.40 | 9.38 |
| Analytical value (%) | 64.22 | 5.38 | 9.35 |

MS(m/e): 894(M⁺)
The thus-obtained compound exhibited an absorption maximum at a wavelength of 441.0 nm in a chloroform solution and the gram extinction coefficient of the compound was 0.48 × 10⁵ ml/g·cm.

### Example 39

### Synthesis of Compound of Concrete Example (2-32)

In a mixed solution of 26 g of acetic acid and 13 g of propionic acid, was dissolved 2.63 g of a compound represented by the following formula (8-c) and 3.79 g of 40% nitrosylsulfuric acid was added dropwise thereto at 0 to 5 degree centigrade. The resulting solution was stirred at the same temperature for 2 hours and diazotized.
In 60 g of 3% aqueous caustic soda solution, were dissolved 2.00 g of a compound represented by the following formula (9-k) and 0. 68 g of urea and, the obtained diazo solution was introduced dropwise thereto at 0 to 5 degree centigrade. The resulting solution was stirred at the same temperature for 2 hours, and then the precipitated crystal was filtered out, washed with water and dried to obtain 3.17 g of a compound represented by the following formula (1-o),

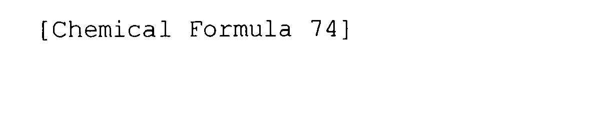

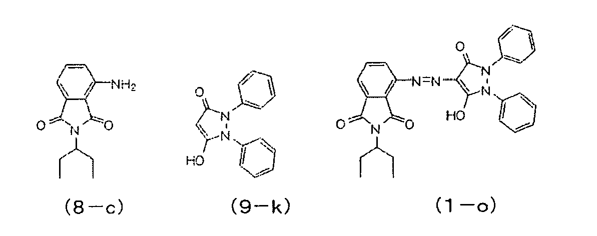

Then, 1.5 g of the compound represented by the formula (1-o) was dissolved in 45 g of methanol, and 0.39 g of copper acetate was added thereto at 60 degree centigrade. The resulting mixture was stirred at the same temperature for 2 hours. The reaction solution was cooled to room temperature, and then poured into 45 g of water. The precipitated solid was filtered out, washed with water and dried to obtain 0.91 g of a compound of Concrete Example (2-32),

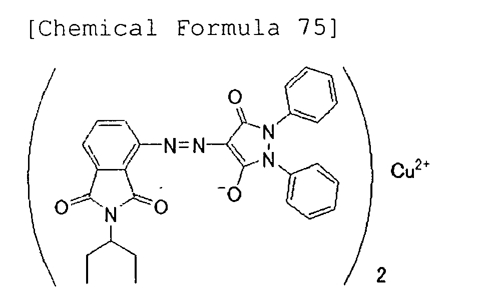

### Compound of Concrete Example (2-32)

From the following analytical results, the obtained compound was confirmed as a desired product.
Elemental Analytical Value (C₅₆H₄₈N₁₀O₈Cu)

**[Table 21]**

| | C | H | N |
|---|---|---|---|
| Calculated value (%) | 63.90 | 4.60 | 13.31 |
| Analytical value (%) | 63.85 | 4.61 | 13.28 |

MS(m/e): 1051(M⁺)
The thus-obtained compound exhibited an absorption maximum at a wavelength of 418.0 nm in a chloroform solution and the gram extinction coefficient of the compound was 0.51 × 10⁵ ml/g·cm.

### Example 40

### Synthesis of Compound of Concrete Example (3-8)

In a mixed solution of 20 g of acetic acid and 10 g of propionic acid, was dissolved 4.65 g of a compound represented by the following formula (8-c) and 6.99 g of 40% nitrosylsulfuric acid was added dropwise thereto at 0 to 5 degree centigrade. The resulting solution was stirred at the same temperature for 2 hours and diazotized.
In a mixed solution of 60 g of methanol and 20 g of water, were dissolved 2.91 g of a compound represented by the following formula (9-1), 0.40 g of urea and 4.00 g of sodium acetate and, the obtained diazo solution was introduced dropwise at 0 to 5 degree centigrade. Upon dropwise thereto addition, the pH was maintained at 3 to 5 with the aqueous caustic soda solution. The solution was stirred at the same temperature for 2 hours, and then the precipitated crystal was filtered out, washed with water and dried to obtain 7.37 g of a compound represented by the following formula (1-p),

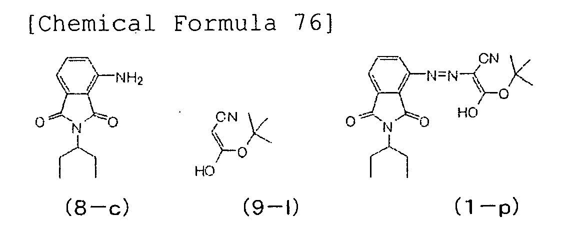

Then, 3.84 g of the compound represented by the formula (1-p) was dissolved in 60 g of methanol, and 1.46 g of triethylamine was added thereto. The resulting mixture was heated to 45 degree centigrade and 1.09 g of copper acetate was added thereto. The solution was stirred at the same temperature for 6 hours and cooled to room temperature. Then, the precipitated solid was filtered out, washed with methanol and dried to obtain 2.84 g of a compound of Concrete Example (3-8),

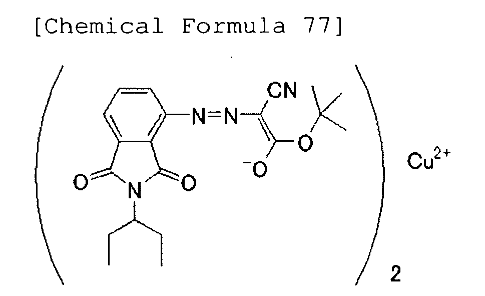

### Compound of Concrete Example (3-8)

From the following analytical results, the obtained compound was confirmed as a desired product.
Elemental Analytical Value (C₄₀H₄₆N₈O₈Cu)

**[Table 22]**

| | C | H | N |
|---|---|---|---|
| Calculated value (%) | 57.86 | 5.58 | 13.49 |
| Analytical value (%) | 57.83 | 5.61 | 13.50 |

MS(m/e): 829(M⁺)
The thus-obtained compound exhibited an absorption maximum at a wavelength of 380.5 nm in a chloroform solution and the gram extinction coefficient of the compound was 0.52 × 10⁵ ml/g·cm.

### Example 41

### Synthesis of Compound of Concrete Example (3-10)

In a mixed solution of 20 g of acetic acid and 15 g of propionic acid, was dissolved 3.94 g of a compound represented by the following formula (8-d) and 4.56 g of 40% nitrosylsulfuric acid was added dropwise thereto at 0 to 5 degree centigrade. The resulting solution was stirred at the same temperature for 2 hours and diazotized.
In 18 g of methanol, were dissolved 1.50 g of a compound represented by the following formula (9-m) and 0.74 g of urea and, the obtained diazo solution was introduced dropwise thereto at 0 to 5 degree centigrade. The resulting solution was stirred at the same temperature for 2 hours. The reaction solution was poured into 400 g of water, and the precipitated solid was filtered out, washed with water and dried to obtain 5.00 g of a compound represented by the following formula (1-q),

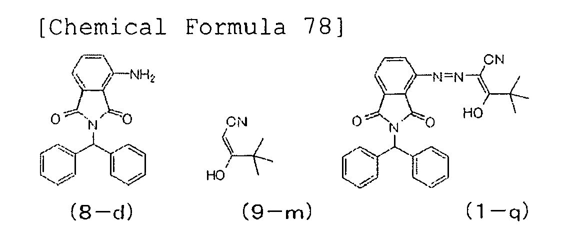

Then, 4.00 g of the compound represented by the formula (1-q) was dissolved in 45 g of methanol, and 0.94 g of triethylamine was added thereto. The resulting mixture was heated to 50 degree centigrade and 0.86 g of copper acetate was added thereto. The solution was stirred at the same temperature for 2 hours. The solution was cooled to room temperature, and then the precipitated solid was filtered out, washed with methanol and dried to obtain 4.35 g of a compound of Concrete Example (3-10),

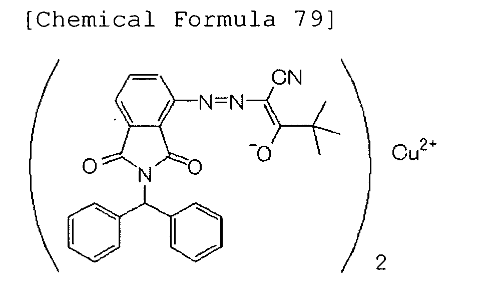

### Compound of Concrete Example (3-10)

From the following analytical results, the obtained compound was confirmed as a desired product.
Elemental Analytical Value (C₅₆H₄₆N₈O₆Cu)

**[Table 23]**

| | C | H | N |
|---|---|---|---|
| Calculated value (%) | 67.90 | 4.68 | 11.31 |
| Analytical value (%) | 67.92 | 4.74 | 11.30 |

MS(m/e): 989(M⁺)
The thus-obtained compound exhibited an absorption maximum at a wavelength of 423.5 nm in a chloroform solution and the gram extinction coefficient of the compound was 0.29 × 10⁵ ml/g·cm.

### Example 42

### Synthesis of Compound of Concrete Example (2-40)

In a mixed solution of 211.1 g of acetic acid and 100.3 g of propionic acid, was dissolved 20.2 g of a compound represented by the following formula (8-g) and 25.8 g of 40% nitrosylsulfuric acid was added dropwise thereto at 0 to 3 degree centigrade. The resulting solution was stirred at the same temperature for 1 hour and diazotized. In 490 g of 1% aqueous caustic soda solution, were dissolved 11.4 g of a compound represented by the following formula (9-n) and 4.9 g of urea and, the obtained diazo solution was introduced dropwise thereto at 0 to 5 degree centigrade. The resulting solution was stirred at the same temperature for 1 hour, and then the precipitated crystal was filtered out, washed with water and dried to obtain 28.2 g of a compound represented by the following formula (1-r),

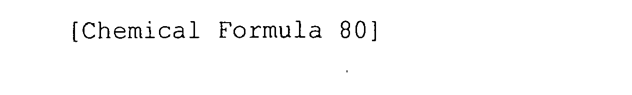

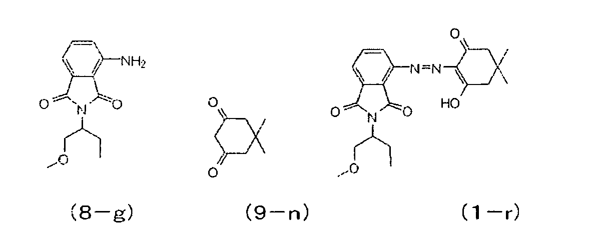

Then, 12.00 g of the compound represented by the formula (1-r) was dissolved in a mixed solution of 118.1 g of methanol and 3.0 g of triethylamine, and 3.30 g of copper acetate monohydrate was added thereto at 50 to 55 degree centigrade. The resulting mixture was stirred at the same temperature for 3 hours, and the precipitated crystal was filtered out and washed with water. The obtained crystal was dissolved in 39.4 g of methanol, and 50 g of water was added thereto to precipitate the crystal. The precipitated crystal was filtered out, washed with water and then dried to obtain 2.03 g of a compound of Concrete Example (2-40),

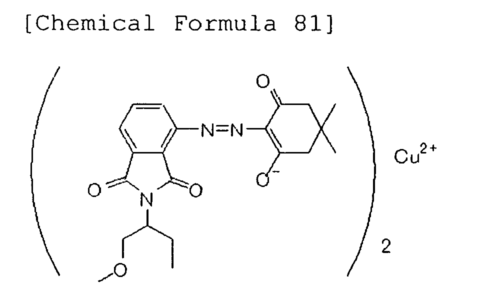

### Compound of Concrete Example (2-40)

From the following analytical results, the obtained compound was confirmed as a desired product.
Elemental Analytical Value (C₄₀H₄₄N₆O₁₀Cu)

**[Table 24]**

| | C | H | N |
|---|---|---|---|
| Calculated value (%) | 58.63 | 5.62 | 9.77 |
| Analytical value (%) | 58.60 | 5.61 | 9.79 |

MS(m/e): 859(M⁺)
The thus-obtained compound exhibited an absorption maximum at a wavelength of 421.0 nm in an acetone solution and the gram extinction coefficient of the compound was 2.85 × 10⁴ ml/g·cm.

### Example 43

### Synthesis of Compound of Concrete Example (2-45)

In a mixed solution of 376.2 g of acetic acid and 178.7 g of propionic acid, was dissolved 35.6 g of a compound represented by the following formula (8-c) and 48.7 g of 40% nitrosylsulfuric acid was added dropwise thereto at 0 to 3 degree centigrade. The resulting solution was stirred at the same temperature for 1 hour and diazotized. In 920 g of 1% aqueous caustic soda solution, were dissolved 46.0 g of a compound represented by the following formula (9-o) and 9.2 g of urea and, the obtained diazo solution was introduced dropwise at 0 to 5 degree centigrade, and then 157.4 g of methanol was added thereto. The solution was stirred at the same temperature for 2 hours, and then the precipitated crystal was filtered out, washed with water and dried to obtain 54.2 g of a compound represented by the following formula (1-s),

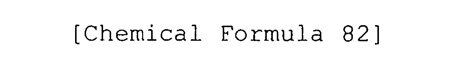

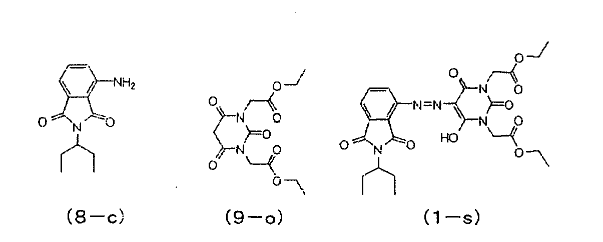

Then, 27.0 g of the compound represented by the formula (1-s) was dissolved in a mixed solution of 212.5 g of methanol and 5.2 g of triethylamine, and 4.69 g of copper acetate anhydride was added thereto at 40 to 45 degree centigrade. The resulting mixture was stirred at the same temperature for 2 hours, and the precipitated crystal was filtered out, washed with methanol and dried to obtain 23.8 g of a compound of Concrete Example (2-45),

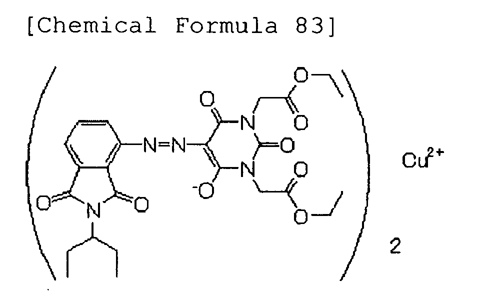

### Compound of Concrete Example (2-45)

From the following analytical results, the obtained compound was confirmed as a desired product.
Elemental Analytical Value (C₅₀H₅₆N₁₀O₁₈Cu)

**[Table 25]**

| | C | H | N |
|---|---|---|---|
| Calculated value (%) | 52.28 | 4.91 | 12.19 |
| Analytical value (%) | 52.25 | 4.90 | 12.17 |

MS(m/e): 1147(M⁺)
The thus-obtained compound exhibited an absorption maximum at a wavelength of 402.5 nm in an acetone solution and the gram extinction coefficient of the compound was 4.29 × 10⁴ ml/g·cm.

### Example 44

### Synthesis of Compound of Concrete Example (2-49)

In a mixed solution of 63 g of acetic acid and 29.8 g of propionic acid, was dissolved 6.0 g of a compound represented by the following formula (8-c) and 8.21 g of 40% nitrosylsulfuric acid was added dropwise thereto at 0 to 5 degree centigrade. The resulting solution was stirred at the same temperature for 1 hour and diazotized. In 412 g of water, were dissolved 4.12 g of a compound represented by the following formula (9-p), 1.55 g of urea and 1.03 g of sodium hydroxide and, the obtained diazo solution was added dropwise thereto at 0 to 5 degree centigrade. The resulting solution was stirred at the same temperature for 1 hour, and subsequently stirred at room temperature for 1 hour. The precipitated crystal was leached, washed with water and dried to obtain 8.74 g of a compound represented by the following formula (1-t),

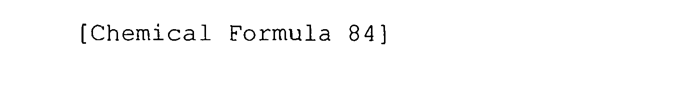

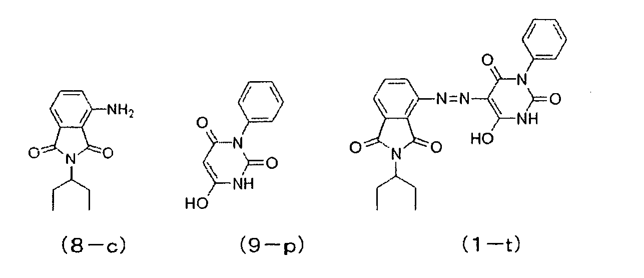

Subsequently, 5.0 g of the compound represented by the formula (1-t), 2.66 g of p-toluenesulfonic acid-2,2,2-trifluoroethyl and 1.54 g of potassium carbonate were dissolved in 52.8 g of 1,3-dimethyl-2-imidazolidinone, and the resulting solution was stirred at 120 degree centigrade for 4 hours. The reaction solution was poured into 250 g of water, and about 20 drops of concentrated hydrochloric acid were added dropwise such that the pH was 1 to 2. The precipitated crystal was leached and washed with water. The obtained filter medium was suspended in 47.4 g of methanol and stirred at a reflux temperature for 30 minutes, and then cooled to room temperature. The suspension was leached and washed with methanol to obtain 2.19 g of a compound represented by the following formula (1-u),

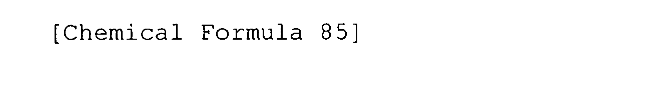

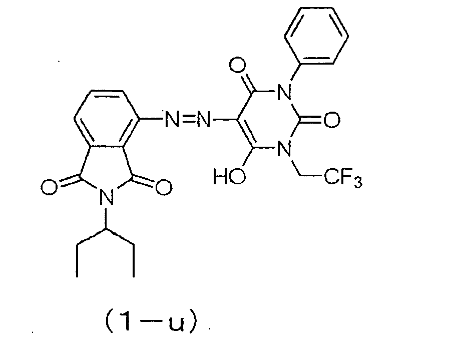

Then, 2.1 g of the compound represented by the formula (1-u), 0.40 g of triethylamine and 0.41 g of copper acetate monohydrate were dissolved in 16.5 g of methanol, and the resulting solution was stirred at 50 to 55 degree centigrade for 4 hours. The precipitated crystal was leached, washed with methanol and dried to obtain 1.74 g of a compound of Concrete Example (2-49),

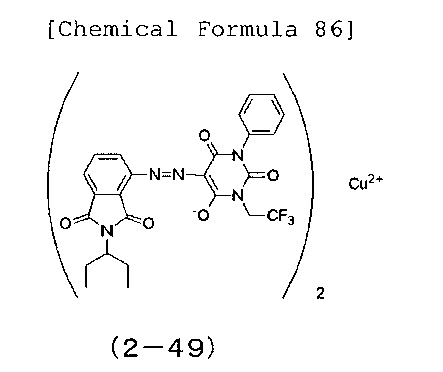

From the following analytical results, the obtained compound was confirmed as a desired product.
Elemental Analytical Value (C₅₀H₄₂N₁₀O₁₀F₆Cu)

**[Table 26]**

| | C | H | N |
|---|---|---|---|
| Calculated value (%) | 53.60 | 3.78 | 12.50 |
| Analytical value (%) | 53.63 | 3.76 | 12.49 |

MS(ESI) (m/z): 1131 (M⁺)

### Example 45

### Synthesis of Compound of Concrete Example (2-47)

In a mixed solution of 430.5 g of acetic acid and 203.5 g of propionic acid, was dissolved 41.5 g of a compound represented by the following formula (8-c) and 56.8 g of 40% nitrosylsulfuric acid was added dropwise thereto at 0 to 5 degree centigrade. The resulting solution was stirred at the same temperature for 1 hour and diazotized. In 1,130 g of water, were dissolved 55.0 g of a compound represented by the following formula (9-q), 11.31 g of urea and 11. 3 g of sodium hydroxide and, the obtained diazo solution was added dropwise thereto at 0 to 5 degree centigrade. The resulting solution was stirred at the same temperature for 1 hour, and subsequently stirred at room temperature for 30 minutes. The precipitated crystal was washed with water, washed with methanol and dried to obtain 81.9 g of a compound represented by the following formula (1-v),

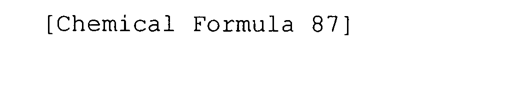

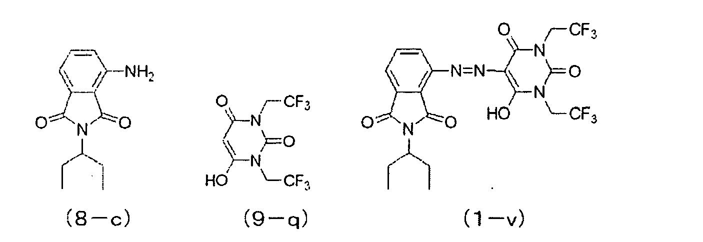

Then, 80.0 g of the compound represented by the formula (1-v) 15.12 g of triethylamine and 15.5 g of copper acetate monohydrate were dissolved in 948.0 g of methanol, and the resulting solution was stirred at 40 to 45 degree centigrade for 3 hours. The precipitated crystal was leached, washed with methanol and dried to obtain 82.6 g of a compound of Concrete Example (2-47),

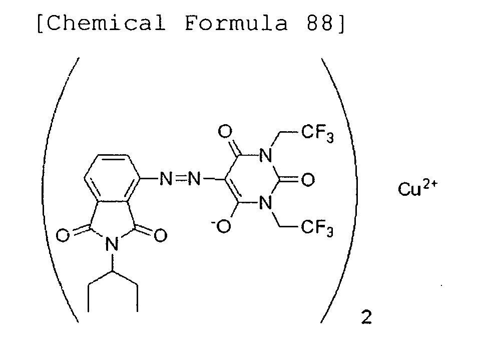

### Compound of Concrete Example (2-47)

From the following analytical results, the obtained compound was confirmed as a desired product.
Elemental Analytical Value (C₄₂H₃₆N₁₀O₁₀F₁₂Cu)

**[Table 27]**

| | C | H | N |
|---|---|---|---|
| Calculated value (%) | 44.55 | 3.20 | 12.37 |
| Analytical value (%) | 44.57 | 3.18 | 12.34 |

MS (ESI) (m/z): 1131 (M⁺)

### Example 46

### Synthesis of Compound of Concrete Example (2-43)

In a mixed solution of 60 g of acetic acid and 30 g of propionic acid, was dissolved 6.0 g of a compound represented by the following formula (8-g) and 8.09 g of 40% nitrosylsulfuric acid was added dropwise thereto at 0 to 5 degree centigrade. The resulting solution was stirred at the same temperature for 1 hour and diazotized. In 100 g of water, were dissolved 4.25 g of a compound represented by the following formula (9-r), 1.46 g of urea and 0.98 g of caustic soda and, the obtained diazo solution was introduced dropwise thereto at 0 to 5 degree centigrade. The resulting solution was stirred at the same temperature for 2 hours, and then the precipitated crystal was filtered out, washed with water and dried to obtain 4.43 g of a compound represented by the following formula (1-w),

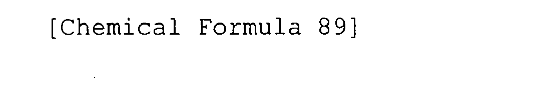

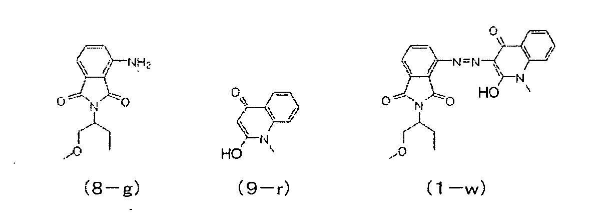

Then, 4.2 g of the compound represented by the general formula (1-w) was dissolved in a mixed solution of 65 g of methoxy ethanol and 1.0 g of triethylamine, and 0.98 g of copper acetate monohydrate was added thereto at 50 to 55 degree centigrade. The resulting mixture was stirred at the same temperature for 3 hours. The precipitated crystal was filtered out, washed with methanol and dried to obtain 2.94 g of a compound of Concrete Example (2-43),

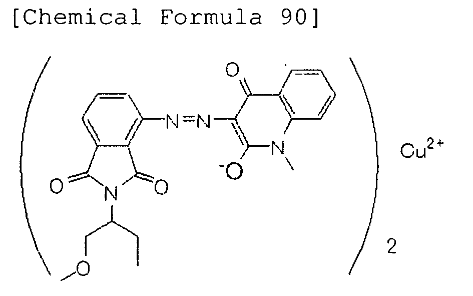

### Compound of Concrete Example (2-43)

From the following analytical results, the obtained compound was confirmed as a desired product.
Elemental Analytical Value (C₄₆H₄₂N₈O₁₀Cu)

**[Table 28]**

| | C | H | N |
|---|---|---|---|
| Calculated value (%) | 59.38 | 4.55 | 12.04 |
| Analytical value (%) | 59.36 | 4.56 | 12.06 |

MS(m/e): 929(M⁺)
The thus-obtained compound exhibited an absorption maximum at a wavelength of 440.0 nm in an acetone solution and the gram extinction coefficient of the compound was 2.31 × 10⁴ ml/g·cm.

### Example 47

### Synthesis of Compound of Concrete Example (2-50)

In a mixed solution of 340 g of acetic acid and 170 g of propionic acid, was dissolved 34.0 g of a compound represented by the following formula (8-d) and 34.5 g of 40% nitrosylsulfuric acid was added dropwise thereto at 0 to 5 degree centigrade. The resulting solution was stirred at the same temperature for 1 hour and diazotized. In 510 g of 50% aqueous methanol solution, were dissolved 30.3 g of a compound represented by the following formula (9-r), 6.21 g of urea and 4.14 g of caustic soda and, the obtained diazo solution was introduced dropwise thereto at 0 to 5 degree centigrade. The resulting solution was stirred at the same temperature for 2 hours, and then the precipitated crystal was filtered out, washed with water and dried to obtain 63.2 g of a compound represented by the following formula (1-x),

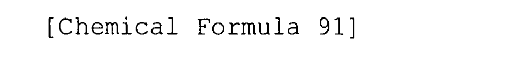

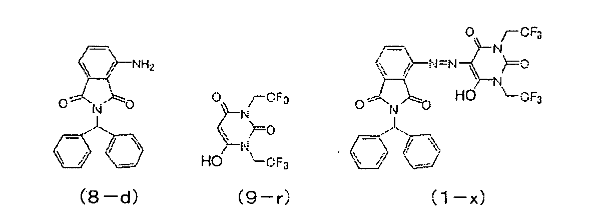

Then, 63.0 g of the compound represented by the formula (1-x) was dissolved in a mixed solution of 945 g of methanol and 10.1 g of triethylamine, and 9.96 g of copper acetate monohydrate was added thereto at 50 to 55 degree centigrade. The resulting mixture was stirred at the same temperature for 3 hours. The precipitated crystal was filtered out, washed with methanol and dried to obtain 47.9 g of a compound of Concrete Example (2-50),

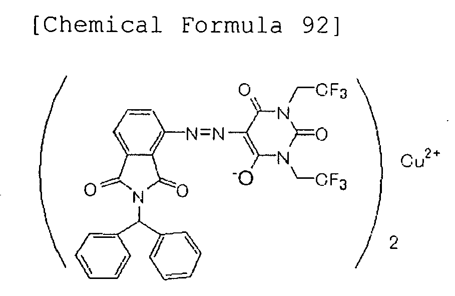

### Compound of Concrete Example (2-50)

From the following analytical results, the obtained compound was confirmed as a desired product.
Elemental Analytical Value (C₅₈H₃₆N₁₀O₁₀F₁₂Cu)

**[Table 29]**

| | C | H | N |
|---|---|---|---|
| Calculated value (%) | 52.60 | 2.74 | 10.58 |
| Analytical value (%) | 52.57 | 2.73 | 10.60 |

MS (m/e): 1323 (M⁺)
The thus-obtained compound exhibited an absorption maximum at a wavelength of 414.0 nm in an acetone solution and the gram extinction coefficient of the compound was 3.39 × 10⁴ ml/g·cm.

### Example 48

### Synthesis of Compound of Concrete Example (2-13)

In a mixed solution of 220 g of acetic acid and 50 g of propionic acid, was dissolved 27.1 g of a compound represented by the following formula (8-c) and 39.0 g of 40% nitrosylsulfuric acid was added dropwise thereto at 0 to 5 degree centigrade. The resulting solution was stirred at the same temperature for 1 hour and diazotized. In 270 g of methanol, was dissolved 25.0 g of a compound represented by the following formula (9-s) and, the obtained diazo solution was introduced dropwise thereto at 0 to 5 degree centigrade. The resulting solution was stirred at the same temperature for 2 hours, and then the precipitated solid was filtered out, washed with water, washed with methanol and dried to obtain 35.0 g of a compound represented by the following formula (1-y),

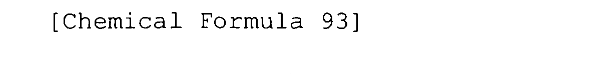

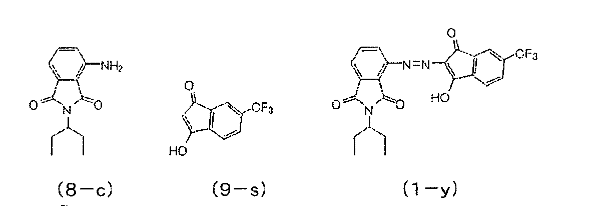

Then, 34.0 g of the compound represented by the formula (1-y) was dissolved in 540 g of methyl cellosolve, and 7.40 g of copper acetate monohydrate was added thereto at 110 degree centigrade. The resulting mixture was stirred at the same temperature for 3 hours. The precipitated crystal was filtered out, washed with methyl cellosolve, washed with methanol and dried to obtain 23.3 g of a compound of Concrete Example (2-13),

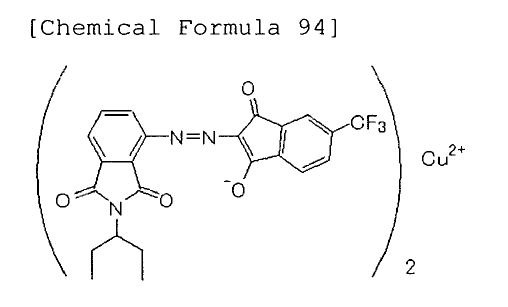

### Compound of Concrete Example (2-13)

From the following analytical results, the obtained compound was confirmed as a desired product.
Elemental Analytical Value (C₄₆H₃₄N₆O₈F₆Cu)

**[Table 30]**

| | C | H | N |
|---|---|---|---|
| Calculated value (%) | 56.59 | 3.51 | 8.61 |
| Analytical value (%) | 56.57 | 3.55 | 8.59 |

MS (m/e): 975 (M⁺)
The thus-obtained compound exhibited an absorption maximum at a wavelength of 435.5 nm in an acetone solution and the gram extinction coefficient of the compound was 4.48 × 10⁴ ml/g·cm.

### Examples 49 to 83

Optical recording media were prepared in the same manner as in Example 14, except that compounds of Concrete Examples illustrated in Table 2-2 to 2-5 among compounds of Concrete Examples illustrated in Tables 1-11 to 1-22 were properly used.

With respect to the prepared optical recording media, evaluation test was carried out as follows.
On an optical tester (Pulstec Industrial Co., Ltd., model No.: ODU-1000) equipped with a blue laser head having a wavelength of 405 nm and a numerical aperture of 0.65, recording was carried out at a recording frequency of 64.8 MHz, a linear velocity of 6.61 m/s and a shortest mark length of 0.204 µm. After recording, reproduction was carried out at a reproducing laser power of 0.4 mW and a linear velocity of 6.61 m/s on the same apparatus. Evaluation was carried out and as a result, good recording characteristics could be confirmed for all media. Here, as evaluation properties, the recording sensitivity, SN ratio at PRSNR (partial response signal to noise ratio) and the degree of modulation were measured. Incidentally, the definition and measurement method of PRSNR has been described in the book commercially available from DVD Format Logo Licensing Co., Ltd. (part of Annex H of DVD Specifications for High Density Read-Only Disc PART 1 Physical Specifications Version 0.9). PRSNR is preferably not less than 15, the degree of modulation is preferably not less than 0.4, and the recording sensitivity is preferably not more than 10 mW.
Reproduction was further repeatedly carried out 100,000 times, and then PRSNR and the degree of modulation were measured and as a result, excellent stability against reproduction light could be confirmed for all media.

### Comparative Example 3

With respect to the optical recording medium prepared in Comparative Example 1, evaluation test was carried out in the same manner as in Examples 49 to 83.
A good signal waveform was obtained such that the recording sensitivity was 7.2 mW, PRSNR was 20 and the degree of modulation was 0.71. However, reproduction was repeatedly carried out 100, 000 times and as a result, PRSNR was not more than 10, the degree of modulation was 0.32, the signal waveform became distorted, and the stability against reproduction light became remarkably worse.

### Comparative Example 4

With respect to the optical recording medium prepared in Comparative Example 2, evaluation test was carried out in the same manner as in Examples 49 to 83.
The sensitivity was remarkably worse such that the recording sensitivity was not less than 16 mW, and a signal waveform was distorted such that PRSNR was not more than 10 and the degree of modulation was 0.44. Furthermore, reproduction was repeatedly carried out 100,000 times and as a result, PRSNR was not more than 10, the degree of modulation was not more than 0.30, and the stability against reproduction light became remarkably worse.

### Example 84

An optical recording medium was prepared in the same manner as in Example 14, except that 0.2 g of a mixture of a compound of Concrete Example (38) and a compound of Concrete Example (2-10) (weight ratio; 1:1) was dissolved in 10 ml of 2,2,3,3-tetrafluoro-1-propanol, instead of the compound of Concrete Example (3) illustrated in Table 1-1.
With respect to the prepared optical recording medium, evaluation test was carried out in the same manner as in Examples 49 to 83 and as a result, good recording characteristics and excellent stability against reproduction light could be confirmed.

### Examples 85 to 90

Optical recording media were prepared in the same manner as in Example 84, except that a compound of Concrete Example illustrated in Table 2-6 among compounds of Concrete Examples illustrated in Tables 1-1 to 1-22 was properly used.
With respect to the prepared optical recording media, evaluation test was carried out in the same manner as in Examples 49 to 83 and as a result, good recording characteristics and excellent stability against reproduction light could be confirmed.

As for Examples 49 to 76, and Comparative Examples 3 and 4, the recording sensitivity, PRSNR, degree of modulation, and PRSNR and degree of modulation after reproduction of 100,000 times were illustrated in Tables 2-2 to 2-4. It could be confirmed that the optical recording media illustrated in the Examples exhibited good recording characteristics and excellent stability against reproduction light.

[Table 31]

**Table 2-2**

| | Compound No. | Recording Sensitivity (mW) | PRSNR | |
|---|---|---|---|---|
| | | | Degree of modulation | |
| | | | Initial | After reproduction of 100,000 times |
| Example 49 | 2-1 | 8.4 | 24 | 20 |
| | | | 0.59 | 0.59 |
| Example 50 | 2-2 | 7.0 | 24 | 21 |
| | | | 0.58 | 0.58 |
| Example 51 | 2-5 | 7.8 | 28 | 25 |
| | | | 0.71 | 0.70 |
| Example 52 | 3-1 | 8.0 | 23 | 21 |
| | | | 0.69 | 0.70 |
| Example 53 | 2-9 | 8.0 | 20 | 18 |
| | | | 0.53 | 0.55 |
| Example 54 | 2-14 | 7.0 | 25 | 21 |
| | | | 0.66 | 0.66 |
| Example 55 | 2-17 | 7.1 | 29 | 25 |
| | | | 0.73 | 0.72 |
| Example 56 | 2-20 | 5.8 | 29 | 25 |
| | | | 0.74 | 0.73 |
| Example 57 | 2-23 | 8.0 | 23 | 20 |
| | | | 0.53 | 0.54 |
| Example 58 | 2-29 | 6.8 | 21 | 19 |
| | | | 0.51 | 0.50 |

[Table 32]

**Table 2-3**

| | Compound No. | Recording Sensitivity (mW) | PRSNR | |
|---|---|---|---|---|
| | | | Degree of modulation | |
| | | | Initial | After reproduction of 100,000 times |
| Example 59 | 3-5 | 7.8 | 24 | 20 |
| | | | 0.51 | 0.53 |
| Example 60 | 2-32 | 7.2 | 25 | 23 |
| | | | 0.61 | 0.61 |
| Example 61 | 2-33 | 8.8 | 23 | 20 |
| | | | 0.61 | 0.63 |
| Example 62 | 3-6 | 7.6 | 26 | 24 |
| | | | 0.55 | 0.56 |
| Example 63 | 3-7 | 8.2 | 25 | 23 |
| | | | 0.59 | 0.59 |
| Example 64 | 3-8 | 4.8 | 22 | 19 |
| | | | 0.50 | 0.51 |
| Example 65 | 2-35 | 7.0 | 25 | 21 |
| | | | 0.51 | 0.50 |
| Example 66 | 2-40 | 7.2 | 22 | 20 |
| | | | 0.50 | 0.52 |
| Example 67 | 2-43 | 9.1 | 19 | 17 |
| | | | 0.49 | 0.50 |
| Example 68 | 2-45 | 7.8 | 26 | 24 |
| | | | 0.56 | 0.55 |

[Table 33]

**Table 2-4**

| | Compound No. | Recording Sensitivity (mW) | PRSNR | |
|---|---|---|---|---|
| | | | Degree of modulation | |
| | | | Initial | After reproduction of 100,000 times |
| Example 69 | 2-46 | 8.6 | 20 | 18 |
| | | | 0.48 | 0.50 |
| Example 70 | 2-48 | 5.6 | 21 | 18 |
| | | | 0.60 | 0.60 |
| Example 71 | 2-54 | 8.4 | 23 | 21 |
| | | | 0.72 | 0.70 |
| Example 72 | 2-56 | 8.0 | 20 | 18 |
| | | | 0.55 | 0.55 |
| Example 73 | 2-57 | 7.6 | 25 | 23 |
| | | | 0.59 | 0.59 |
| Example 74 | 2-75 | 7.7 | 21 | 19 |
| | | | 0.58 | 0.59 |
| Example 75 | 3-26 | 6.5 | 21 | 18 |
| | | | 0.50 | 0.52 |
| Example 76 | 3-39 | 6.8 | 23 | 20 |
| | | | 0.53 | 0.51 |
| Comparative Example 3 | Structural Formula (a) | 7.2 | 20 | 10 or less |
| | | | 0.71 | 0.32 |
| Comparative Example 4 | Structural Formula (b) | 16.0 or more | 10 or less | 10 or less |
| | | | 0.44 | 0.30 or less |

As for Examples 77 to 83, the recording sensitivity, PRSNR, the degree of modulation, and PRSNR and the degree of modulation after reproduction of 100,000 times were illustrated in Table 2-5. It could be confirmed that the optical recording medium illustrated in the Example was excellent in initial recording characteristics, the change in PRSNR and the degree of modulation before after reproduction of 100,000 times were extremely small as well, and the stability against reproduction light was particularly excellent.

[Table 34]

**Table 2-5**

| | Compound No. | Recording Sensitivity (mW) | PRSNR | |
|---|---|---|---|---|
| | | | Degree of modulation | |
| | | | Initial | After reproduction of 100,000 times |
| Example 77 | 2-13 | 7.2 | 28 | 28 |
| | | | 0.60 | 0.62 |
| Example 78 | 2-28 | 7.2 | 29 | 28 |
| | | | 0.61 | 0.05 |
| Example 79 | 3-12 | 8.6 | 27 | 28 |
| | | | 0.45 | 0.46 |
| Example 80 | 2-52 | 5.8 | 27 | 27 |
| | | | 0.56 | 0.55 |
| Example 81 | 2-47 | 7.8 | 31 | 32 |
| | | | 0.64 | 0.66 |
| Example 82 | 2-49 | 7.2 | 26 | 26 |
| | | | 0.58 | 0.60 |
| Example 83 | 2-50 | 7.9 | 29 | 30 |
| | | | 0.63 | 0.64 |

As for Examples 84 to 90, the recording sensitivity, PRSNR, the degree of modulation, and PRSNR and the degree of modulation after reproduction of 100,000 times were illustrated in Table 2-6. It could be confirmed that the optical recording medium illustrated in the Example exhibited good recording characteristics and excellent stability against reproduction light.

[Table 35]

**Table 2-6**

| | Compound No. | | Mixing ratio (Weight ratio) | Recording Sensitivity (mW) | PRSNR | |
|---|---|---|---|---|---|---|
| | | | | | Degree of modulation | |
| | | | | | Initial | After reproduction of 100,000 times |
| Example 84 | 38 | 2-10 | 1:1 | 5.8 | 32 | 30 |
| | | | | | 0.65 | 0.64 |
| Example 85 | 38 | 2-10 | 4:1 | 6.2 | 33 | 29 |
| | | | | | 0.66 | 0.68 |
| Example 86 | 38 | 2-13 | 1:1 | 6.3 | 32 | 30 |
| | | | | | 0.66 | 0.65 |
| Example 87 | 2-50 | 2-15 | 3:1 | 6.2 | 32 | 28 |
| | | | | | 0.64 | 0.65 |
| Example 88 | 2-50 | 3-8 | 1:1 | 5.6 | 27 | 24 |
| | | | | | 0.59 | 0.60 |
| Example 89 | 2-49 | 2-34 | 1:1 | 6.0 | 29 | 27 |
| | | | | | 0.68 | 0.66 |
| Example 90 | 2-13 | 2-15 | 1:1 | 5.7 | 28 | 26 |
| | | | | | 0.65 | 0.64 |

### INDUSTRIAL APPLICABILITY

According to the present invention, by using the azo metal chelate compound according to the present invention for a recording layer, it is possible to provide an optical recording medium capable of performing recording and reproducing with a laser having a wavelength of 300 to 900 nm, particularly with a blue-violet laser having a wavelength of 400 to 410 nm, which is highly paid attention to as a high-density optical recording medium.

## Claims

1. An optical recording medium having at least a recording layer containing an organic dye and a reflective layer on a substrate, wherein the recording layer contains a metal chelate compound of azo compounds represented by the general formula (1) below and a metal, 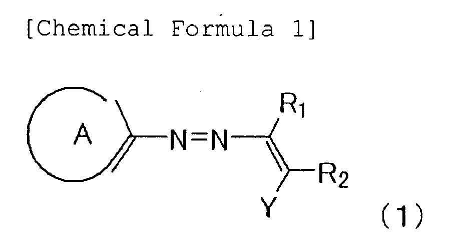 wherein, in the formula, ring A represents a carbocyclic aromatic ring which may have a substituent or a heterocyclic aromatic ring which may have a substituent, and two adjacent atoms in the ring are bonded with each other via a linking group represented by the general formula (a) below and form at least one imide ring;
R₁ and R₂ each independently represent a hydrogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, a substituted or unsubstituted hetero arylcarbonyl group, a substituted or unsubstituted alkylaminothiocarbonyl group, a substituted or unsubstituted arylaminothiocarbonyl group, a substituted or unsubstituted alkylthiocarbonyl group, a substituted or unsubstituted arylthiocarbonyl group, a substituted or unsubstituted hetero arylthiocarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted arylcarbonyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted acylthio group, a substituted or unsubstituted arylcarbonylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group, and may be bonded with each other to form a hydrocarbon ring which may have a substituent or a heterocyclic ring which may have a substituent;
when ring A has substituents, and the hydrocarbon ring or the heterocyclic ring formed by bonding R₁ and R₂ has substituents, substituents may be bonded with each other to form a ring; and Y represents a group having an active hydrogen, 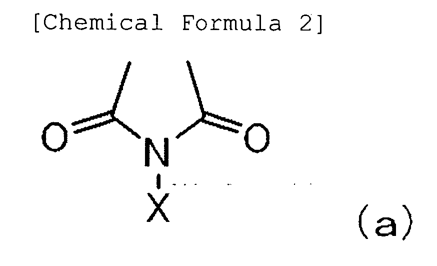 wherein, in the formula, X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group.

2. The optical recording medium as set forth in claim 1 having at least a recording layer containing an organic dye and a reflective layer on a substrate, wherein said recording layer contains a metal chelate compound of azo compounds represented by the general formula (2) below and a metal, 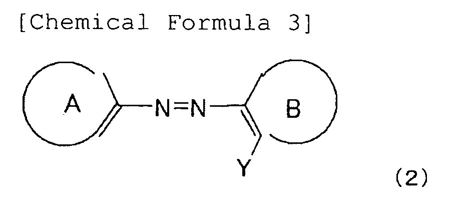 wherein, in the formula, ring A represents a carbocyclic aromatic ring which may have a substituent or a heterocyclic aromatic ring which may have a substituent, and two adjacent atoms in the ring are bonded with each other via a linking group represented by said general formula (a) and form at least one imide ring; ring B represents a hydrocarbon ring which may have a substituent or a heterocyclic ring which may have a substituent; when rings A and B have substituents, substituents may be bonded with each other to form a ring; and Y represents a group having an active hydrogen.

3. The optical recording medium as set forth in claim 2, wherein said recording layer contains a metal chelate compound of-azo compounds represented by the general formula (3) below and a metal, 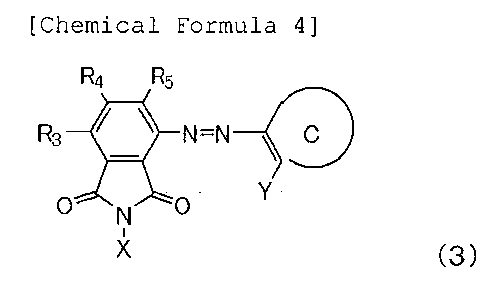 wherein, in the formula, X represents the same as those described above;
R₃ to R₅ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
Y represents a group having an active hydrogen; and
ring C represents the general formulae (C-1) to (C-7) below, 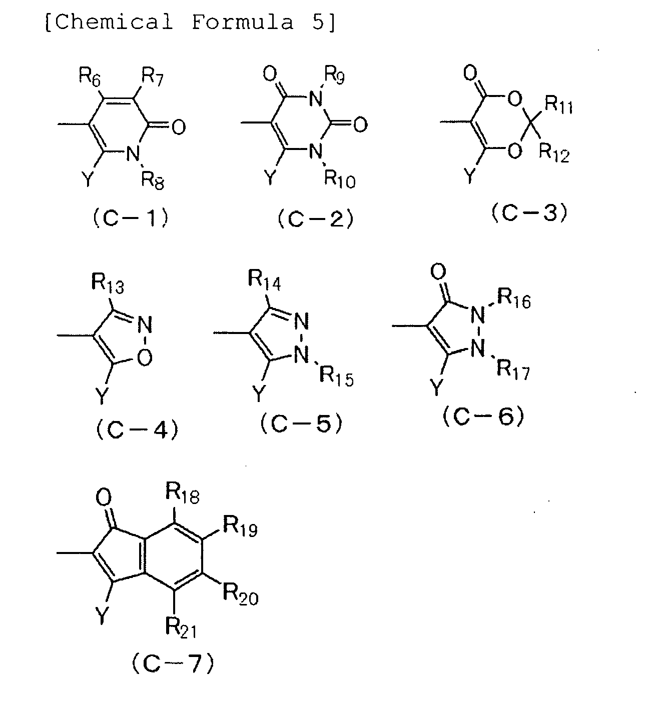 wherein, in the formula, R₆ to R₂₁ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylsulfonyl group, a substituted or unsubstituted arylsulfonyl group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
R₁₁ and R₁₂ may be bonded with each other to form a 5 to 6-membered ring; and Y represents the same as those described above.

4. The optical recording medium as set forth in claim 2, wherein said recording layer contains a metal chelate compound of an azo compound represented by the general formula (4) below and a metal, 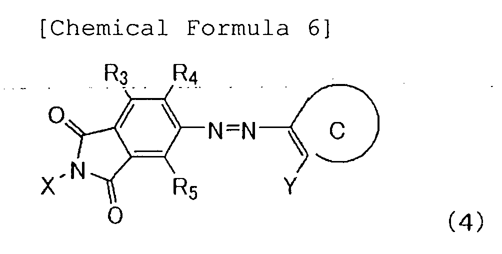 wherein, in the formula, X, R₃ to R₅, Y and ring C represent the same as those described above.

5. The optical recording medium as set forth in claim 1, wherein said recording layer contains a metal chelate compound of azo compounds represented by the general formula (5) below and a metal, 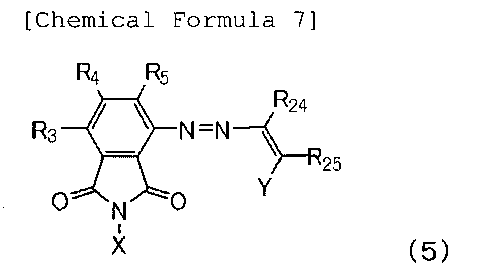 wherein, in the formula, X, R₃ to R₅ and Y represent the same as those described above;
R₂₄ represents a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, or a substituted or unsubstituted hetero arylcarbonyl group; and
R₂₅ represents a hydroxyl group, an amino group, a substituted or unsubstituted alkyl group, a substituted or unsubstituted aralkyl group, a substituted or unsubstituted aryl group, a substituted or unsubstituted hetero aryl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted alkylamino group, a substituted or unsubstituted arylamino group, or a substituted or unsubstituted hetero arylamino group.

6. The optical recording medium as set forth in claim 1, wherein said recording layer contains a metal chelate compound of azo compounds represented by the general formula (6) below and a metal, 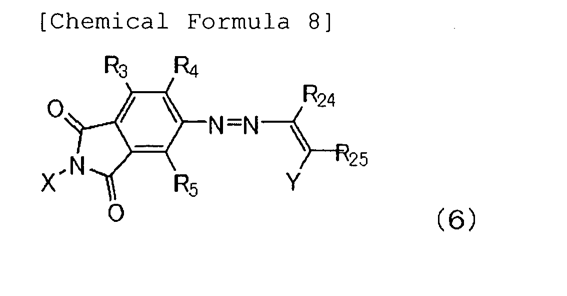 wherein, in the formula, X, R₃ to R₅, Y, R₂₄ and R₂₅ represent the same as those described above.

7. A metal chelate compound of azo compounds represented by the general formula (1) below and a metal, 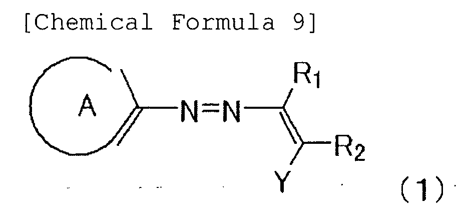 wherein, in the formula, ring A represents a carbocyclic aromatic ring which may have a substituent or a heterocyclic aromatic ring which may have a substituent, and two adjacent atoms in the ring are bonded with each other via a linking group represented by the general formula (a) below and form at least one imide ring;
R₁ and R₂ each independently represent a hydrogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, a substituted or unsubstituted hetero arylcarbonyl group, a substituted or unsubstituted alkylaminothiocarbonyl group, a substituted or unsubstituted arylaminothiocarbonyl group, a substituted or unsubstituted alkylthiocarbonyl group, a substituted or unsubstituted arylthiocarbonyl group, a substituted or unsubstituted hetero arylthiocarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted arylcarbonyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted acylthio group, a substituted or unsubstituted arylcarbonylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group, and may be bonded with each other to form a hydrocarbon ring which may have a substituent or a heterocyclic ring which may have a substituent;
when ring A has substituents, and the hydrocarbon ring or the heterocyclic ring formed by bonding R₁ and R₂ has substituents, substituents may be bonded with each other to form a ring; and Y represents a group having an active hydrogen, 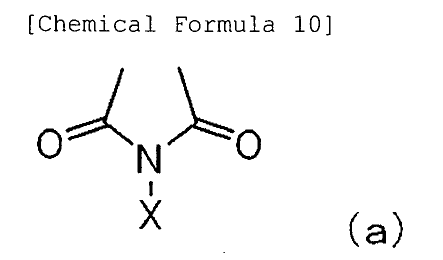 wherein, in the formula, X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted heteroc arylamino group, a substituted or unsubstituted alkylcarbonylamino group, a substituted or unsubstituted arylcarbonylamino group, or a group having a substituted or unsubstituted metalocenyl group.

8. A metal chelate compound of azo compounds represented by the general formula (2) below and a metal, 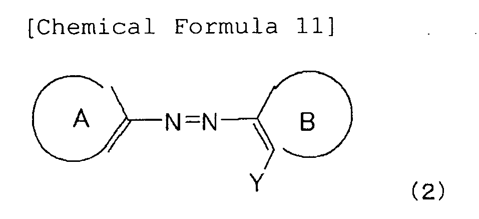 wherein, in the formula, ring A represents a carbocyclic aromatic ring which may have a substituent or a hetero aromatic ring which may have a substituent, and two adjacent atoms in the ring are bonded with each other via a linking group represented by said general formula (a) and form at least one imide ring; ring B represents a hydrocarbon ring which may have a substituent or a heterocyclic ring which may have a substituent;
when rings A and B have substituents, substituents may be bonded with each other to form a ring; and
Y represents a group having an active hydrogen.

9. A metal chelate compound of azo compounds represented by the general formula (3) below and a metal, 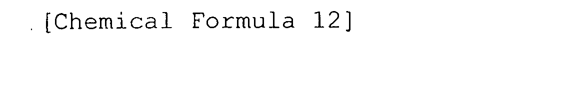 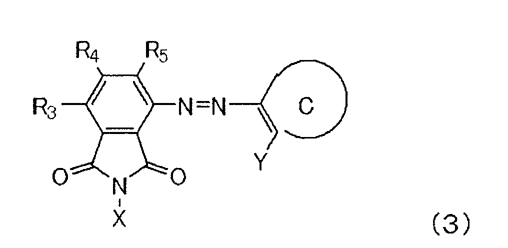 wherein, in the formula, X represents the same as those described above;
R₃ to R₅ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
Y represents a group having an active hydrogen; and ring C represents the general formulae (C-1) to (C-7) below, 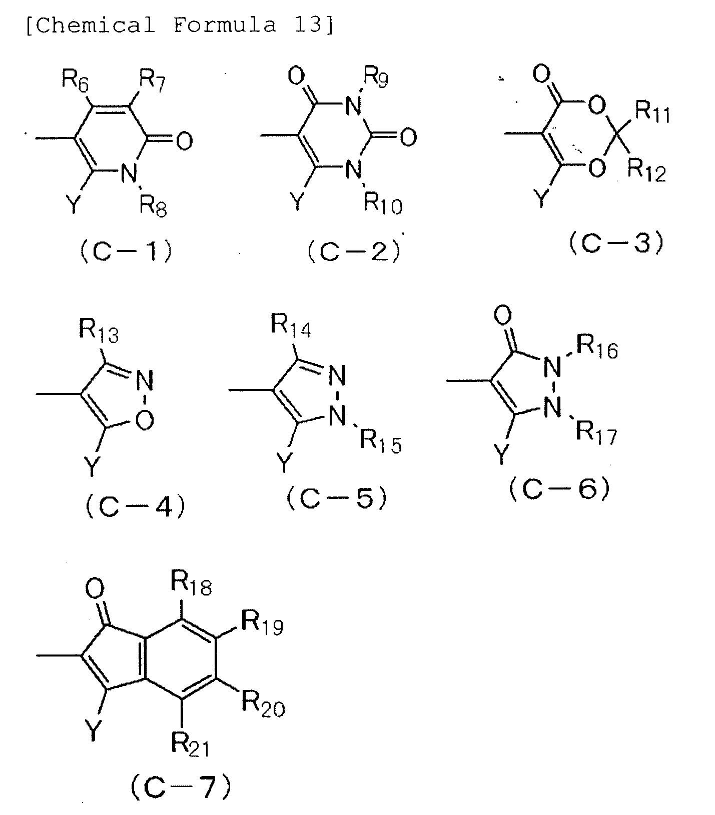 wherein, in the formula, R₆ to R₂₁ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylsulfonyl group, a substituted or unsubstituted arylsulfonyl group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group;
R₁₁ and R₁₂ may be bonded with each other to form a 5 to 6-membered ring; and Y represents the same as those described above.

10. A metal chelate compound of azo compounds represented by the general formula (4) below and a metal, 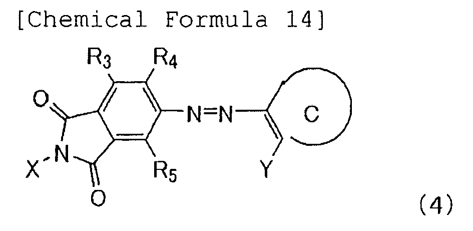 wherein, in the formula, X, R₃, to R₅, Y and ring C represent the same as those described above.

11. A metal chelate compound of azo compounds represented by the general formula (5) below and a metal, 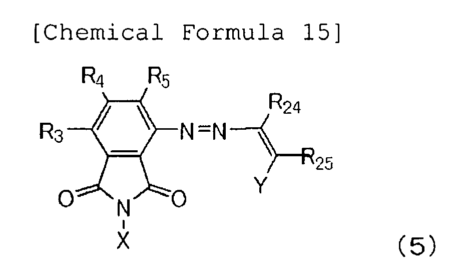 wherein, in the formula, X, R₃ to R₅ and Y represent the same as those described above;
R₂₄ represents a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted alkylaminocarbonyl group, a substituted or unsubstituted arylaminocarbonyl group, a substituted or unsubstituted acyl group, a substituted or unsubstituted arylcarbonyl group, or a substituted or unsubstituted hetero arylcarbonyl group; and
R₂₅ represents a hydroxyl group, an amino group, a substituted or unsubstituted alkyl group, a substituted or unsubstituted aralkyl group, a substituted or unsubstituted aryl group, a substituted or unsubstituted hetero aryl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted alkylamino group, a substituted or unsubstituted arylamino group, or a substituted or unsubstituted hetero arylamino group.

12. A metal chelate compound of azo compounds represented by the general formula (6) below and a metal, 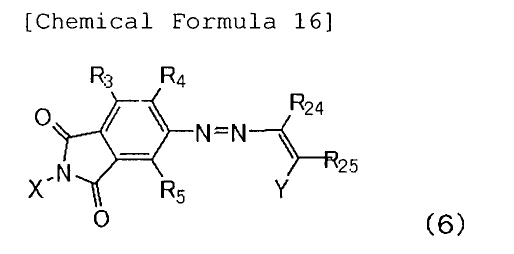 wherein, in the formula, X, R₃ to R₅, Y, R₂₄ and R₂₅ represent the same as those described above.

13. An azo compound represented by the general formula (2) below, 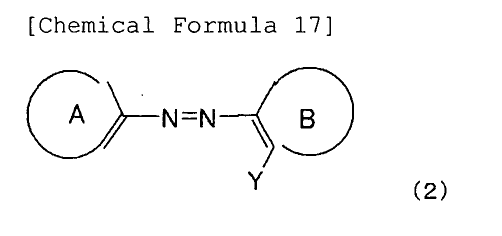 wherein, in the formula, ring A represents a carbocyclic aromatic ring which may have a substituent or a heterocyclic aromatic ring which may have a substituent, and two adjacent atoms in the ring are bonded with each other via a linking group represented by the general formula (a) below and form at least one imide ring;
ring B represents a hydrocarbon ring which may have a substituent or a heterocyclic ring which may have a substituent;
when rings A and B have substituents, substituents may be bonded with each other to form a ring; and
Y represents a group having an active hydrogen, 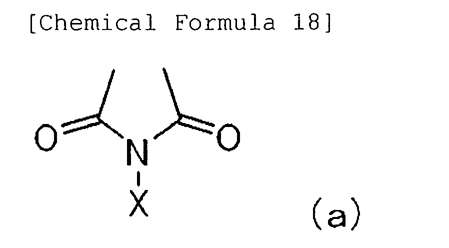 wherein, in the formula, X represents a hydrogen atom, a hydroxyl group, an amino group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted heteroaryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group.

14. The azo compound as set forth in claim 13, represented by the general formula (10) below, 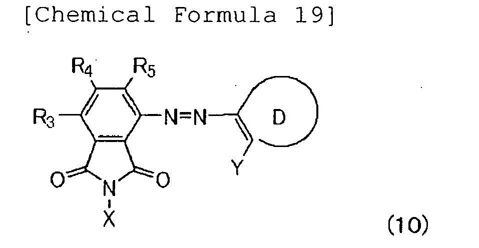 wherein, in the formula, X, R₃ to R₅ and Y represent the same as those described above; and ring D represents the general formulae (D-1) to (D-5) below, 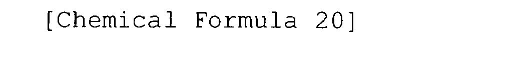 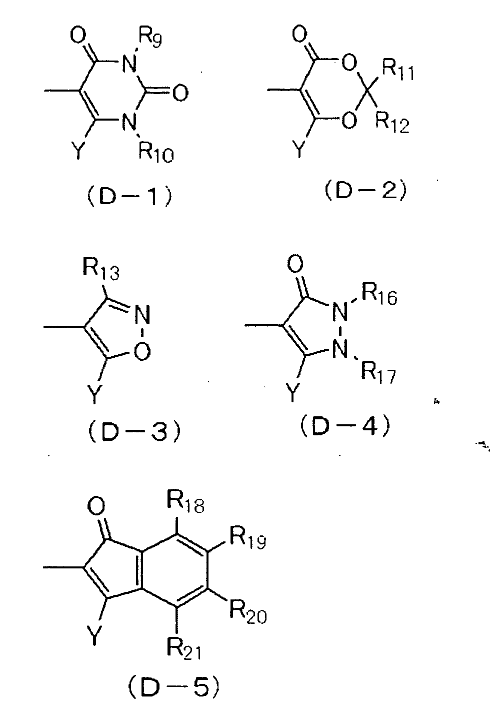 wherein, in the formula, R₉ to R₁₃ and R₁₆ to R₂₁ each independently represent a hydrogen atom, a halogen atom, a hydroxyl group, an amino group, a nitro group, a cyano group, a carboxyl group, a substituted or unsubstituted alkoxycarbonyl group, a substituted or unsubstituted aralkyloxycarbonyl group, a substituted or unsubstituted aryloxycarbonyl group, a substituted or unsubstituted acyl group, a substituted or unsubstituted arylcarbonyl group, a substituted or unsubstituted hetero arylcarbonyl group, a substituted or unsubstituted alkyl group, a substituted or unsubstituted aralkyl group, a substituted or unsubstituted aryl group, a substituted or unsubstituted hetero aryl group, a substituted or unsubstituted alkenyl group, a substituted or unsubstituted alkynyl group, a substituted or unsubstituted metalocenyl group, a substituted or unsubstituted alkoxy group, a substituted or unsubstituted aralkyloxy group, a substituted or unsubstituted aryloxy group, a substituted or unsubstituted hetero aryloxy group, a substituted or unsubstituted acyloxy group, a substituted or unsubstituted alkylthio group, a substituted or unsubstituted aralkylthio group, a substituted or unsubstituted arylthio group, a substituted or unsubstituted hetero arylthio group, a substituted or unsubstituted alkylsulfonyl group, a substituted or unsubstituted arylsulfonyl group, a substituted or unsubstituted alkylamino group, a substituted or unsubstituted arylamino group, a substituted or unsubstituted hetero arylamino group, a substituted or unsubstituted alkylcarbonylamino group, a substituted or unsubstituted arylcarbonylamino group, or a group having a substituted or unsubstituted metalocenyl group; and
R₁₁ and R₁₂ may be bonded with each other to form a 5 to 6-membered ring.

15. The azo compound as set forth in claim 13, represented by the general formula (11) below, 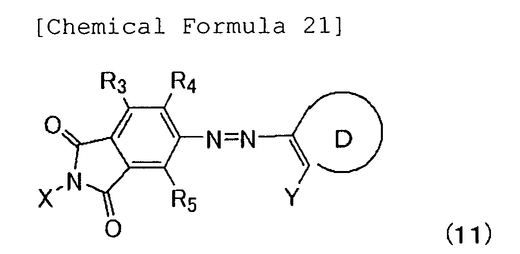 wherein, in the formula, X, R₃ to R₅, Y and ring D represent the same as those described above.
